# EUROPEAN PATENT SPECIFICATION

(11) **EP 4 196 595 B1**
(45) Date of publication and mention of the grant of the patent: **12.03.2025**
(21) Application number: 21777373.8
(22) Date of filing: 11.08.2021
(51) Int. Cl.: C12N 15/86, A61P 25/00

(54) **METHOD FOR THE TREATMENT OF WWOX ASSOCIATED DISEASES**
VERFAHREN ZUR BEHANDLUNG VON WWOX-ASSOZIIERTEN ERKRANKUNGEN
PROCÉDÉ DE TRAITEMENT DE MALADIES ASSOCIÉES À WWOX

(30) Priority: 11.08.2020 US 202063064181 P; 29.03.2021 US 202163167277 P
(43) Date of publication of application: 21.06.2023
(73) Proprietor: Yissum Research Development Company of the Hebrew University of Jerusalem Ltd., 9139002 Jerusalem (IL)
(72) Inventor: AQEILAN, Rami, 9154901 Jerusalem (IL); REPUDI, Srinivas, 9670403 Jerusalem (IL)
(74) Representative: Grund, Martin
(86) International application number: PCT/IL2021/050982
(87) International publication number: WO 2022/034591

(56) References cited:
- WO-A1-2017/147720
- WO-A1-2021/089856
- STEINBERG D J ET AL: "Modeling genetic epileptic encephalopathies using brain organoids", EMBO MOLECULAR MEDICINE, vol. 13, no. 8, 15 July 2021 (2021-07-15), US, XP055866466, ISSN: 1757-4676, Retrieved from the Internet <URL:https://onlinelibrary.wiley.com/doi/full-xml/10.15252/emmm.202013610> DOI: 10.15252/emmm.202013610
- REPUDI S ET AL: "Neonatal neuronal WWOX gene therapy rescues Wwox null phenotypes", EMBO MOLECULAR MEDICINE, 7 November 2021 (2021-11-07), US, XP055866338, ISSN: 1757-4676, Retrieved from the Internet <URL:https://onlinelibrary.wiley.com/doi/full-xml/10.15252/emmm.202114599> DOI: 10.15252/emmm.202114599

## Description

### BACKGROUND

Germline mutations of WW domain-containing oxidoreductase (WWOX) has been documented in epilepsy, ataxia and disorders of sex development (DSD) patients. More recently, genetic meta-analysis of diagnosed Alzheimer's disease identifies the WWOX gene as a new risk locus. Several lines of evidence strongly suggest that WWOX expression is required for normal development and function of central nervous system (CNS) and that mutations in WWOX result in neurological disorders in infants known today as WWOX-related epileptic encephalopathy (WOREE) syndrome.

In WOREE, autosomal recessive WWOX non-sense mutations, partial and complete deletions are associated with very severe disease leading to very early onset death. Heterozygous parents harboring single mutated allele of WWOX do not exhibit phenotypic symptoms. Whether these carriers are more susceptible to adult epilepsy is not yet known. Most of the patients characterized with WOREE harbor compound heterozygous mutations of WWOX making it hard to individually target each mutation. A milder form of the disease is associated with WWOX missense mutations and is referred to as spinocerebellar ataxia, autosomal recessive, 12 (SCAR12). The mechanism by which WWOX regulates homeostasis of CNS is largely unknown. Whether WWOX is downstream of other major effectors that antagonize epilepsy and other forms of neuropathy is also unknown.

Recent evidence also links subtle mutations of WWOX with autism spectrum disorders (ASD). Copy number variants (CNVs) overlapping WWOX were reported in many ASD affected individuals characterized with less severe phenotypes and IQ levels approximate to the normal ranges defining WWOX as an ASD candidate gene. Inherited CNVs of WWOX were determined as a low penetrance risk factor for ASD. Furthermore, mega analysis of multiple sclerosis patient samples has revealed more than 200 autosomal susceptibility variants including those in WWOX. Accordingly, perturbation in WWOX goes beyond a single neurological disease and suggest WWOX as a critical player in several neurological disorders.

In recent years, brain organoids have gained a lot of interest by the scientific community due to their capability to model human diseases. Organoids generated from human pluripotent stem cells harboring WWOX mutations were shown in Steinberg D.J. et al. (2021), "Modeling genetic epileptic encephalopathies using briain organoids", EMBO MOL. MED., vol.13(8):p1-20.

Moreover, an AAV vector carrying a transgene under control of a promoter, and of which WWOX is one example of a transgene, and for use in the treatment of a CNS disease has been suggested in WO 2021/089856 A1.

In addition, WO 2017/147720 A1 discloses exosomes genetically modified to incorporate a nucleic acid, of which a transgene encoding WWOX is one example, for the treatment of a CNS disorder.

### SUMMARY

The invention is defined by the appended claims.

Thus, the invention relates to a WW domain-containing oxidoreductase (WWOX) wild type gene, or a functional derivative thereof encoding an amino acid sequence having at least 95% sequence identity with the amino acid sequence of SEQ ID NO: 2, for use in a method for the treatment of a WWOX-associated CNS disease, the method comprising: administering to the brain of a patient in need of such treatment, said WWOX wild type gene or functional derivative thereof, under control of a regulatory element that results in expression of WWOX in the brain, wherein the WWOX-associated CNS disease is selected from WWOX-related epileptic encephalopathy (WOREE) syndrome or spinocerebellar ataxia, autosomal recessive 12 (SCAR12).

Methods of treatment as such presented in the present disclosure are not according to the invention and are for illustration purposes only.

The present disclosure provides methods and compositions for the treatment of WWOX-associated CNS disease. The methods involve expressing a heterologous WWOX gene in the brain of the subject, and involve expressing the heterologous WWOX gene in neurons to treat or ameliorate conditions such as WOREE syndrome and SCAR12.

### BRIEF DESCRIPTION OF THE DRAWINGS

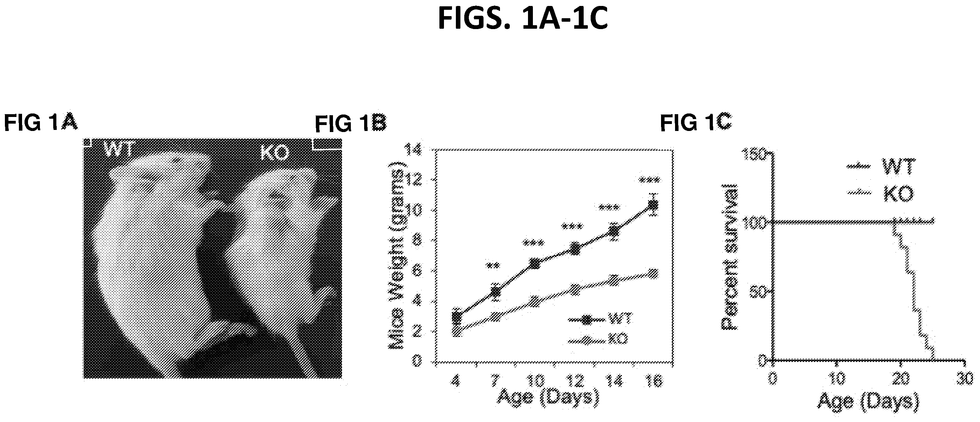

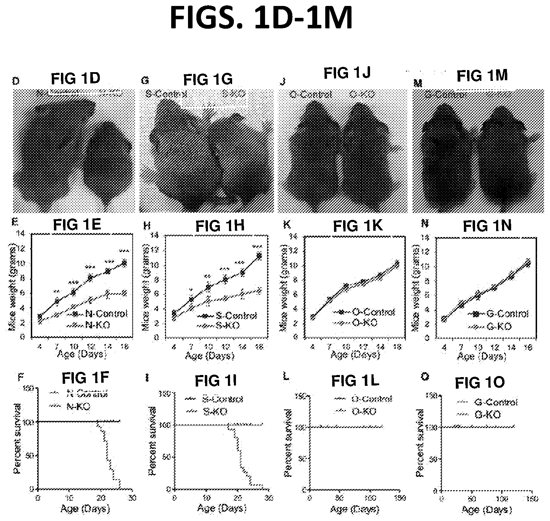

FIGS. 1A-1M provide a summary of a study, showing the phenotypes of the conditional deletion of murine *Wwox* in brain cells. (A) A representative image of *Wwox* null (KO) and wild type (WT) mice at P18. (B) Graph showing weight (in grams, g) gain as function of time. Retarded growth in *Wwox* null is evident from 4 days onwards compared to wild type. Data points represents the average weight of the mice (WT, *n =* 3; KO, *n* = 3). Error bars represents ±SEM (***P* < 0.01, *** *P<* 0.001, Student's t test). (C) Kaplan Meier survival curve showing post-natal lethality of *Wwox* null by the age of 3-4 weeks (WT, *n =* 10; KO, *n =* 11). (*P* = 0.0023, Log-rank Mantel-Cox test). (D) Conditional deletion of *Wwox* gene in neural stem/progenitor cells shows global developmental delay in N-KO compared to the control mice (N-Control). A representative image of these mice shown at P17. (E) N-KO mice exhibit reduced body weight corresponding to N-Control. Data points represents the average weight of the mice, 4 mice per genotype. Error bars represents ±SEM (***P* < 0.01, *** *P* < 0.001, Student's t test). (F) Kaplan Meier survival curve showing post-natal lethality by 3-4 weeks of age in N-KO *(n =* 14) compared to the N-Control *(n =* 12). (*P* =0.0002, Log-rank Mantel-Cox test). (G-I) A representative image of mice (P17) with conditional ablation of *Wwox* in neurons (S-KO) showing (G) growth retardation and (H) reduced body weight (Data points represents the average weight of the mice, 4 mice per each genotype. Error bars represents ±SEM ***P* < 0.01, *** *P* < 0.001, Student's t test) and (I) premature death (S-Control *n =* 13, S-KO *n =* 15). (*P* value 0.0001, Log-rank Mantel-Cox test). (J-L) Conditional ablation of *Wwox* either in oligodendrocytes (O-KO) or in astrocytes (G-KO) does not cause phenotypic abnormalities like developmental delay (J and M, shown at P17), weight loss (K and N) and postnatal lethality (L and O) in either in O-KO or in G-KO mice compared to their corresponding control group (G-Control, O-Control) (in K and L, O- Control *n =* 11 and O-KO *n =* 10, *P* value 1.0, no significance, Log-rank Mantel-Cox test) (G-Control *n =* 8, G-KO *n =* 9 mice were used in N and M, *P* value 1.0, no significance, Log-rank Mantel-Cox test).

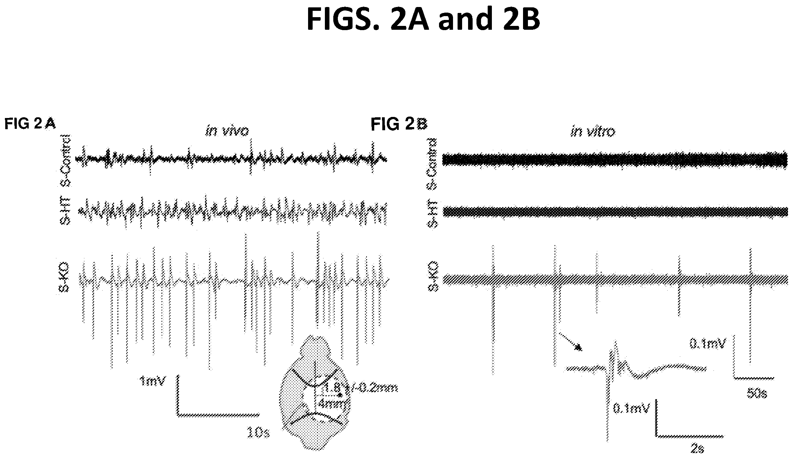

FIGS. 2A-F provide a summary of a study showing neocortical hyperexcitability in the S-KO neocortex. (A) *In vivo* recordings (P13-P17) from S- Control, S-HT and S-KO indicating bursting activity in S-KO (shown in red) as compared to heterozygote (S-HT, shown in blue) and S-Control, (shown in black). (B) *In vitro* recordings (P13-P17) from the superficial neocortex indicating spontaneous neocortical bursting in isolated neocortical slice preparations. Inset shows expanded trace of one bursting event from the S-KO example. Inset of whole brain shows the positioning of the *in vivo* recording electrodes. Of an evaluation of all data that included both spontaneous activity and data with electrical stimulation artifacts obtained from 11 S-Control, 23 S-HT and 42 S-KO slices from 7 S-Control, 14 S-HT and 24 S-KO animals, 0 S-Control slices showed busting (0%), 4 slices from 3 S-HT animals showed bursting (~20%), and 36 slices from 20 S-KO animals showed bursting (~84%). (C) A second example of an *in vivo* recording and its time-frequency spectrogram. (D) Power spectral analysis for *in vivo* and *in vitro* datasets for S-Controls, S-HTs and S-KOs obtained from data clear of stimulation artifacts. Gray bars indicate regions of significance as identified frequency-by-frequency. *In vivo* 12-20Hz shows elevated power in S-KO as compared to S-HT and 7-15Hz for S-KO as compared to S-Control. *In vitro* 3-13Hz showed elevated power of S-KO as compared to S-HT and S-Control. *In vitro* data z-score normalized to mean and standard deviation of 320-400Hz. Box plots show normalized power with frequency bands indicated on the horizontal axis. For 0-4.9Hz and 5-9Hz, S-KO power was significantly elevated as compared to S-Control only (*In vivo,* S-Control, *n =* 5 subjects; S-KO, *n =* 7 subjects; S-HT, *n=* 7 subjects, * *P* < 0.05; *in vitro* * *P* < 0.05;** *P<* 0.01 student's t test, S-KO as compared to S-Control and S-HT; S-Control, *n =* 11 slices, 7 animals; S-HT, *n* = 11 slices, 8 animals; S-KO, *n* =34 slices, 20 subjects). (E) Responses to electrical stimulation from layer V, recording in layer II/III of the neocortex. Arrows indicate the stop and start of the 1^{st} peak response and caret symbols indicate the stop and start of the 2^{nd} peak response at 100uA stimulus strength. (F) Amplitude of the 1^{st} and 2^{nd} peak responses (1^{st} peak: * *P* = 0.0127. 2^{nd} peak: ** *P* = 0.008, * *P* = 0.0202; Wilcoxon rank sum test S-Control *n =* 6 slices, 3 subjects; S-HT, *n =* 8 slices, 5 subjects; S-KO, *n =* 8 slices, 6 subjects).

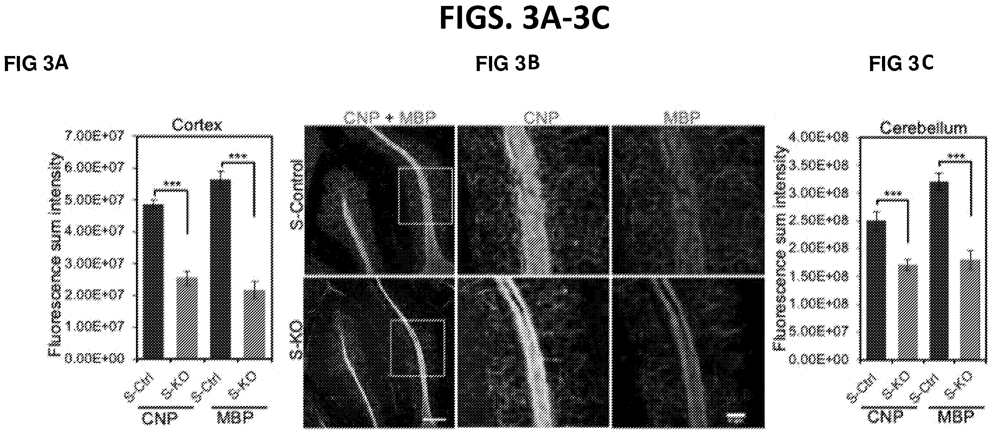

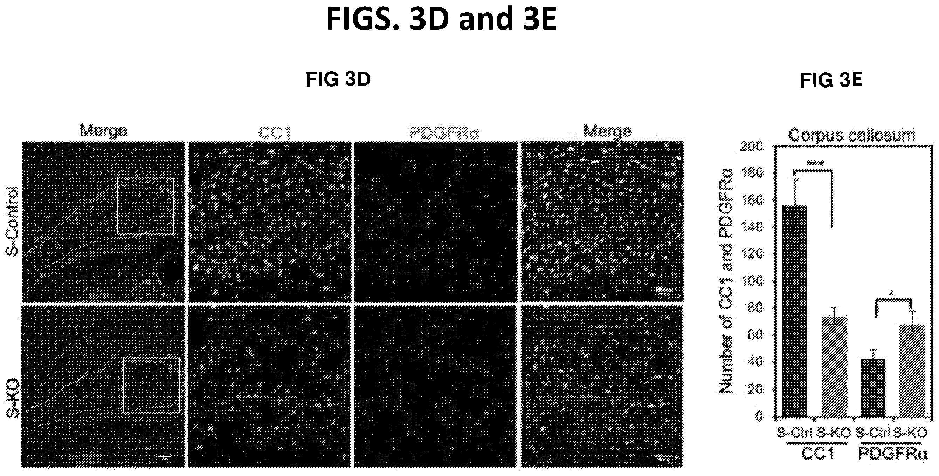

FIGS. 3A-3E provide a summary of a study, showing that neuronal deletion of *Wwox* impairs myelination and oligodendrocyte maturation. (A) Quantification of fluorescence sum intensity of CNP and MBP from three identical sections in the cortex region shows reduced intensity in S-KO *(n* = 3) compared to S-Control *(n =* 3). (B) Representative images of brain sections immunostained with anti-CNP and anti-MBP are shown from cerebellum. (C) Quantification of fluorescence intensity of CNP and MBP display reduced intensity in S-KO compared to S-Control. (D) Sagittal section of the brain tissues immunostained for CC1 and anti-PDGFRα. Images showing reduced number of matured oligodendrocytes in corpus callosum (marked with dotted white line and the magnified area is shown with white square) of S-KO compared to S-Control at P17. (E) Quantification of CC1 and PDGFRα positive cells in corpus callosum showing significantly reduced CC1 positive cells and increased in number of PDGFRα positive cells in SK-O compared to S-Control. Data points represent number of cells counted in the area (0.5mm²) from three independent sections of S-Control (*n* = 3) and S-KO mice *(n* = 3). Error bar represents ±SEM (* *P* ≤ 0.01, ** *P* ≤ 0.001). Scale bars A) 50 µm, B) 2 µm and C) 50 µm.

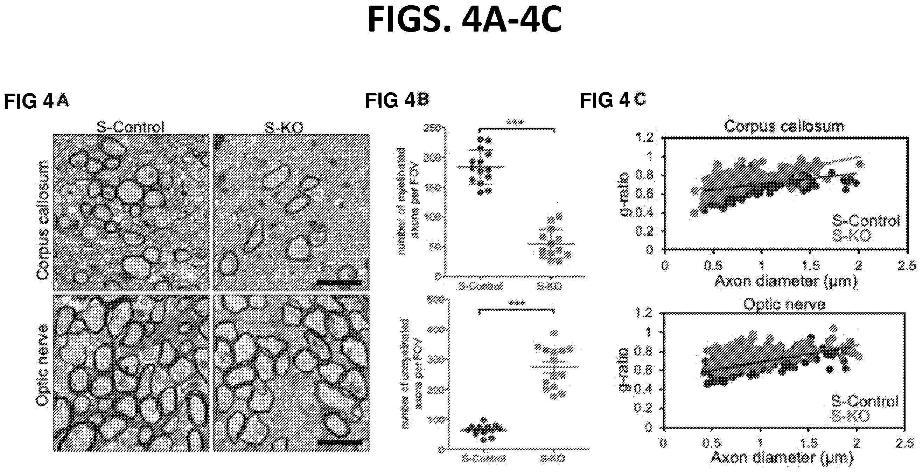

FIGS. 4A-4D provide a summary of a study, showing that neuronal deletion of *Wwox* reduces the myelination and axonal conductivity. (A) Electron micrograph (EM) pictures from mid sagittal section of corpus callosum showing a smaller number of myelinated axons and more unmyelinated axons in the optic nerve of S-KO *(n* = 3), compared to S-Control (*n* = 3) at P17. (B) Quantification of number of myelinated axons in corpus callosum and unmyelinated axons in optic nerve per field of view in S-Control vs S-KO. Graphs represent the myelinated axons (S-Control, *n* = 2500 and S-KO, *n =* 1200) in corpus callosum and unmyelinated axons (S-Control *n =* 500 and S-KO, *n =* 3000) in optic nerve were counted from EM images per FOV (Field of view). Error bar represents ±SEM (*** *P* ≤ 0.001, student's t test). (C) g- ratio analysis showing reduced myelin thickness of axons in corpus callosum and optic nerve in S-KO *(n* = 3) compared to the S-Control (*n* = 3). Axon diameter and myelin thickness were calculated from the electron micrograph images of corpus callosum (*n* = 300 per each genotype) and optic nerve (*n* = 300 per each genotype) (*** *P* ≤ 0.001, student's t test). (D) Representative examples of the evoked responses from corpus callosum stimulation, (i) recording at a vertical distance of 250µm from the stimulator. Inset shows placement of electrode and stimulus. (N1 **P* = 0.0104; N2 = 0.5737 Wilcoxon rank sum test) (ii) Latency to N1 and N2 from the onset of the stimulus. (ii) Ratio of the amplitude of N1 and the amplitude of N2. (N1/ N2). (** *P* = 0.0030; Wilcoxon rank sum *n* = 8 slices, 5 subjects; S-Control; *n* = 8 slices 6 subjects S-KO).

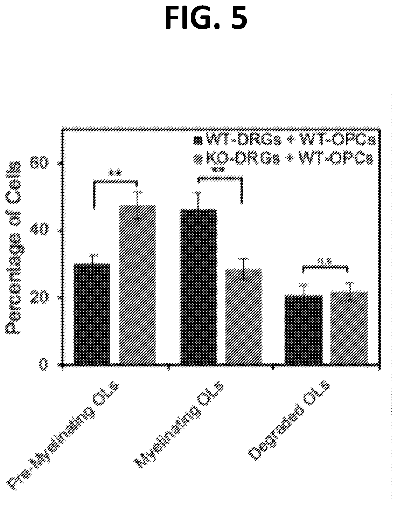

FIGS. 5 provide a summary of a study, showing the non-cell autonomous function of WWOX in OPCs differentiation. Quantification of pre-myelinating, myelinating and degraded oligodendrocytes counted (area covered 14mm diameter). Results are shown in a box plot from two independent experiments (WT-DRGs+WT-OPCs, *n* = 4; KO-DRGs+WT-OPCs, *n* = 4). (***P<* 0.01. n.s no significance). Scale bars A) 100 µm, D) 50 µm.

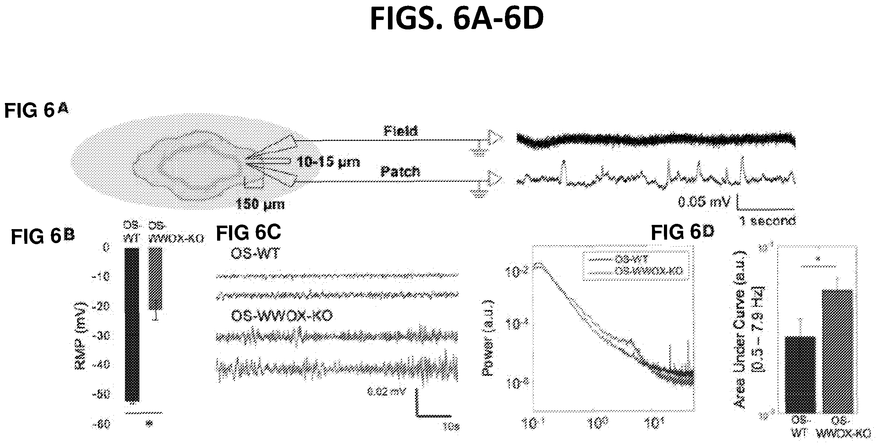

FIGS 6A-6G provide a summary of a study, showing that WWOX-deficient oligocortical spheroids exhibit hyperexcitability and hypomyelination. (A) Schematic illustration of oligocortical spheroids slice set up. An electrode is used for local field potential recordings (LFP) while a second one is used for whole-cell patch recordings. Electrodes are positioned 150µm from the edge of the slice and 10-15µm apart. (B) Resting membrane potential of cells in whole-cell patch clamp recordings for OS-WT and OS-WWOX-KO organoids at week 15 (OS-WT *n =* 3, OS-WWOX-KO *n* = 4*;* OS-WT RMP = -52.1 ± 0.90 mV, OS-WWWOX-KO RMP = -21.28 ± 6.91 mV). (**P <* 0.01, Student's t test). (C) Mean spectral power of OS-WT and OS-WWOX-KO organoids at week 15 in baseline condition (*n =* 9 slices, 3 organoids for OS-WT, *n =* 6 slices, 2 organoids for OS-WWOX-KO; OS-WT AUC = 0.0285 ± 0.0097, OS-WWWOX-KO AUC = 0.0541 ± 0.0093. (**P* < 0.05, Student's t test). (D) Area under the curve of the mean spectral power in (C) for delta and theta ranges (0.5-7.9 Hz). (**P* < 0.05, Student's t test). (E) Week 30 OS stained for CNP (OLs) and MBP (OLs). Images on the right side are enlargement of the boxed area on the left. (OS-WT, *n =* 5; OS-WWOX-KO, *n =* 5). (F) A representative electron micrograph images showing more myelinated axons OS-WT *(n =* 3) compared to OS-WWOX-KO *(n* = 3) in week 37 organoids. Enlarged area shown in a box. (G) Bar graph represents the percentage of myelinated and unmyelinated axons in OS-WT *(n =* 2) and OS-WWOX-KO *(n =* 3). Scale bars in (E) and (F) 100 µm, (G) 40 µm and (H) left panel 500 nm and right panel 200 nm.

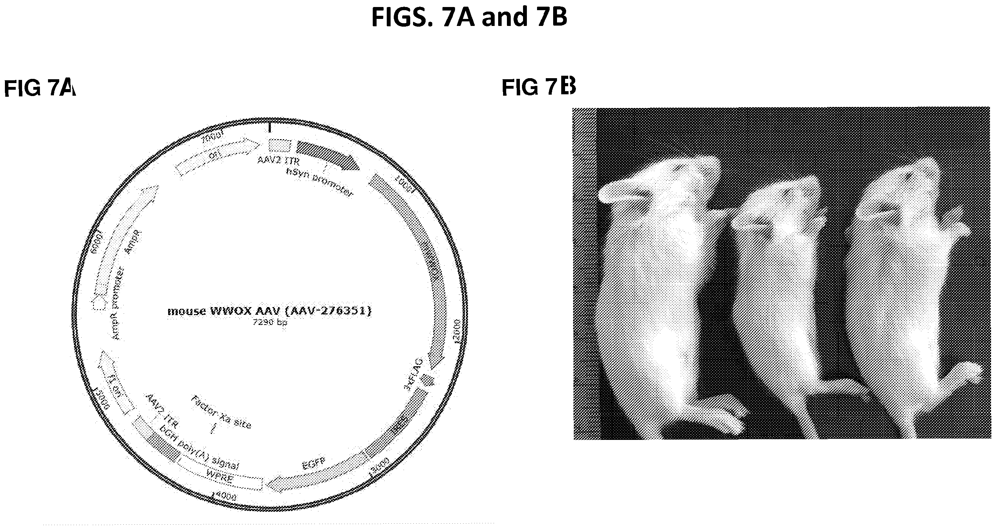

FIGS. 7A-7F provide a summary of a studying, showing that restoration of WWOX in Synapsin I-positive neurons improves growth of *Wwox* null mice and extends their life span. (A) Illustration of the plasmid vector construct containing murine *Wwox* gene under human Synapsin I promoter. The *Wwox* gene sequence is followed by an IRES promoter and *EGFP* gene sequence. (B) Physical appearance of wild type (WT), *Wwox* null injected either with *AAV9-hSynI-GFP* (the control virus) or *AAV9-hSynI-mWwox-IRES-GFP* virus at P17. Graphs showing body weight (C) and blood glucose levels (D) of the mice at indicated days. Error bars represent ±SEM, n=4 mice per genotype. (E) Kaplan-Meier survival graph indicates prolonged life span of *Wwox* knockout mice injected with *AAV9-hSynI-mWwox* (n=18) compared to mice injected with *AAV9-hSynI-GFP* (n=6) or the non-injected (n=10) (p value <0.0001, Log-rank Mantel-Cox test). (F) Kaplan-Meier survival graph indicates prolonged life span of *Wwox* knockout mice injected with *AAV9-hSynI-hWWOX* (n=6) [median 92 days] compared to mice injected with *AAV9-hSynI-GFP* (n=4) or the non-injected (n=8) (p value = 0.0001, Log-rank Mantel-Cox test).

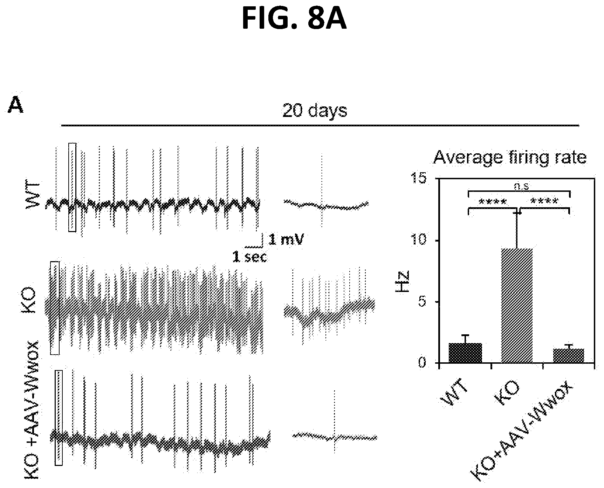

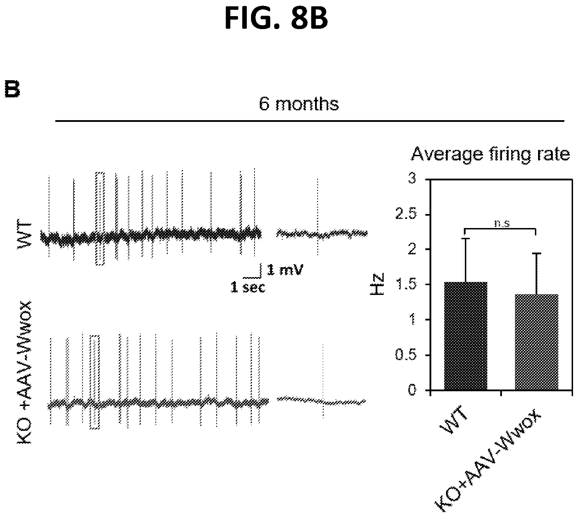

FIGS. 8A and 8B provide a summary of a studying, showing that neuronal restoration of WWOX reduces epileptic activity in neocortex. (A) Representative traces of cell-attached recordings performed in WT, KO and KO-treated with *AAV9-hSynI-mWwox* [KO+A-Wwox] pups at P20-21 days. Traces represent spontaneous neocortical activity (action potentials shown). The panels represent 12 s recording with an inset presenting a zoom-in of an 0.5 s interval. A clear hyperactivity of the KO brain is observed in these representative traces with the KO showing bursts of action potentials. The graph shows the averages over 20 neurons from WT pups (n=2), 20 neurons from KO+A-Wwox pups (n=2) and 30 neurons from KO pups (n=2) (****p value <0.0001, student's t test). (B) Representative traces of cell-attached recordings performed in a WT and a KO+A-Wwox adult mice (6 months old). Traces present spontaneous neocortical activity (action potentials). The panels present 12 s recording with an inset presenting a zoom-in of an 0.5 s interval. The graph shows the averages over 60 neurons from WT adult mice (n=3) and 60 neurons from KO+A-Wwox adult mice (n=3). There was no significant change observed between the average firing rate of the WT and the KO+A-Wwox adult mice.

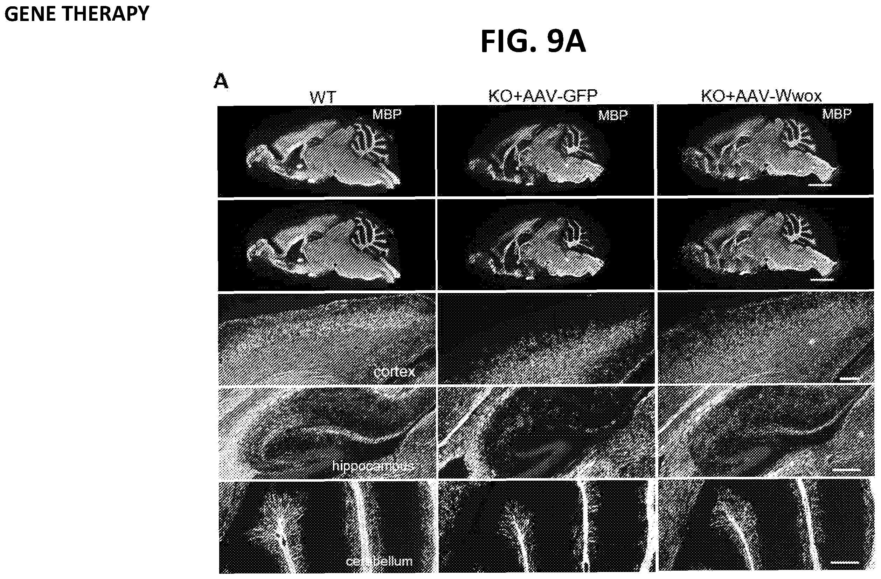

FIGS 9A-9C provide a summary of a study, showing that WWOX restoration in neurons improves myelination by promoting OPCs differentiation in *Wwox* null. (A) Images of whole brain sagittal sections that were immunolabelled with anti-MBP at P17 from indicated mice (n=3 for each group). MBP staining in cortex, hippocampus and cerebellum is presented (magnified from the top panel). (B) Sagittal section of the brain (P17) tissues immunostained for CC1 and PDGFRα. Images showing increased number of matured oligodendrocytes in corpus callosum of the *Wwox* null after treatment with *AAV9-hSynI-mWwox* compared to *Wwox* null injected with *AAV9-hSynI-GFP* virus. (C) Graph represents the quantification of CC1 and PDGFRα positive cells in corpus callosum (area 0.5 mm²) of WT (n=3), KO (n=3) and KO+A-Wwox (n=3) counted from three similar brain sagittal sections per mouse in each genotype. Error bars represent ±SEM. (*p value <0.01, **p value <0.001, ***p value <0.0001). Scale bars (A) 2 mm (top panel), 250 µm (middle and lower panels), (B) 250 µm (top panel), 50 µm (middle and lower panels).

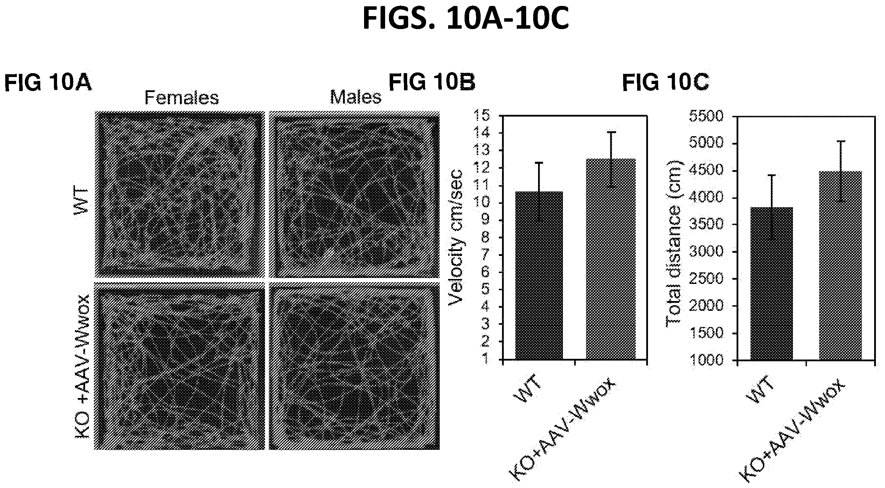

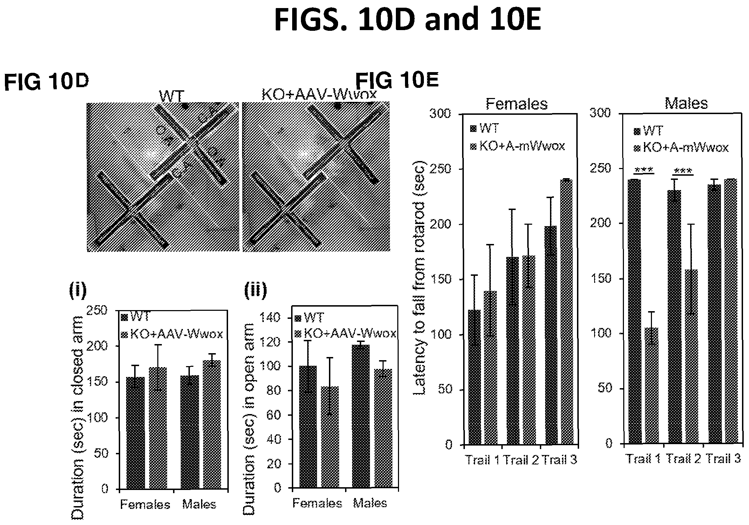

FIGS. 10A-10E provide a summary of a study, showing that WWOX restoration in neurons reverses the abnormal behavioral phenotypes of *Wwox* null mice. (A) Representative images of open field test showing tracking pattern (periphery and the center) of WT (females, n=7, males n=6) and *AAVmWwox-*injected KO mice (females n=7, males n=5) at 8-10 weeks. (B) and (C) Graphs represent the velocity and distance travelled (cm) from the open field tracking. (D) Mice tracking images of elevated plus maze test from WT and KO+AAV-Wwox mice (age 8-10 weeks). O. A. (open arm) C.O. (closed arm) are shown with dotted white boxes on the left mice tracking image. Graphs represent the duration (s) in closed arm (i) and open arm (ii) from WT (females, n=7, males n=6) and KO+AAV-Wwox (females n=7, males n=5). (E) Graphs represent the latency (s) of the mice (WT and rescued mice at 8-10 weeks) to fall from the rotarod in different trials. Females (WT, n=7; KO+-mWwox, n=5) and males (WT, n=5; KO+-mWwox, n=5) are shown separately (***p value <0.0001).

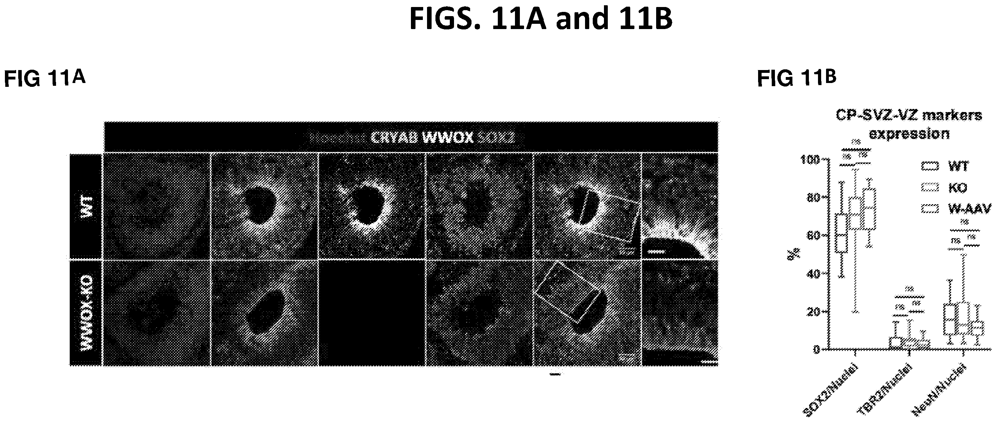

FIGS. 11A-11F provide a summary of a study relating to the generation and characterization of WWOX knockout cerebral organoids. (A) Week10 COs stained for the ventricular radial glial (vRG) marker CRYAB, together with WWOX and the pan-radial glia marker SOX2. The images on the right are enlargements of the boxed area. The dashed line defines the ventricular space (WT: n = 8 from 3 batches, KO: n = 8 from 3 batches). Scale = 50 µm (left), 25 µm (right). (B) Quantification of markers that represent the different populations that compose the ventricular-like zone (VZ), subventricular zone (SVZ), and the cortical plate (CP). NeuN marks the mature neurons, TBR2 marks the intermediate progenitors (IPs), and SOX2 marks the ventricular radial glia (vRGs). The boxplot represents the 1st and 3rd quartile, with its whiskers showing the minimum and maximum points and a central band representing the median. Statistical significance was determined using one-way ANOVA with Tukey's multiple comparisons test (WT: n = 8 from 3 batches; KO: n = 8 from 3 batches; W-AAV: n = 4 from 1 batch). (C) qPCR analysis for the assessment of expression levels of different neural markers in 15 weeks COs: SOX2 and PAX6 (progenitor cells), TUBB3 (pan-neuronal), SLC17A6 and SLC17A7 (VGLUT2 and VGLUT1; glutamatergic neurons), and GAD1 and GAD2 (GAD67 and GAD65; GABAergic neurons). The y-axis indicates relative expression fold change. Data are represented as mean SEM. Statistical significance was determined using one-way ANOVA with Tukey's multiple comparisons test (WT: n = 4 from 1 batch; KO: n = 4 from 1 batch; and W-AAV: n = 4 from 1 batch). (D) Immunofluorescent (IF) staining for the glutamatergic neuron marker VGLUT1 and GABAergic neuron marker GAD67 (GAD1) in week 10 COs (WT: n = 8 from 3 batches; KO: n = 8 from 3 batches; and W-AAV: n = 4 from 1 batch). Scale = 100 µm. Quantification of the images is shown. VGLUT1 and GAD67 were quantified as the surface area covered by the staining and were normalized to the number of nuclei in each image. The boxplot represents the 1st and 3rd quartile, with its whiskers showing the minimum and maximum points and a central band representing the median. As the samples were not normally distributed, statistical significance was determined using the Kruskal-Wallis test with Dunn's multiple comparisons test. Outliers were removed using the ROUT test (Q = 1%). Data information: n.s (non-significant), *P ≤ 0.05, and ****P ≤ 0.0001.

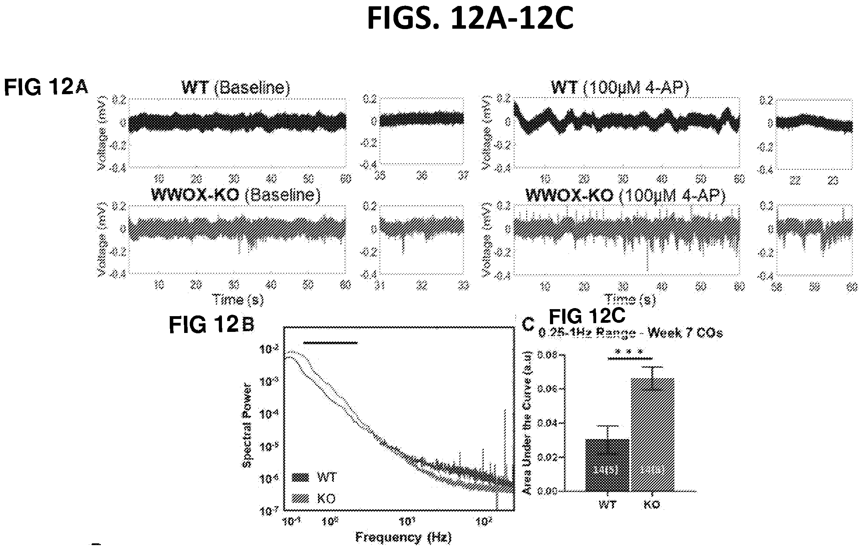

FIGS. 12A-12C provide a summary of a study, showing that WWOX-KO cerebral organoids demonstrated hyperexcitability and epileptiform activity. Sample recordings from 7-week-old hESC-derived cerebral organoids (COs). (A) Sample traces show visible differences in local field potential, with WWOX-KO COs showing increased activity compared with WT in baseline condition (left) and in the presence of 100 µM 4-AP (right). (B) Mean spectral power of week 7 WT and KO COs in baseline conditions. The line marks 0.25- to 1-Hz frequency range. Statistical significance was determined using the two-tailed unpaired Welch's t-test (WT: n = 14 slices, 5 organoids, and 3 batches; KO: n = 14 slices, 8 organoids, and 3 batches). (C) Normalized area under the curve of the mean spectral power in (B) for the 0.25- to 1-Hz frequency range. Data represented by mean SEM. Statistical significance was determined using the two-tailed unpaired Welch's t-test. The numerals in all bars indicate the number of analyzed slices and organoids (i.e., slices (organoids)). (D) and (E) WWOX's coding sequence was reintroduced into week 6 WWOX-KO COs using lentiviral transduction (lenti-WWOX). (D) Immunofluorescent staining showing WWOX expression in different populations in WWOX-KO organoids following infection with lentivirus. NT = non-treated. Scale = 50 µm. (E) Normalized area under the curve of the mean spectral power of WT line, 2 KO lines, and 2 KO lines infected with lenti-WWOX at week 7 in baseline condition, for the 0.25- to 1-Hz frequency range. Data represented by mean SEM. The numerals in all bars indicate the number of analyzed slices and organoids (i.e., slices (organoids)). Statistical significance was determined using one-way ANOVA with Tukey's multiple comparisons test. Data information: ***P ≤ 0.001 and ****P ≤ 0.0001.

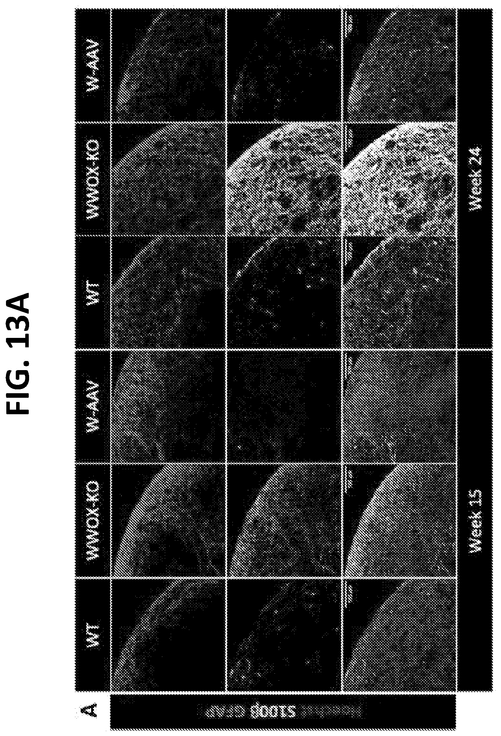

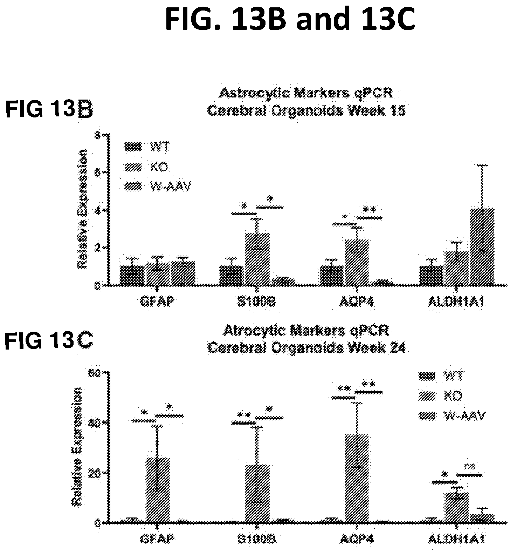

FIGS. 13A-13F provide the summary of a study, showing that WWOX-KO cerebral organoids exhibited impaired astrogenesis and DNA damage response. (A) Week 15 and week 24 COs stained for the astrocytic and radial glial marker GFAP, and the astrocyte-specific marker S100b (WT W15: n = 9 from 3 batches; KO W15: n = 16 from 3 batches; W-AAV W15: n = 4 organoids from 1 batch; WT W24: n = 10 from 4 batches KO W24: n = 9 from 3 batches; and W-AAV W24: n = 4 from 1 batch). Scale = 100 µm. (B) qPCR analysis of astrocytic markers in COs at week 15. The y-axis indicates relative expression fold change. Data are represented as mean ± SEM. Statistical significance was determined using one-way ANOVA with Tukey's multiple comparisons test (WT: n = 4 from 1 batch; KO: n = 4 from 1 batch; and W-AAV: n = 4 from 1 batch). (C) qPCR analysis of astrocytic markers in COs at week 24. The y-axis indicates relative expression fold change. Data are represented as mean ± SEM. Statistical significance was determined using one-way ANOVA with Tukey's multiple comparisons test (WT: n = 4 from 2 batches; KO: n = 3 from 2 batches; and W-AAV n = 3 from 1 batch). (D) IF staining of week 6 COs for astrocytic markers in the surrounding of VZs (WT: n = 6 from 2 batches, and KO: n = 6 from 2 batches). Scale = 100 µm (left) and 50 µm (right). € Staining for the DNA damage markers cH2AX and 53BP1 in the nuclei of cells in the VZ of week 6 COs at physiological conditions, together with the pan-radial glial marker SOX2 (WT: n = 8 from 3 individual batches; KO: n = 12 from 3 individual batches; and W-AAV: n = 4 from 1 batch). Scale = 50 µm (left), 25 µm (right). (F) Quantification of cH2AX (top) and 53BP1 foci (bottom) in the nuclei of cells composing the innermost layer of the VZ, normalized to the total number of nuclei in this layer. The boxplot represents the 1st and 3rd quartile, with its whiskers showing the minimum and maximum points and a central band representing the median. Statistical significance was determined using one-way ANOVA with Tukey's multiple comparisons test (WT: n = 8 organoids from 3 batches; KO: n = 12 organoids from 3 batches; and W-AAV: n = 4 from 1 batch). Data information: n.s (non-significant), *P ≤ 0.05, **P ≤ 0.01, and ****P ≤ 0.0001.

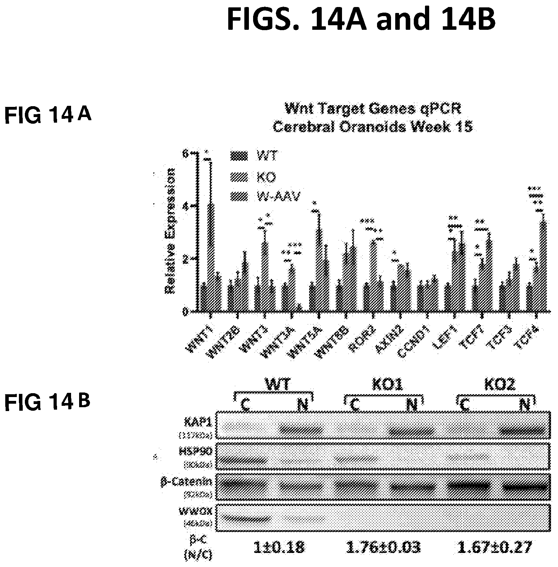

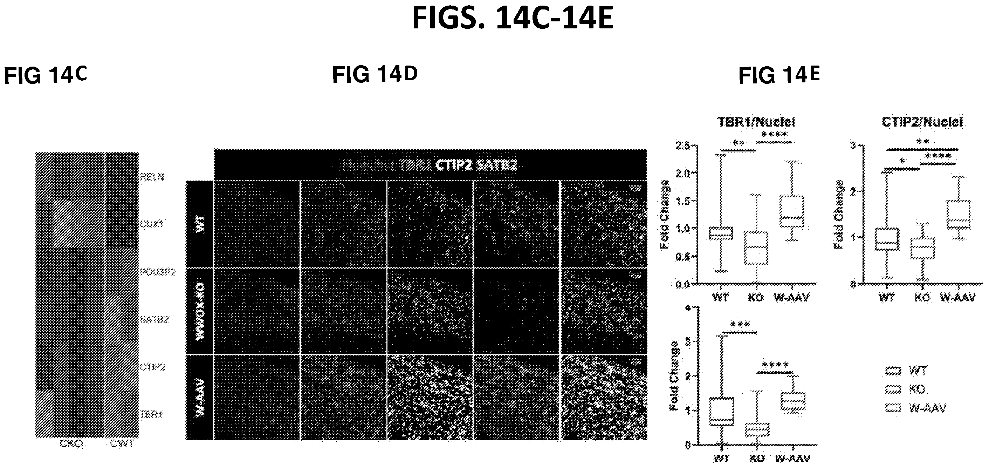

FIGS. 14A-14E provide a summary of a study, showing that cerebral organoid RNA sequencing revealed major differentiation defects. RNA sequencing (RNA-seq) of week 15 COs and transcriptome analysis (WT: n = 2, KO: n = 4). (A) qPCR analysis for selected Wnt target genes validating the results of the RNA-seq. The y-axis indicates relative expression fold change. Data are represented as mean_SEM. Statistical significance was determined using one-way ANOVA with Tukey's multiple comparisons test (WT: n = 4 from 1 batch; KO: n = 4 from 1 batch; and W-AAV: n = 4 from 1 batch). (B) Week 16 COs were subfractionated into a cytoplasmic (C) and nuclear (N) fractions. The experiment was run twice with a total of 2 WT organoids and 4 KOs organoids (2 for each KO line). KAP-1 marks the nucleus, and HSP90 marks the cytoplasm. The numbers at the bottom are a quantification of the nuclear fraction of b-catenin band intensities normalized to the cytosolic fraction [b-C (N/C)]. (C) Heatmap showing the expression levels of markers of the six different layers of the human cortex in week 15 organoids from deepest to the most superficial: TBR1, BCL11B (CTIP2), SATB2, POU3F2 (BRN2), CUX1, and RELN. (D) IF staining in week 15 COs validating the decreased levels of the deep layer cortical markers CTIP2 (BCL11B) and TBR1, and superficial layer marker SATB2 (WT: n = 3; KO: n = 4; and W-AAV: n = 4). Scale = 50 µm. (E) Quantification of the cortical markers seen in G, normalized to the total number of nuclei. The y-axis indicates the fold change compared with the average of the WT COs. The boxplot represents the 1st and 3rd quartile, with its whiskers showing the minimum and maximum points and a central band representing the median. Statistical significance was determined using one-way ANOVA with Tukey's multiple comparisons test (WT: n = 9 from 3 batches; KO: n = 16 from 3 batches; and WAAV: n = 4 organoids from 1 batch). Data information: *P ≤ 0.05, **P ≤ 0.01, ***P ≤ 0.001, and ****P ≤ 0.0001.

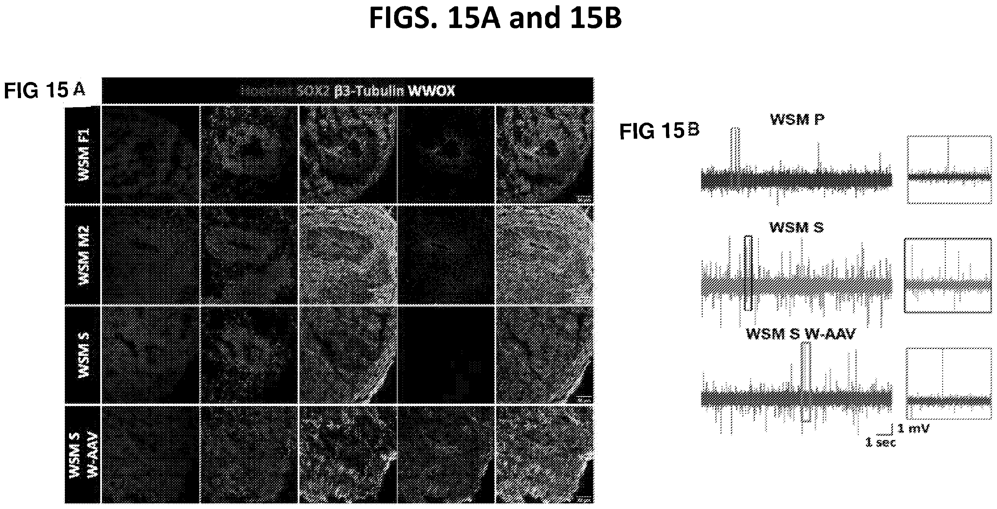

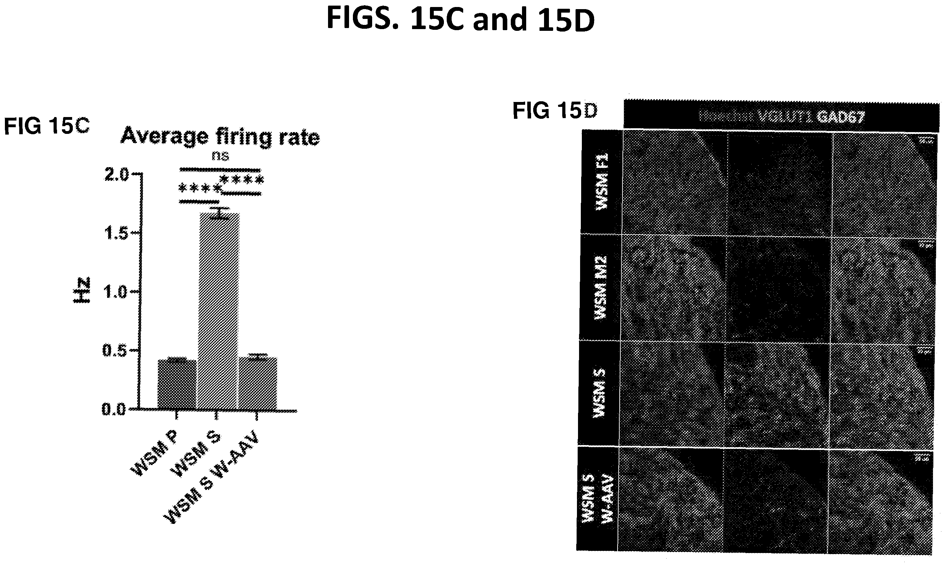

FIGS. 15A-15E provide a summary of a study, showing that WWOX-related epileptic encephalopathy cerebral organoids recapitulate neuronal abnormalities. Peripheral blood mononuclear cells (PBMCs) were isolated from a patient with WOREE syndrome and from his healthy parents and were reprogrammed into iPSCs, and subsequently were differentiated into COs. (A) Week 10 WSM COs stained for the progenitor marker SOX2, neuronal marker b3-tubulin, and WWOX (WSM F1: n = 2 from 1 batch; WSM M2: n = 2 from 1 batch; WSM S2: n = 2 from 1 batch; WSM S5: n = 2 from 1 batch; WSM S5 W-AAV3: n = 2 from 1 batch; and WSM S5 W-AAV6: n = 2 from 1 batch). Scale = 50 µm. (B) Representative traces of spontaneous spikes recorded from neurons of WSM P, WSM S, and WSM S W-AAV week 7 COs. Each recording is 12 s long, and the zoom-in is of 0.5 s (box on the right). (C) Average firing rate over 24 neurons from WSM P COs (4 organoids), 41 neurons from WSM S COs (3 organoids), and 40 neurons from WSM S W-AAV organoids (3 organoids). Statistical significance was determined using one-way ANOVA with Tukey's multiple comparisons test. Bars represents the mean _ SEM. (D) GAD67 and VGLUT1 immunostaining in week 10 WSM COs (WSM F1: n = 2 from 1 batch; WSM M2: n = 2 from 1 batch; WSM S2: n = 2 from 1 batch; WSM S5: n = 2 from 1 batch; WSM S5 W-AAV3: n = 2 from 1 batch; and WSM S5 W-AAV6: n = 2 from 1 batch). Scale = 50 µm. (E) Quantification of the data shown in Fig 15D. Statistical significance was determined using one-way ANOVA with Tukey's multiple comparisons test (WSM F1: n = 2 from 1 batch; WSM M2: n = 2 from 1 batch; WSM S2: n = 2 from 1 batch; WSM S5: n = 2 from 1 batch; WSM S5 W-AAV3: n = 2 from 1 batch; and WSM S5 W-AAV6: n = 2 from 1 batch). Data information: ns (non-significant), *P ≤ 0.05, **P ≤ 0.01, ***P ≤ 0.001, and ****P ≤ 0.0001.

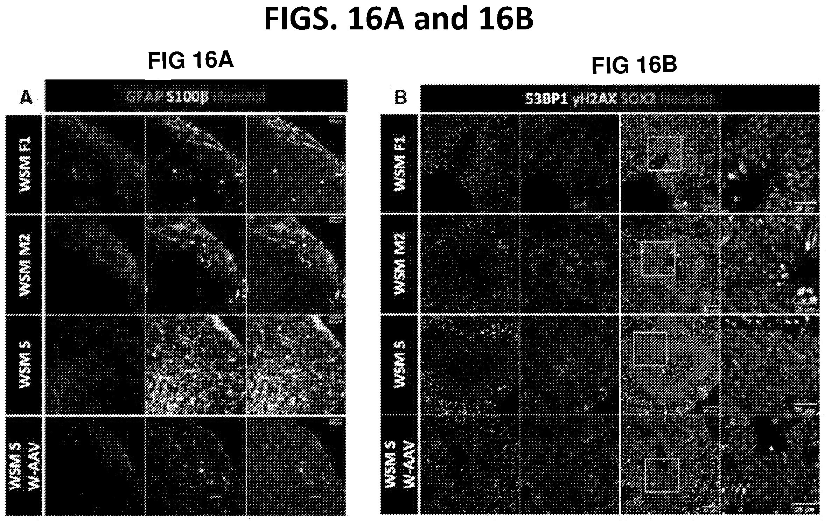

FIGS. 16A-16F provide a summary of a study, showing that WWOX-related epileptic encephalopathies depict molecular abnormalities similar to a complete WWOX loss. (A) Week 15 WSM COs stained for astrocytic markers GFAP and S100b. (WSM F1: n = 2 from 1 batch; WSM M2: n = 3 from 1 batch; WSM S2: n = 2 from 1 batch; WSM S5: n = 2 from 1 batch; WSM S5 W-AAV3: n = 2 from 1 batch; and WSM S5 W-AAV6: n = 2 from 1 batch). Scale = 50 µm. (B) Impaired DNA damage response in WOREE-derived organoids. SOX2 marks the radial glia in the VZ (WSM F1: n = 4 from 1 batch; WSM M2: n = 4 from 1 batch; WSM S2: n = 4 from 1 batch; WSM S5: n = 4 from 1 batch; WSM S5 W-AAV3: n = 4 from 1 batch; and WSM S5 W-AAV6: n = 4 from 1 batch). Scale = 50 µm (left) and 25 µm (right). (C) Quantification of DNA damage foci, marked by cH2AX (left) and 53BP1 (right), in the nuclei of the cells in the VZs of week 6 WSM COs. The boxplot represents the 1st and 3rd quartile, with its whiskers showing the minimum and maximum points and a central band representing the median. Statistical significance was determined using one-way ANOVA with Tukey's multiple comparisons test (WSM F1: n = 4 from 1 batch; WSM M2: n = 4 from 1 batch; WSM S2: n = 4 from 1 batch; WSM S5: n = 4 from 1 batch; WSM S5 W-AAV3: n = 4 from 1 batch; and WSM S5 W-AAV6: n = 4 from 1 batch). (D) qPCR analysis for selected Wnt target genes in week 10 WSM COs. The y-axis indicates relative expression fold change. Data are represented as mean SEM. Statistical significance was determined using one-way ANOVA with Tukey's multiple comparisons test (WSM P: n = 6 from 1 batch; WSM S: n = 4 from 1 batch; and WSM S W-AAV: n = 4 from 1 batch). (E) Week 15 WSM COs stained for the cortical layers' markers CTIP2 and SATB2. (WSM F1: n = 2 from 1 batch; WSM M2: n = 3 from 1 batch; WSM S2: n = 2 from 1 batch; WSM S5: n = 2 from 1 batch; WSM S5 W-AAV3: n = 2 from 1 batch; and WSM S5 W-AAV6: n = 2 from 1 batch). Scale = 50 µm. (F) Quantification of the staining presented in E. The boxplot represents the 1st and 3rd quartile, with its whiskers showing the minimum and maximum points and a central band representing the median. Statistical significance was determined using one-way ANOVA with Tukey's multiple comparisons test (WSM F1: n = 2 from 1 batch; WSM M2: n = 3 from 1 batch; WSM S2: n = 2 from 1 batch; WSM S5: n = 2 from 1 batch; WSM S5 W-AAV3: n = 2 from 1 batch; and WSM S5 W-AAV6: n = 2 from 1 batch). Data information: *P ≤ 0.05, **P ≤ 0.01, ***P ≤ 0.001, and ****P ≤ 0.0001.

### DETAILED DESCRIPTION

The present disclosure provides methods for the treatment and compositions for use in the treatment of WW domain-containing oxidoreductase (WWOX)-associated CNS disease. In various embodiments, the present invention involves the expression of a heterologous WWOX gene in the brain of the subject, and in various embodiments involves the expression of the heterologous WWOX gene in neurons for use in the treatment of WOREE syndrome and SCAR12.

The term "WWOX-associated CNS disease" refers to diseases resulting from or associated with a mutated WWOX gene or with abnormal WWOX expression. Mutation could lead to complete or partial genomic deletion leading to loss of protein or truncation (non-sense mutations) or in milder conditions missense mutation. These diseases are those that are manifested in the CNS. Examples of such diseases are: WWOX-related epileptic encephalopathy (WOREE) syndrome; spinocerebellar ataxia, autosomal recessive, 12 (SCAR12), multiple sclerosis, Alzheimer,'s disease, West syndrome, autism, and disorder of sexual development (DSD).

The disclosure provides for a method for the treatment of WWOX-associated CNS disease. The method comprises administering to the brain of a patient in need of such treatment, a WWOX wild type gene, or a functional derivative thereof, under control of regulatory element(s) that result in expression of WWOX in the brain. The WWOX-associated CNS disease is selected from WOREE syndrome, SCAR12, Alzheimer's disease, West syndrome, autism, multiple sclerosis and DSD.

In some embodiments, the WWOX-associated CNS disease is WOREE syndrome or SCAR12. Alternatively, the patient has compound heterozygous mutations of WWOX.

The term "treatment" or "treating" in the context of this disclosure refers to improving at least one clinical parameter related to the disease, and further includes prevention of the disease (or one or more clinical parameters of the disease) from manifestation. The term "treatment" or "treating" also refers to improving at least one symptom or aspect of the disease (as compared to non-treated subjects), such as: survival, growth, number or frequency of epileptic episodes, cognitive function, social function (e.g., in autism) fertility, ataxia, retinopathy, mental retardation, and microcephaly.

The term "WWOX wild type gene" refers to a gene that comprises the WWOX coding sequence represented by SEQ ID NO: 1, or which codes for the amino acid sequence of SEQ ID NO: 2. The term "WWOX wild type gene" further includes the cDNA sequence (as represented by SEQ ID NO: 1), or comprises the gene sequence with one or more introns. For example, the full gene sequence with introns is represented by NCBI Reference Sequence: NC_000016.10. The term "WWOX wild type gene" further includes naturally occuring nucleotide polymorphisms or amino acid modifications (with respect to SEQ ID NO: 1 or SEQ ID NO: 2, respectively) in the human population that are not associated with disease or loss of WWOX function or expression. The WWOX gene can be a functional equivalent of the WWOX wild type gene, that is, the WWOX gene may encode one or more amino acid modifications (such as one, two, three, four, or five amino acid modifications) independently selected from insertions, deletions, or substitutions, and which do not significantly impact WWOX activity or expression (e.g., in neurons). Generally, a functional derivative will encode an amino acid sequence having at least 95% sequence identity, or at least 96% sequence identity, or at least 97% sequence identity, or at least 98% sequence identity, or at least 99% sequence identity with the amino acid sequence of SEQ ID NO: 2. A WWOX wild type gene may further comprise regulatory elements, including a promoter and 5'- and 3'- untranslated regions, although these regulatory elements are not restricted to the naturally occurring WWOX gene sequences, but instead can be selected to achieve the desired level of mRNA expression or turnover, and/or desired cell-specificity of gene expression. In some embodiments, the WWOX wild type gene does not include substantial untranslated regions, that is, may consist essentially of or consist of the WWOX coding sequence. In accordance with embodiments of the invention, the WWOX wild type gene will comprise at least a heterologous promoter. As used herein, a "heterologous promoter" is a promoter that is placed in a non-native location, such as in position to control expression of a coding sequence that it does not control in nature.

In some embodiments, the WWOX wild type gene encodes the amino acid sequence of SEQ ID NO: 2. In some embodiments, the WWOX wild type gene is a cDNA sequence, which in some embodiments comprises the nucleotide sequence of SEQ ID NO: 1 or SEQ ID NO: 3.

The term "promoter" refers to a DNA sequence capable of controlling the expression of an RNA, such as transcription of the WWOX wild type gene. Promoter sequences contain at least proximal elements for controlling gene expression, and may optionally further comprise more distal upstream elements, the latter elements often referred to as enhancers. Accordingly, an "enhancer" is a DNA sequence that can stimulate promoter activity, or is an innate element of the promoter or a heterologous element inserted to enhance the level or tissue specificity of a promoter. Promoters may be derived in their entirety from a native gene, or may be composed of different elements derived from different promoters found in nature, or even comprise synthetic DNA segments. It is further recognized that the exact boundaries of regulatory sequences may not be completely defined, and thus DNA fragments of some variation may have identical promoter activity.

In some embodments, the regulatory element includes a promoter that directs expression of the WWOX gene in neurons. For example, the promoter can be a universal promoter. Examples of universal promoters include CMV promoter, E2F1 promoter, and U1snRNA promoter, or a derivative thereof. In some embodiments, the promotor is a universal promoter, and the construct is delivered specifically or selectively to neurons. In some embodiments, the regulatory element is a promoter (a neuron-specific promoter) that is expressed specifically in neurons. Exemplary neuron-specific promoters include synapsin I promoter, CamKII promoter, MeCP2 promoter, NSE promoter, and Hb9 promoter, or a derivative thereof. In some embodiments, the promoter is not expressed or is expressed at a lower level in glial cells. In some embodiments, the promoter is not expressed or is expressed at a lower level in oligodendrocytes and/or astrocytes.

In some embodiments, the regulatory element is a synapsin I promoter, or functional derivative thereof for directing neuron-specific expression. In some embodiments, the promoter is a synapsin I promoter, for example, represented by GeneBank accession number NM_006950 (SEQ ID NO: 4), which confers highly neuron-specific and long-term transgene expression. The structure of the synapsin I promoter is described in Schloch et al., Neutron-specific Gene Expression of Synapsin I, J. Biol. Chem. 271(6): 3317-3323 (1996). In various embodiments, the synapsin I promoter is a functional derivative thereof that comprises (i.e., maintains) the NRSE/RE-1 sequence, which imparts neuron-specific expression. In various embodiments, the synapsin I promoter comprises a nucleotide sequence of at least about 250 nucleotides, or at least about 300 nucleotides, or at least about 350 nucleotides of the 3' end of SEQ ID NO: 4. The synapsin I promoter (or portion thereof) may have up to about 20%, or up to about 10%, or up to about 5% nucleotide modifications, as long as the neuron-specific or neuron-selective expression of the promoter is maintained.

In some embodiments, the WWOX wild type gene, or a functional derivative thereof, under control of a regulatory element comprises the nucleotide sequence substantially as set forth in SEQ ID NO: 5.

In other embodiments, the regulatory element is a promoter that directs expression of the WWOX gene in oligodendrocytes. Exemplary promoters include MBP promoter, PLP1 promoter, and CNP promoter, or a derivative thereof. In some embodiments, the regulatory element is a promoter that directs expression of the WWOX gene in astrocytes. Exemplary promoters include GFAP promoter or S100b promoter, or a derivative thereof.

In some embodiments, the WWOX wild type gene or the promoter further comprises one or more enhancer sequences, which can comprise distal portions of the synapsin I promoter or other neuron-specific promoter. In some embodiments, the promoter comprises one or more neuron-specific or neuron-selective enhancers, to increase expression levels in neurons. See, for example, Charron G. et al., Multiple Neuron-specific Enhancers in the Gene Coding for the Human Neurofilament Light Chain. J. Biol. Chem. 270(51):3064-30610 (1995).

In various embodiments, the WWOX wild type gene or functional derivative comprises untranslated sequences (e.g., a 3'-UTR) that enhance mRNA stability. For example, in some embodiments the WWOX wild type gene may contain a β-globin mRNA 3'-UTR, or components of the β-globin mRNA 3'-UTR that confer mRNA stability. In some embodiments, the WWOX wild type gene includes 3' untranslated sequences from mRNAs that exhibit low turnover in neurons. In some embodiments, the transcribed WWOX nucleotide sequence comprises one or more woodchuck hepatitis post-transcriptional regulatory elements (WPRE), which can enhance stability. The WPRE in some embodiments is included in the 3' UTR of the WWOX gene.

In some embodiments, the WWOX wild type gene is delivered with one or more detectable labels, including but not limited to a fluorescent protein, such as a GFP or RFP, allowing for visualization of expression of the WWOX-containing expression construct.

In some aspects, the WWOX wild type gene or functional derivative thereof (or fragment thereof) is delivered with a Cas endonuclease enzyme or polynucleotide encoding a Cas endonuclease enzyme, and guide RNA (gRNA) or polynucleotide encoding the gRNA, to direct or enhance insertion of the WWOX wild type gene or a portion thereof. In some embodiments, the WWOX-associated CNS disease is characterized by a known mutation in WWOX. In such embodiments, a gRNA complementary to the mutated region, or a DNA sequence coding for said gRNA, are delivered along with a Cas endonuclease. In these embodiments, the WWOX wild type gene may be a fragment of the WWOX wild type gene to replace the mutated sequence cleaved by the Cas endonuclease enzyme (e.g., Cas9).

For example, where the exact mutation in the WWOX gene in the individual is known, the individual is treated by administering to the brain of the individual a Cas endonuclease (e.g., Cas9) enzyme and a gRNA targeted to the mutated sequence, so that the mutated sequence can be edited to revert to the wild type form either by cleaving the mutated region and/or by replacing the sequence of the mutated region by the sequence of the wild type region. For some mutations, mere cleavage of the mutated sequence by the Cas endonuclease will result in a functional WWOX gene. For other mutations, it will be required to not only cleave the mutated sequence, but also to replace it by a wild type sequence (which is a fragment of the WWOX wild type gene). In such a case, the method comprises also the administration of a donor DNA sequence, corresponding to a fragment of the WWOX wild type gene; to replace the mutated sequence.

In some embodiments, the Cas endonuclease (e.g., Cas9) may be administered into the brain cells as a protein or may be administered as a polynucleotide encoding the enzyme and capable of being expressed in the brain cells (e.g., in neurons). In some embodiments, the Cas endonuclease is expressed via a neuron-specific promoter (e.g., synapsin I promoter), as described herein. In some embodiments, the Cas endonuclease is delivered as an mRNA, and thus does not require transcription in transfected cells.

Where a polynucleotide coding for the Cas endonuclease is used, promotors and delivery vectors as described herein for the WWOX gene may be used, or deliver vectors may be used that are capable of delivering longer polynucleotides (which may be better suited for delivering Cas endonuclease-encoding polynucleotides) such as AAV6 and Lentivirus vectors. Where the Cas endonuclease is delivered as a protein, delivery particles, liposomes etc. may be used for its delivery to the brain (e.g., to neurons).

Likewise, the gRNA may be delivered as an RNA molecule or as a DNA molecule coding for the gRNA, using the delivery and promoter systems as described herein for the WWOX gene. In some embodiments, the gRNA is expressed via a neuron-specific promoter.

The WWOX polynucleotide, to replace the mutated DNA, can be administered as a separate sequence or can be delivered as part of a vector containing a sequence coding for the Cas endonuclease and the gRNA. In such a case the donor DNA can be cleaved out of the vector, for example by use of "donor-specific" second set of guide RNA that with the aid of the Cas endonuclease, can cleave the donor out of the vector with blunt ends.

Cas endonuclease (e.g., Cas9) molecules of a variety of species can be used in the methods and compositions described herein, including *S. pyogenes* and *S. thermophilus* Cas9. Other Cas endonucleases are described in US Patent Publication No. 20160010076. The constructs and methods described herein can include the use of any Cas endonuclease, including Cas9 enzymes, and their corresponding gRNAs or other gRNAs that are compatible. The Cas9 from *Streptococcus thermophilus* LMD-9 CRISPR1 system has been shown to function in human cells. (See, Cong et al., Science 339, 819 (2013)).

Guide RNAs generally speaking come in two different systems: System 1, which uses separate crRNA and tracrRNAs that function together to guide cleavage by Cas9, and System 2, which uses a chimeric crRNA-tracrRNA hybrid that combines the two separate gRNAs in a single system (referred to as a single guide RNA or sgRNA, see also Jinek et al., Science 2012; 337:816-821). The tracrRNA can be variably truncated and a range of lengths has been shown to function in both the separate system (system 1) and the chimeric gRNA system (system 2).

Cas endonuclease can be guided to specific 17-20 nt genomic targets bearing an additional proximal protospacer adjacent motif (PAM), e.g., of sequence NGG, using a gRNA, e.g., a sgRNA or a tracrRNA/crRNA, bearing 17-20 nts at its 5' end that are complementary to the complementary strand of the genomic DNA target site. Thus, embodiments can employ a single guide RNA comprising a crRNA fused to a normally trans-encoded tracrRNA, e.g., a single Cas9 guide RNA as described in Mali et al., Science 2013 Feb. 15; 339(6121):823-6, with a sequence at the 5' end that is complementary to the target sequence, e.g., of 25-17, optionally 20 or fewer nucleotides (nts), e.g., 20, 19, 18, or 17 nts, preferably 17 or 18 nts, of the complementary strand to a target sequence immediately 5' of a protospacer adjacent motif (PAM), e.g., NGG, NAG, or NNGG. The gRNAs can include X.N which can be any sequence, wherein N (in the RNA) can be 0-200, e.g., 0-100, 0-50, or 0-20, that does not interfere with the binding of the ribonucleic acid to Cas9.

In some embodiments, the gRNA includes one or more Adenine (A) or Uracil (U) nucleotides on the 3' end. In some embodiments the gRNA includes one or more U, e.g., 1 to 8 or more Us at the 3' end of the molecule, as a result of the optional presence of one or more Ts used as a termination signal to terminate RNA PolIII transcription.

In some embodiments, the gRNA is targeted to a site that is at least three or more mismatches different from any sequence in the rest of the genome in order to minimize off-target effects. Modified RNA oligonucleotides such as locked nucleic acids (LNAs) have been demonstrated to increase the specificity of RNA-DNA hybridization by locking the modified oligonucleotides in a more favorable (stable) conformation. Thus, the gRNAs disclosed herein may comprise one or more modified RNA oligonucleotides. For example, the truncated guide RNAs molecules described herein can have one, some or all of the region of the guide RNA complementary to the target sequence are modified, e.g., locked (2'-O-4'-C methylene bridge), 5'-methylcytidine, 2'-O-methyl-pseudouridine, or in which the ribose phosphate backbone has been replaced by a polyamide chain (peptide nucleic acid), e.g., a synthetic ribonucleic acid.

The gRNA may be provided per se or in an expression vector. The vectors for expressing the gRNAs can include RNA Pol III promoters to drive expression of the gRNAs, e.g., the H1, U6 or 7SK promoters. These human promoters allow for expression of gRNAs in mammalian cells following plasmid transfection. Alternatively, a T7 promoter may be used, e.g., for in vitro transcription, and the RNA can be transcribed in vitro and purified. Vectors suitable for the expression of short RNAs, e.g., siRNAs, shRNAs, or other small RNAs, can be used.

The delivery of the sequences (promotor and gene and optionally additional sequences) can be done by any delivery system suitable for delivery to the CNS, either by direct delivery to the CNS or by systemic delivery. In various embodiments, the WWOX wild type gene or functional derivative thereof is being delivered using a viral vector, polymeric nanoparticles, inorganic nanoparticles, lipid nanoparticles, or exosomes. In some embodiments, the WWOX wild type gene or functional derivative thereof is being delivered by a viral vector. The viral vector can be an adeno-associated virus (AAV) delivery system. In some embodiments, the AAV delivery system is AAV9, which crosses the blood-brain barrier better than other AAV serotypes. Additional viral delivery systems that may be used include Lentivirus and Herpes Simplex Virus delivery systems.

Another suitable delivery vehicle for the CNS comprises nanoparticles, typically having a size of less than 200 nm, or less than about 150 nm, or less than about 100 nm. These may include lipid-based nanoparticles, polymer nanoparticles, dendrimers and inorganic nanoparticles, some of which may be tailored to pass through the blood brain barrier (BBB). In some embodiments, the delivery system actively targets delivery by using ligands of transporters or receptors to enhance nanoparticle uptake across the BBB. The preferred pathway for this approach is receptor (or transporter)-mediated transcytosis by which a cargo (e.g., nanoparticles) transports between the apical and basolateral surface in the brain ECs. For example, low-density lipoproteins undergo transcytosis through the ECs by a receptor-mediated process, bypassing the lysosomal compartment and releasing at the basolateral surface of the brain side. Further, since the BBB contains transporters to amino acids, using the naturally present arginine transporter for the delivery is one approach for delivery to the brain.

Another vehicle for brain delivery is exosomes which are small extracellular vesicles secreted by cells. The major advantage of exosomes versus other synthetic nanoparticles is their non-immunogenic nature, leading to a long and stable circulation.

In some embodiments, delivery to the brain employs compounds or electric stimulation to transiently open the BBB and allow high concentrations of systemically administered polynucleotides to reach the brain. An example of such a compound is cereport (a bradykinin analog) or regadenoson (an adenosine receptor agonist). Another manner to increase penetration is via Ultrasound, which is an attractive technique to facilitate drugs to cross the BBB. Microbubble-enhanced diagnostic ultrasound (MEUS), a non-invasive technique, effectively helps drugs cross the BBB. Another approach is transcranial magnetic stimulation (TMS), which stimulates neuronal activity and increases glutamate release, facilitating delivery across the BBB. (Review by Xiaowei Don. 2018; 8(6): 1481-149).

Routes of administration of the desired delivery vehicles may be systemic delivery without further manipulations (using particles or viral vector, that inherently enter the BBB); or may be systemic in connection with various manipulations (such as microbubble-enhanced diagnostic ultrasound (MEUS), transcranial magnetic stimulation (TMS)) to transiently open the BBB. In other embodiments, delivery is by nasal administration.

In still other embodiments, delivery to the brain is by delivery to the cerebrospinal fluid via intracerebroventricular route. Another option is delivery to the cisterna magna route of injection, which is an alternative method for delivery into cerebrospinal fluid (CSF) which results in wide-spread gene delivery throughout the CNS. In some embodiments, the administration is by direct injection into the parenchyma or injection into the cerebrospinal fluid via the intracerebroventricular, and by intrathecal (cisternal or lumbar) route.

In some embodiments, the individual to be treated is a pediatric or neonatal patient (e.g., a patient with WOREE or SCAR12). In some embodiments, early treatment prevents manifestation of some clinical parameters of disease, such as growth impairments, epileptic episodes, impairment of cognitive function, and mental retardation. In some embodiments, the individual is an adult patient (e.g., with WOREE or SCAR12), and treatment can ameliorate one or more clinical parameters, such as epileptic episodes. In various embodiments, the individual or patient exhibits one or more symptoms selected from growth impairment, epileptic episodes, impairment of cognitive function, impairment of social function, impairment of fertility, ataxia, retinopathy, mental retardation, and microcephaly. In various embodiments, the treatment substantially reduces frequency and/or severity of epileptic episodes for patients with WOREE or SCAR12.

In various embodiments, there are no more than 10, 9, 8, 7, 6, 5, or 4 administration episodes to an individual. In various embodiments, there are no more than three administration episodes. In some embodiments, there are no more than two administration episodes. For example, in various embodiments there is one administration episode.

For example, in some embodiments, the invention provides an AAV9 gene delivery system comprising a WWOX wild type gene (i.e., encoding the polypeptide of SEQ ID NO: 2) under control of a synapsin I promoter for use in the treatment of WOREE syndrome or SCAR12, the method comprising administering to the brain of a patient in need of such treatment said delivery system. The AAV9 delivery system may comprise the nucleotide sequence substantially as set forth in SEQ ID NO: 1, SEQ ID NO: 3, SEQ ID NO: 4, and/or SEQ ID NO: 5.

In other aspects, the present disclosure provides an expression construct comprising a WWOX wild type gene, or a functional derivative thereof, under the expression control of a neuron-specific promotor. Exemplary neuron-specific promoters include synapsin I promoter, CamKII promoter, MeCP2 promoter, NSE promoter, and Hb9 promoter, or a derivative thereof. In various embodiments, the promoter is synapsin I or derivative thereof as already described. In some embodiments, the nucleotide sequence comprises a sequence substantially as set forth in SEQ ID NO: 1, 3, 5, and/or 5.

In various embodiments, the expression construct is a viral vector, such as an adeno-associated virus (AAV) delivery system. In some embodiments, the expression construct is an AAV9 delivery system. A specific example for such a vector is depicted in FIG. 7A.

In some embodiments, the expression construct is contained in a pharmaceutical composition for administration into the brain. The composition will further comprise a pharmaceutically acceptable carrier suitable for injection, including direct injection to the brain or CNS, or systemic administration.

Disclosed is a method for treating WOREE syndrome or SCAR12, comprising, administering the pharmaceutical composition to a patient in need. Thus, the present invention provides for a use of the pharmaceutical composition in the treatment of WOREE or SCAR12.

### Definitions

In order that the application may be more completely understood, several definitions are set forth below. Such definitions are meant to encompass grammatical equivalents.

The term "a" or "an" refers to one or more of that entity, i.e. can refer to a plural referent. As such, the terms "a" or "an", "one or more" and "at least one" are used interchangeably herein. In addition, reference to "an element" by the indefinite article "a" or "an" does not exclude the possibility that more than one of the elements is present, unless the context clearly requires that there is one and only one of the elements.

The term percent "identity" or "sequence identity" in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection.

The term "about", unless the context requires otherwise, means ±10% of an associated value.

### EXAMPLES

### Example 1: Neuronal deletion of Wwox, associated with WOREE syndrome, causes epilepsy and myelin defects

The WW domain-containing oxidoreductase (WWOX) gene maps to chromosome 16q23.1-q23.2 encompassing one of the most active chromosomal fragile sites, FRA16D.^{1, 2} WWOX encodes a 46kDa protein with documented tumor suppressor function in several types of cancer through its protein-protein interactions' ability.^{3, 4} Indeed, WWOX acts as a scaffold protein, regulating localization, stability and function of its partners.⁵ Several lines of evidence link WWOX function with maintaining genomic stability, cellular metabolism and cytoskeleton organization.^{1, 3, 6, 7} Remarkably, high expression levels of WWOX are observed in cells of the central nervous system (CNS)^{8, 9}, suggesting a critical role for WWOX in CNS biology. However, the precise role of WWOX in CNS development and diseases is unknown.

In recent years, germline recessive mutations (missense, nonsense and partial/complete deletions) in the *WWOX* gene were associated with SCAR12 (spinocerebellar ataxia, autosomal recessive -12, OMIM 614322) and the WOREE syndrome (WWOX-related epileptic encephalopathy), the latter also known as early infantile epileptic encephalopathy-28 (EIEE28, OMIM 616211).^{10, 11} The severity of this disease was postulated to largely depend on the type of mutation and its effect on WWOX expression. For example, the most severe phenotype was observed in children with the WOREE syndrome harboring complete loss of WWOX and resulting in intractable seizures and prenatal or postnatal death.¹⁰ Individuals with SCAR12, mostly due to missense mutations in WWOX, displayed a milder phenotype including ataxia and epilepsy.^{10, 11} Recently, WWOX mutations were also found in patients with West Syndrome^{12, 13}, characterized by epileptic spasms with hypsarrhythmia. Brain magnetic resonance images (MRI) of children carrying mutations in WWOX revealed abnormalities in most cases including hypoplasia of the corpus callosum, progressive cerebral atrophy, delayed myelination and optic nerve atrophy.^{10, 14-18} How defects in WWOX lead to these neurological abnormalities is largely unknown.

Targeted deletion of murine Wwox and a spontaneous Wwox mutation in *Lde* rats phenocopied the complex human neurological phenotypes including epileptic seizures, growth retardation, ataxia and post-natal lethality^{11, 19-21}. To further shed light on the key cellular and molecular players in Wwox models and in the human disease, we conditionally generated mouse models harboring Wwox deletion in either neural stem and progenitors (using *Nestin-Cre;* N-KO), mature neurons (*Synaspin I-Cre;* S-KO), oligodendrocytes (*Olig2-Cre;* O-KO) or astrocytes (*GFAP-Cre;* G-KO), and studied their phenotypes and disease mechanisms, together with our previously described Wwox-null mice.^{19, 20} Remarkably, we found that deletion of Wwox either in neural stem and progenitors (N-KO) or neuronal cells (S-KO mice) exhibited severe epilepsy, ataxia and premature death by 3-4 weeks, recapitulating the phenotypes observed in the Wwox-null mice. Characterizing the molecular and cellular changes in S-KO mice uncovered marked hypomyelination and reduced number of mature oligodendrocytes resulting from a non-cell autonomous function of WWOX. Furthermore, modeling complete loss of WWOX in human embryonic stem cells (hESCs) and generation of human oligocortical spheroids (OS) further confirmed the role of WWOX in epilepsy and hypomyelination. These findings underscore the central role of WWOX in CNS biology and its therapeutic potential in curing WWOX-related neurological disorders.

### Materials and Methods

### Cell culture and plasmids

WiBR3 hES cells were maintained in 5% CO₂ conditions on irradiated DR4 mouse embryonic fibroblasts (MEF) feeder plates in FGF/KOSR conditions: DMEM-F12 (Gibco; 21331-020 or Biological Industries; 01-170-1A) supplemented with 15% Knockout Serum Replacement (KOSR, Gibco; 10828-028), 1% GlutaMax (Gibco; 35050-038), 1% MEM non-essential amino acids (NEAA, Biological Industries; 01-340-1B), 1% Sodium-pyruvate (Biological Industries; 03-042-1B), 1% Penicillin-Streptomycin (P/S, Biological Industries; 03-031-113), and 8ng/mL bFGF (Peprotech; 100-18B). Medium was changed daily, and cultures were passaged every 5-7 days by trypsinization with Trypsin type C (Biological Industries; 03-053-1B). For 24-48hr after trypsinization, hESCs were treated with Rho-associated kinase inhibitor (ROCKi, also known as Y27632) (Cayman; 10005583) at a 10µM concentration. For transfection of hESCs, cells were cultured in 10µM ROCKi 24h before electroporation. Cells were detached using Trypsin C solution and resuspended in PBS (with Ca²⁺ and Mg²⁺) mixed with a total of 100µg DNA constructs (px330 plasmid containing the sgRNA targeting exon 1 mixed in a 1:5 ratio with pNTK-GFP), and electroporated in Gene Pulser Xcell System (Bio-Rad; 250 V, 500µF, 0.4cm cuvettes). Cells were subsequently plated on MEF feeder layers in the above-mentioned conditions. 48hr-later, GFP-positive cells were sorted and subsequently plated sparsely (2,000 cells per 10cm plate) on MEF feeder plates for colonies isolation, ~10 days later.

### Mice

Generation of Wwox null mice was previously documented¹⁹ and these mice were maintained in FVB background. Mice carrying two *loxp* sites (*Wwox*^{*flox*/*flox*}) flanking Exon 1 of the Wwox genomic locus was previously documented²². In the current study, we used these mice to conditionally ablate WWOX expression in cells of CNS. Mice carrying transgenic *Cre* recombinase under the promoter of *Nestin* (original Jax line stock: 003771, B6.Cg-Tg(Nes-cre)1Kln/J) is a generous gift from Dr. Tal Burstyn-Cohen at the Hebrew University- Hadassah Dental School of Medicine. The *Synapsin I-Cre* (Stock:003966, *B6.Cg-Tg(Syn1-cre)671Jxm*/*J*), *Olig2-Cre* (Stock:025567, *B6.129-Olig2^{tm1.1l(cre)Wdr}*/*J*) and *GFAP-Cre* (*B6.Cg-Tg(Gfap-cre*)*77.6Mvs*/*2J*) mice lines were purchased from Jackson laboratory, USA. *Wwox*^{*flox*/*flox*} mice carrying a transgenic *Cre* recombinase considered as homozygous conditional deletion of *Wwox* and represented as N-KO *Wwox*^{*flox*/*flox*}; *Nestin-Cre⁺*), S-KO (*Wwox*^{*flox*/}*^{flox}; Synapsin-Cre⁺*)*,* O-KO *Wwox*^{*flox*/*flox*}; *Olig2*^{*cre*/*+*}) and G-KO (*Wwox*^{*flox*/}*^{flox}; Gfap-Cre⁺*). All mice were PCR-genotyped using specific primers by extracting tail/ear DNA. All the conditional models were maintained in C57BL6/J;129sv mixed genetic background. All conditional models contained *Rosa26-loxp-STOP-tdTomato* reporter allele. Animals were maintained in a SPF unit at 12h -light/dark cycle with ad libitum access to the food and water. All animal related experiments were performed in accordance and prior approval with the Hebrew University- Institutional Animal Care Use Committee (HU-IACUC).

### DRG-OPC co-culture

Co-culture experiments of DRG neurons and OPCs were done following a previously published protocol ²³. Briefly, DRG neurons were isolated from mouse embryos at E13.5. Embryos were genotyped and the DRGs were collected in cold L-15 medium. Tissues were dissociated in 0.25% trypsin, triturated, centrifuged, and re-suspended in NB medium (Neurobasal, B27 supplement, 0.5 mM L-glutamine, and penicillin-streptomycin). Pre-cleaned 13mm diameter glass coverslips were placed in 4-well dishes and coated with Matrigel (1 hr at RT) then poly-D-lysine (30 min at RT) prior to dissection. Cells were plated at a density of 40,000 cells/13mm coverslips in NB medium and maintained in a humidified incubator at 37°C and 5% CO₂. Cultures were treated with fluorodeoxyuridine at DIV2, 4 and 6 to eliminate non-neuronal cells. Fifty percent of cell media was replaced every third day and OPCs were added on DIV15. OPCs were isolated from mouse pups aged P0-P2, respectively. Cortices were isolated in ice-cold L-15 medium and dissociated using syringe (19G followed by 21G for mouse tissue), triturated, centrifuged, and re-suspended in glial plating medium (DMEM containing 10% fetal bovine serum, penicillin streptomycin) on PDL-coated flasks. Glial cells were maintained in a humidified incubator at 37°C and 5% CO₂, and fifty percent of cell medium was changed every third day. At DIV10 OPCs were isolated by shaking the flasks vigorously, followed by depletion of astrocytes by fast adhesion to culture dishes (10 min at 37°C x3) or purified OPCs (200,000/coverslip) were seeded on DRG neuronal cultures and maintained in co-culture medium (DMEM containing B27 and N2 supplements, 5mg/ml N-Acetyl-Cysteine, 5mM forskolin, penicillin-streptomycin). The medium was changed every other day for 9-11 days, then cultures were fixed and stained for analysis.

### Oligocortical Spheroids Generation and Culture

Cerebral organoids were generated from hESCs as previously described.²⁴ Briefly, Human WiBR3 cells were maintained on mitotically inactivated MEFs. 4-7 days before protocol initiation, cells were passaged onto MEF-coated 60mm plates and grown until 70-80% confluency was reached. On day 0, hESCs colonies were detached from MEFs with 0.7mg/ml collagenase D solution (Sigma; 11088858001) and dissociated to single cell suspension with Trypsin type C for 2 minutes. After dissociation, cells were counted and suspended in hESCs medium, composed of DMEM/F12 supplemented 20% KOSR, 1% GlutaMax, 1% NEAA, 1% P/S and 100µM 2-mercaptoethanol (Sigma; M3148), supplemented with 10µM Dorsomorphin (Sigma; P5499) or 100nm LDN-193189 (Axon medchem; Axon1509), and 10µM SB-431542 (Sigma; S4317) and 10µM Rocki, sterilized through 0.22µm filter. 10,000 cells were seeded in each well of an ultra-low attachment (ULA) 96 v-well plates (S-Bio Prime; MS-9096VZ) for embryoid bodies (EBs) formation. EBs were fed every day by aspirating and replacing half of the medium (~100µl per well) up to day 6, with fresh addition of Dorsomorphin/LDN-193189 and SB-431542.

At days 7-50, all used mediums were based upon Neural Medium (NM), composed of Neurobasal medium (Gibco; 21103049 or Biological Industries; 06-1055-110-1A), 2% B27 supplement (Gibco; 17504044), 1% GlutaMax, 1% P/S, and from day 27, 1% Matrigel (Corning; 356231) sterilized through 0.22µm filter. At day 7, ~75% of the medium was replaced with EB-expansion (EBX) medium, composed NM, supplemented with 20ng/ml FGF-2 and 20ng/ml EGF (Peprotech; AF-100-15). Half of the EBX medium was changed every day until day 15, after which half of the medium was changed every other day. Around day 20, the spheroids over-grew the 96-well plate, and therefore were transferred to a 24-well ULA (Corning; 3473), with half-medium changes every other day up to day 26. At day 27, spheroids were moved to 90mm sterile, non-treated, culture dishes (Miniplast; 825-090-15-017) and the medium was changed to Neuro-differentiation medium (NDM), composed of NM supplemented with 20ng/ml BDNF (Peprotech; 450-02) and 20ng/ml NT-3 (Peprotech; 450-03). At day 41, medium was changed to NM without supplementation.

From day 51 onwards, all mediums were based on Oligo Maturation Medium (OMM), containing Neurobasal medium supplemented 1% B27 supplement, 1% GlutaMax, 1% P/S, and 1% Matrigel with half medium changes every two days. For OPCs expansion, the medium was changed on day 51 to OPCs expansion medium (OEM), composed of OMM supplemented with 10ng/ml PDGF-AA (R&D systems; 221-AA) and 10ng/ml IGF-1 (R&D systems; 291-G1), with half medium changes every two days. For oligodendrocyte differentiation, OMM was supplemented with 40ng/ml T3 (Sigma; T2877), making up the oligo differentiation medium (ODM). Finally, from day 71 onward, spheroids were cultured in OMM, with complete medium changes every two days.

Throughout the protocol, spheroids were cultured at static conditions at 37°C and 5% CO₂, growth factor and cytokines were added freshly before medium changes, and the spheroids were transferred into a fresh plate at least once every 30 days. For all analysis, organoids from the same batch were used, unless stated otherwise.

### Immunofluorescence

Mice from different genotypes (P17-P18) were euthanized by CO₂ and transcardially perfused with 2% PFA/PBS. Dissected brains were post fixed on ice for 30min. For immunofluorescence brains were incubated in 30% sucrose at 4°C overnight then embedded in OCT and sectioned (12-14µm) using cryostat. Sagittal sections were washed with PBS and blocked with 5% goat serum containing 0.5% of Triton X-100 then incubated for 1h at room temperature followed by incubation with primary antibodies for overnight at 4°C. Then, sections were washed with PBS and incubated with corresponding secondary antibodies tagged with Alexa fluorophore for 1 hr at room temperature followed by washing with PBS and mounted with mounting medium.

Oligocortical spheroids fixation and immunostaining were performed as previously described ²⁵. Briefly, organoids were washed three times in PBS, then transferred for fixation in 4% ice-cold paraformaldehyde for 45 min, washed three times in cold PBS, and cryoprotected by over-night equilibration in 30% sucrose solution. The next day, spheroids were embedded in OCT, snap frozen on dry ice, and sectioned at 10µm by Leica CM1950 cryostats. For immunofluorescent staining, sections were warmed to room temperature and washed in PBS for rehydration, permeabilized in 0.1% Triton X in PBS (PBT), and then blocked for 1hr in blocking buffer containing 5% normal goat serum (NGS), 0.5% BSA in PBT. The sections were then incubated at 4°C overnight with primary antibodies diluted in blocking solution. The day after, sections were then washed in 3 times while shaking in PBS containing 0.05% Tween-20 (PBST) and incubated with secondary antibodies 1.5hr. Slides were washed four times in PBST while shaking and then mounted using immunofluorescence mounting medium (DAKO; s3023).

### Luxol Fast Blue staining

Luxol Fast Blue (LFB) staining was preformed following previously published protocol²³ using Nova Ultra luxol fast blue stain kit. Briefly, paraffin embedded brain sections (6 µm) from at least three mice of each genotype were dewaxed followed and rehydrated to 95% ethanol after which sections were incubated in LFB solution (0.1% LFB in 95% ethanol/0.5% acetic acid) overnight at 56°C. Sections were then washed in 95% ethanol and ddH₂O followed by 0.05% lithium carbonate for 30s, and then with 70% ethanol until the gray matter was colorless and white matter appeared blue. Sections were then rinsed in ddH₂O before counterstaining with preheated 0.1% Cresyl Violet acetate solution for 30-40s. Finally, sections were rinsed in ddH2O, dehydrated with 100% ethanol and xylene and mounted with resinous medium.

### Electron microscopy

Mice were anesthetized and perfused with a fixative containing 4% PFA, 2.5% glutaraldehyde, and 0.1 M cacodylate buffer. Brains were isolated and incubated in the fixative overnight at room temperature and processed as previously described.²⁶ Samples were examined using a FEI Tecnai T12 transmission electron microscope or Tecnai F20 S/TEM equipped with a XF416 TVIP camera or a US4000 Gatan camera, respectively. EM micrographs were analyzed using computer-assisted ImageJ analysis software. To calculate g-ratio, myelinated axons (~600, 100 axons per mouse, n = 3 per genotype) from EM images either from corpus callosum or optic nerve were analyzed, by dividing inner axonal diameter over the total axonal diameter.

### Image acquisition and analysis

LFB stained sections were imaged using panoramic digital slide scanner (3DHISTECH). Immuno stained sections were imaged using panoramic digital slide scanner or Olympus FV1000 confocal laser scanning microscope or Nikon A1R+ confocal microscope. Fluorescence sum intensity of CNP and MBP staining in cortex and cerebellum was calculated using NIS elements software. The acquired images are processed using the associated microscope software programs namely CaseViewer, F-10-ASW viewer, NIS elements. Images were analyzed using ImageJ software. Images were analyzed while blinded to the genotype and the processing include the global changes of brightness and contrast.

### Spontaneous seizure recordings

Mice undergoing spontaneous seizures were recorded using mobile camera, when monitoring the mice at the animal facility. Spontaneous seizures or abnormal activity (wild running) were observed in *Wwox* mutant mice. Duration of spontaneous seizures (in seconds) was calculated and presented, n = 6.

### Electrophysiology

Electrophysiological recordings were performed in mice with conditional deletion of *Wwox* in neurons using *synapsin-Cre* recombinase. S- Control (*Wwox*^{*+*/*+*}; *Synapsin-Cre⁺*), S-HT (*Wwox*^{*+*/*flox*}; *Synapsin-Cre*⁺) and S-KO (*Wwox*^{*flox*/}*^{flox}; Synapsin-Cre⁺*) either male or female mice aged P13 to P17 were humanely killed for these experiments in accordance with the guidelines outlined by the Canadian Council of Animal Care (CCAC). All surgical procedures were approved and done in accordance with the guidelines of the Animal Care Committee of the University Health Network.

*In vivo preparation.* Mice were injected intraperitoneally with ketamine - xylazine (100mg/kg ketamine with 10mg/kg xylazine) dissolved in phosphate-buffered saline (PBS). The pedal reflex was used to determine the depth of anesthesia. Once the mouse was deeply anaesthetized, it was positioned into a stereotaxic frame. A local anesthetic (lidocane) was injected just above the skull at the site of incision, then, after a brief period (~5min), the skin was removed, and the skull exposed. A drill was used to score the skull, then a set of forceps was used to peel it back to reveal the cortical tissue. Thin walled glass electrodes (1.5 diameter, World Precision Instruments) were pulled using a vertical puller. These were filled with PBS. The electrode was positioned at 1.6-2mm posterior to the bregma and 4mm lateral to the midline. The electrode was positioned at multiple depths, for 3 min at each depth, to record neocortical subcortical brain and hippocampi activity.

*In vitro preparation.* Mice were anesthetized with pentobarbital (50mg/kg). Depth of anesthesia was tested using the pedal reflex. Once the mice were deeply anaesthetized, they were swiftly decapitated and the brain was removed. The cerebellum and olfactory bulbs were removed and the remainder of the tissue was placed caudal-side down onto a platform in a solution of ice-cold sucrose containing (in mM): 248 sucrose, 26 NaHCO₃, 10 glucose, 2 KCl, 3 MgSO₄-7H₂O, 1.25NaH₂PO₄, 1CaCl₂-2H₂O. The neocortex was sectioned coronally 400-500 um thick (0.6mm/s speed, 1mm amplitude) using a Leica 1200 vibratome. After this, slices were incubated in artificial cerebral spinal fluid (ACSF) containing (in mM): 123 NaCl, 25 NaHCO₃, 10 glucose, 3.5 KCl, 1.3 MgSO₄-7H₂O, 1.2 NaH₂PO₄, 1.5 CaCl₂-2H₂O, pH 7.3-7.4. Slices were kept at 34°C for 30min, after which they were removed to room temperature for at least 60min prior to experimentation. Local field potential (LFP) glass electrodes (1.5mm, World Precision Instruments) containing the ACSF were pulled using a vertical puller (Narishige, Japan PP-83) and positioned in neocortical layers II and III or in the CA3 region of the hippocampus. An Olympus BX51 microscope (OLY-150IR camera-video monitor unit) was used as guidance for proper electrode placement. To assess network excitability, layer V cortex or dentate gyrus stimulation was performed using a bipolar concentric tungsten electrode positioned along the same vertical column as the recordings from layers II/III. Current pulses of 0.1ms duration with varying strengths were applied every 30s using a GRASS S88 stimulator connected to a photoelectric stimulus isolation unit. The amplitude of the maximal steady state response for S-Control, S-HT and S-KO mice (100µA) were compared.

### Power Spectral analysis

First the data was decimated so that the final sampling frequency was 1000Hz. Next, the data was notch filtered at 60Hz and its harmonics. The spectral power was analyzed using a fast Fourier transform and bin sizes of 1Hz in MATLAB. For each animal, we averaged the power spectrum over 2.5 min at 300 µm depth using 10s windows with 5s overlap. The power spectrum was plotted as the average across all subjects.

### Electrophysiology recordings from oligocortical spheroids

Organoids at indicated time points were embedded in 3% low temperature gelling agarose (at ~36°C) and incubated on ice for 5 minutes, after which they were sliced to 400µm using a Leica 1200S Vibratome in sucrose solution (in mM: 87 NaCl, 25 NaHCO₃, 2.5 KCl, 25 Glucose, 0.5 CaCl₂, 7 MgCl₂, 1.25 NaHPO₄, 75 Sucrose) at 4°C. Slices were incubated in artificial cerebrospinal fluid (ACSF, in mM: 125 NaCl, 25 NaHCO₃, 2.5 KCl, 10 Glucose, 2.5 CaCl₂, 1.5 MgCl₂; pH 7.38, 300mOsm) for 30 minutes at 37°C, followed by 1 hour at room temperature. During recordings, slices were incubated in the same ACSF at 37°C with perfused carbogen (95% O₂, 5% CO₂), in baseline condition. Local field potential (LFP) and whole-cell patch clamp recordings were done using electrodes pulled from borosilicate capillary glass and positioned 150µm deep from the outer rim of each slice (see Figure 6A). LFP electrodes were filled with ACSF, while patch electrodes were filled with internal solution. Data was recorded using MultiClamp software at a sampling rate of 25,000 Hz. Data was analyzed using MATLAB software. Traces were filtered using (1) 60 notch filter (with 5 harmonics) to eliminate noise and (2) 0.1 Hz high-pass IIR filter to eliminate fluctuations from the recording setup.

### Statistical analysis

All graphs or statistical analysis was preformed using either Excel or GraphPad Prism 5. Results of the experiments were presented as mean ± SEM. The two-tailed unpaired Student's t-test was used to test the statistical significance. Results were considered significant when the *P<* 0.05, otherwise they were represented as ns (no significance). Data analysis was performed while blinded to the genotype.

### Results

### Conditional deletion of Wwox in neural stem/progenitor cells or neuronal cells recapitulate the Wwox null phenotype

Wwox-null mice are born with Mendelian ratio and are indistinguishable from wild type littermates.^{19, 20} Within a few days after birth, mice start to show signs of growth retardation and seizures until they succumb by 3-4 weeks of age (Fig. 1A-C). These phenotypes very much resemble what is observed in WOREE/EIEE28 patients who mostly die between ages 2-4 years.¹⁰ To better understand the precise function of WWOX in CNS, we generated conditional mouse models of the brain considering the high expression of WWOX during embryonic brain development in different regions.⁸ To achieve this, we crossed *Wwox*^{*fl*/*fl*} mice (carrying *loxP* sites)²² with transgenic mice carrying *Cre* recombinase under the promoter and enhancer of rat Nestin *(Nes)²⁷,* which is expressed at embryonic stage E10.5 and facilitates WWOX ablation in neural stem/progenitor cells of the murine brain generating N-KO mice. WWOX ablation was validated in N-KO brain using immunofluorescence. Monitoring N-KO mice revealed their resemblance to the *Wwox* null mice phenotypes. N-KO mice were born in Mendelian ratio and presented no gross abnormalities until 2-3 days after birth when they started to show global developmental delay (Fig. 1D, E) that was evident at P7. Furthermore, N-KO mice showed tremors/seizures and ataxia (lack of coordination in hind limb clasping test) as observed in *Wwox* null mice. All (100%) of the N-KO mice died postnatally by 4 weeks of age (Fig. 1F). Mice carrying one intact allele of *Wwox (Wwox*^{*+*/}*^{fl},* Nestin-Cre+) did not show any visible abnormal phenotype which is in accordance with absence of neurological symptoms in heterozygous carriers in human individuals. These phenotypes indicate a crucial role for WWOX in the CNS and imply that its ablation in Nestin-positive cells and their progenies is responsible for the complex phenotype observed in WWOX-rodent models and human patients.

Since *Nestin* promoter is expressed in neuronal and glial cell precursors, we dissected the effect of WWOX ablation separately in neurons and glial cells. First, we conditionally deleted *Wwox* in neuronal cells using a transgenic mouse line carrying a *Cre* recombinase under the promoter of Synapsin I gene²⁸, which is expressed at E12.5 and results in *Wwox* deletion particularly in most of the differentiated neurons. Specific WWOX ablation in neurons, but not in other cells, was validated by observing intact WWOX levels in oligodendrocytes (OLs) (stained with CC1) of S-KO brain tissues comparable to control mice. Phenotypic analysis of conditional ablation of WWOX in neuronal cells revealed growth retardation (Fig. 1G, H), and premature death (Fig. 1I) by the age of 3-4 weeks, phenocopying the N-KO and *Wwox* null mice. S-KO mice also exhibited uncontrolled spontaneous tonic-clonic seizures starting at P9 and ranging from several seconds to few minutes (68.5 ±13.4 seconds; *n* = 6 of P14-P18). In addition, these mice displayed lack of coordination and ataxic phenotype. The heterozygote (*Wwox*^{*+*/fl}*, Synapsin Cre+)* carrying one intact allele of *Wwox* did not show any abnormal phenotypes and were behaviorally indistinguishable from control mice. The phenotypes of the S-KO model strongly indicate that WWOX plays an essential role in neurons and its deficiency leads to a dramatic neurological phenotype.

To confirm the specific neuronal WWOX function, we examined the consequences of ablating WWOX expression in oligodendrocytes (using *Olig2-Cre*^{*+*/*-*})²⁹ and astrocytes (using *GFAP-Cre*)³⁰ and observed no phenotype abnormalities (Fig. 1J-O). Validation of conditional deletion of WWOX in oligodendrocytes and astrocytes is shown by immunofluorescence staining for CC1 and GFAP respectively. Altogether, these findings indicate that although WWOX is expressed by astrocytes, oligodendrocytes and neurons, its function in neurons is crucial for animal survival and CNS homeostasis.

### Neuronal deletion of Wwox results in epileptic seizures

To characterize the epileptic activity of the S-KO brains, we performed electrophysiological recordings from their brains along with controls (S-Control) and heterozygotes (S-HTs) (Fig. 2A-F). For this, we placed the mice under anesthesia and performed a craniotomy to expose a region of their brain above the sensory cortex. We passively recorded at varying depths of the cortex with a local field potential (LFP) electrode containing saline. Specifically, in the superficial layer of the neocortex, the S-KO displayed large amplitude bursting activity that was not present in S-Control (Fig. 2A). These bursts were not observed in subcortical structures, suggesting a neocortical origin of burst generation. Further characterization of the activity at 300µm recording depth, *in vivo,* showed a median epileptiform burst duration of 350ms (206 to 472ms, 25^{th} and 75^{th} percentiles), with a peak-to-trough amplitude of 2.06mV (2.01 to 3.09mV, 25^{th} and 75^{th} percentiles), and an inter-burst interval of 8.62s (6.25 to 21.27s, 25^{th} to 75^{th} percentile).

These bursts were also observed in LFP recordings obtained from acute neocortical brain slices (Fig. 2B), showing that the neocortex was a generator of these abnormal burst discharges. In total, 0% S-Control slices showed bursting (0 of *n*=11 slices across 7 animals), 17% of slices from S-HT animals showed bursting (4 of n=23 slices across 14 animals) and 86% of slices from S-KO animals showed bursting (36 of *n*=42 slices across 24 animals).

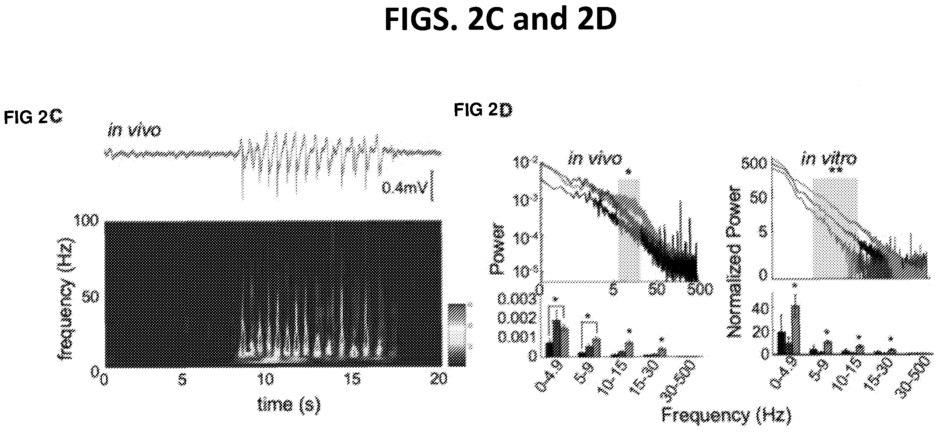

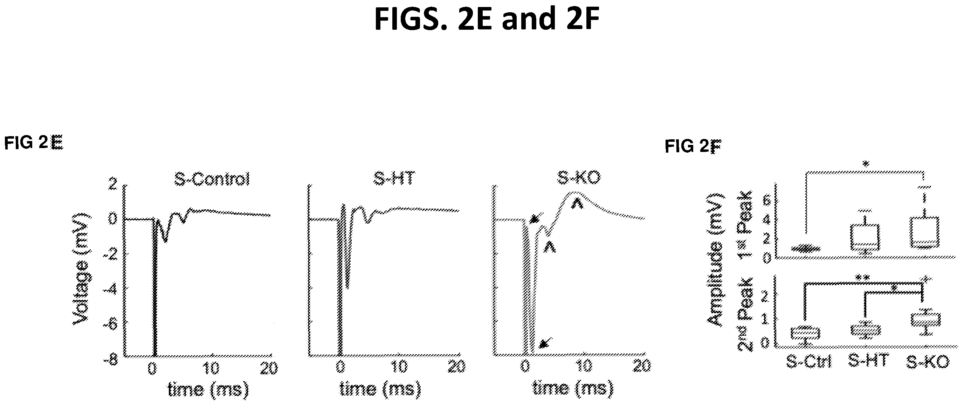

S-KO mice exhibited large amplitude activity, which is attributed to increased excitability of their neocortical circuitry. To assess this, we performed power spectral analysis on the spontaneous field activity (Fig. 2C, D) and recorded the layer II/III field response of the neocortex to electrically evoked stimuli in layer V (Fig. 2E, F). The traces that had spontaneous activity showed increased spectral power within a broad band of frequencies, encompassing delta (<5Hz), theta (5-9Hz), alpha (10-15Hz), and beta (15-30Hz) rhythms both *in vivo* and *in vitro* (Fig. 2C, D). Furthermore, the electrophysiological evoked field responses, *in vitro,* were larger in the S-KOs as compared to the S-Controls and S-HTs (Fig. 2E, F). Since a larger evoked response and increased spectral power are biomarkers for hyperexcitability, these results demonstrated enhanced neocortical excitability of the S-KO mice, with the *in vitro* data suggesting increased innate cortical hyperexcitability.

### RNA-seq and single-nucleus RNA-seq (snRNA-seq) revealed transcriptomic changes of myelination and cellular alternations in Wwox mutant models

To dissect the molecular alterations which underlie the observed phenotypes upon neuronal deletion of WWOX, we performed bulk RNA sequencing (RNA-seq) from whole cortex and hippocampi of S-KO and S-Control mice at P17. Our analysis revealed a total number of 730 upregulated and 579 downregulated genes (*P* value <0.01, fold change >1.5) between the two genotypes highlighting differential expression of several known genes specific for oligodendrocytes and genes elicited during epileptic insults including activation of astrocytes^{31, 32}. Importantly, Gene Ontology (GO) term analysis indicated a significant downregulation of genes associated with myelination and ensheathment of neurons. Gene set enrichment analysis (GSEA) also revealed enrichment of genes associated with myelination are downregulated in S-KO as compared with S-Control mice. In-depth analysis of RNA-seq data from the cortex and hippocampus of the S-KO mice, compared to S-Control, showed significant downregulation of genes involved in maturation (*Gjb1, Gjc2* and *Olig1*) myelin development, maintenance and functionality of oligodendrocytes (OLs)^{31, 33-36} (*Ermn, Ugt8a, Plp1, Otud7b, Mal, Eml1, Mobp, Hist1h2be, Cldnll, Mbp, Gal3st1, Fa2h, Gsn, Adamts4, Cnp, Mog, Oplalin, Enpp, Mag* and *Myrf.* These findings implied that neuronal ablation of WWOX could impact the myelination process.

To test whether the observed molecular changes are directly linked with WWOX function and to further validate our results, we performed bulk RNA-seq on whole hippocampal tissues from the *Wwox*-null and N-KO models and compared them with the S-KO model. Unsupervised clustering analysis of the three models revealed significant downregulation of myelin associated-genes and those involved in OL development, maturation, and myelination process, while grouping the samples according to its WWOX status rather than its Cre-recombinase's promotor. The top 25 DEGs indeed showed downregulation of transcripts associated with myelination including *Cnp, Ugt8a, Plpl, Ermn, Otud7b, Mettl7a1, Prr18, Adamts4, Klhdc7a, Mobp, Cldn11* and *Mbp.* GSEA and GO term analysis showed significant negative FDR values associated with myelination, ensheathment of neurons and axon ensheathment.

To further dissect the molecular effect of neuronal loss of WWOX, we performed single-nucleus RNA-seq (snRNA-seq) analysis on hippocampi of S-KO and S-Control mice. Different cell population clusters were identified based on the expression levels of gene-sets specific to each cell type or the subtype ³⁷⁻⁴¹. Uniform Manifold Approximation and Projection (UMAP) analysis revealed reduced number of matured myelinating oligodendrocyte cells (15%), COPs (committed oligodendrocyte progenitors) (68%) and a greater number of oligodendrocytes progenitor cells (OPCs) (150%) in S-KO compared to S-Control. Altogether, both bulk RNA-seq and snRNA-seq analyses uncovered an impaired maturation of oligodendrocytes indicating a potential non-cell autonomous effect of neuronal deletion of WWOX.

### Neuronal WWOX ablation results in hypomyelination, reduced oligodendrocyte maturation and impaired axonal conductivity

We next investigated whether transcriptomic changes of OL specific genes affects myelination in S-KO mice brain and the other *Wwox* mutant models. To test this, sagittal sections of brains obtained at P18 were immunostained for myelin markers like CNPase (CNP) and myelin basic protein (MBP). Immunofluorescence analysis showed prominent reduced staining of both the myelin markers indicating hypomyelination in S-KO brain tissues compared with the age-matched S-Control. Reduced myelin staining was seen in different regions of the brain including cortex, cerebellum, caudate putamen, fimbria and fornix of S-KO compared to S-Control. Quantification of fluorescence intensity of the CNP and MBP staining revealed significant reduction in cortex and cerebellum of S-KO (Fig. 3A, C). Moreover, Luxol fast blue (LFB) staining of S-KO brains at the age of P18 showed hypomyelination in the white matter tracks compared with age matched controls. In addition, expression levels of OL maturation and myelination genes were decreased in S-KO, as assessed by quantitative real time PCR (qRT-PCR) in cortex at P18 as compared to the corresponding control tissues. Notably, early OPC marker genes, such as *Pdgfra* and *Cspg4* were slightly higher, suggesting a defect specific to maturation of OLs.

Next, we assessed the cellular changes that are associated with hypomyelination in brain tissues of S-KO. Immunostaining with CC1 (marker of mature OLs) and anti-PDGFRα (OPCs) showed a 2-fold reduction in CC1 positive cells and significantly more OPCs in the corpus callosum (Fig. 3E) of S-KO brains compared to the age matched controls. Furthermore, we immunostained brain sections for NG2 and CC1, to test transition of OPCs to matured OLs, and found decreased number of double positive cells in corpus callosum and cerebellum of S-KO tissues further confirming defects in OPCs' differentiation into mature OLs. Of note, no significant OLs cell death was observed in S-KO tissues when stained for CC1 and cleaved caspase 3. Similar results of hypomyelination and defects in OL maturation were observed in brain tissues of N-KO and *Wwox* null brain tissues.

To further evaluate the hypomyelination phenotype observed in S-KO mice, we performed electron microscopy of the corpus callosum and optic nerve at P17. Electron micrograph images demonstrate substantial reduction (~3.5-4 folds) in number of myelinated axons in the corpus callosum and significantly a greater number (~6 folds) of unmyelinated axons in optic nerves of S-KO as compared to age-matched S-Control (Fig. 4A). The number of myelinated axons (S-Control, average=180±40, S-KO, average=55±35) in corpus callosum and unmyelinated axons (S-Control, average=55±20, S-KO, average=270±60) in optic nerve is counted per field of view (FOV) and presented in Fig. 4B. Furthermore, we calculated g-ratio of the myelinated axons and observed significantly higher ratio in S-KO corpus callosum and optic nerve as compared to the age matched control hence implying reduced myelin thickness (Fig. 4C).

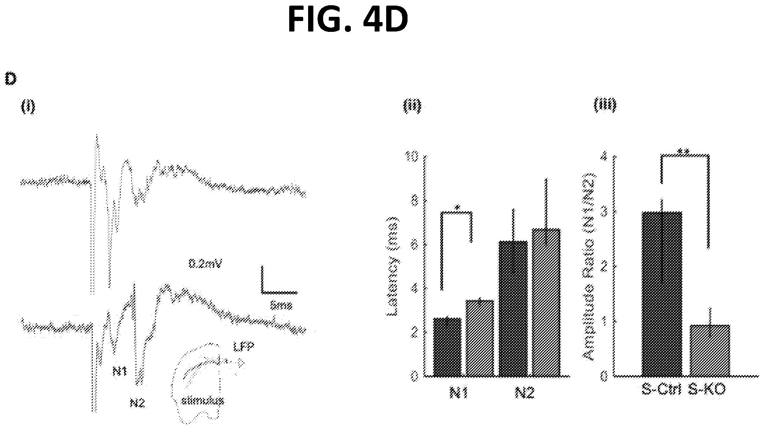

To test whether the observed hypomyelination could delay axonal conductivity and lead to functional deficits ^{42, 43}, we stimulated the corpus callosum and recorded from neocortical layer V in the *in vitro* slice preparation. We found a significant latency of neural conductivity in S-KO compared to that of control (Fig. 4D-i), consistent with impaired myelination. As seen in Fig. 4D, the amplitude ratio of N1 and N2 showed a longer response latency of axonal propagation (Fig. 4D-ii, iii) and significantly lower peak amplitudes of the evoked potentials.

### Neuronal WWOX promotes the differentiation of OPCs to mature oligodendrocytes

So far, our results imply that ablation of WWOX in mature neurons results in hypomyelination due to impaired differentiation of OPCs. To further test whether a non-cell autonomous function of neuronal WWOX regulates the differentiation of OPCs to matured OLs *in vitro,* we performed co-culture assay between wild type OPCs with dorsal root ganglion (DRG) neurons isolated either from WT or *Wwox* null mice. Remarkably, OPCs that were cultured with *Wwox* null DRGs displayed significantly reduced differentiation into myelinating OLs compared to OPCs that were seeded over WT-DRGs (Fig. 5). Interestingly, we observed increased number of pre-myelinating OLs under these conditions likely suggesting a compensatory effect (Fig. 5).

These results promoted us to determine whether *Wwox* specific deletion in oligodendrocytes (O-KO) leads to changes in myelination at early post-natal age. To this end, we examined MBP staining in O-KO and control littermates at P17 and found no major changes. Overall, these findings indicate that neuronal WWOX ablation results in hypomyelination, likely a result of impaired differentiation of OPCs into mature oligodendrocytes.

### Modeling WWOX deletion in human oligocortical spheroids reveals hyperexcitability and hypomyelination

We next sought to assess the human relevance of our findings by modelling WWOX loss in embryonic stem cells (hESC) and generating human brain organoids, known as oligocortical spheroids (OS). These spheroids are composed of functional neurons and glia that models the brains' cytoarchitecture and inter-populational interactions and mimic the natural myelination process observed in humans. To study the effects of WWOX loss on the human neuronal activity and myelination, we utilized the CRISPR/Cas9 system to knock-out *WWOX* in WiBR3 hESC line. OS from both WT (OS-WT) and WWOX-KO hESCs (OS-WWOX-KO) were generated according to a recently published protocol²⁴.

To characterize the functional properties of OS, we performed whole-cell patch and local field potential recordings (LFP) in 15-week-old organoid slices (as described in Methods). An LFP and patch electrode were positioned 10-15 µm apart to compare field and single-cell recordings (Fig. 6A). We found the resting membrane potentials (RMP) of patched cells from both OS-WT and OS-WWOX-KO lines to be less negative than typical mature neurons, suggesting that these cells are still in the process of development (Fig. 6B). As previously noted by Pre et al⁴⁴, the RMP of iPSC-derived neurons becomes more negative during maturation - reaching -50 mV in healthy cells at days 48 to 55, when grown in 2D culture. Comparatively, our spheroids demonstrated a similar RMP at around day 105 (week 15). The difference in time taken to reach -50 mV is likely due to differences in development between 3D spheroids and 2D cultures. OS-WWOX-KO cells displayed significantly more depolarized RMP than their WT counterparts, suggesting a developmental delay in the OS-WWOX-KO. From LFP recordings, we observed a prominent peak in the low-frequency ranges, 0.5-7.9 Hz. The oscillatory activity was quantified by the area under the curve (Fig. 6C, D), which was significantly higher for the OS-WWOX-KO over the frequencies of interest. Differences in signal characteristics were also observed, as shown in sample recordings and their corresponding time-frequency spectrograms. Overall, there is an increase in low-frequency activity, particularly in the delta and theta ranges in the OS-WWOX-KO, which is not present in OS-WT. Increased low-frequency activity is generally attributed to seizure-like, epileptiform activity⁴⁵, elucidating early signs of the OS-WWOX-KO phenotype in developing spheroids, and consistent with data from human patients and the Wwox-null mouse phenotype.

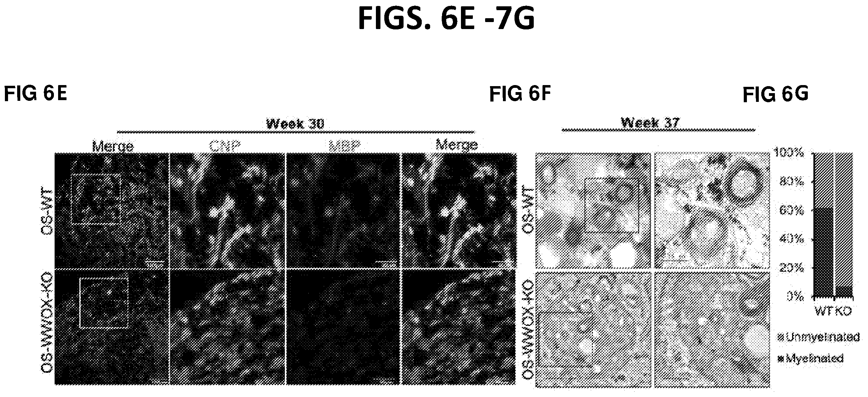

To assess the oligodendrocyte status, we followed the timeline for oligodendrocyte and myelin development and maturation as previously described.²⁴ First, we immunostained for CC1 and anti-PDGFRα in week 14 OS, the first time point in which mature OLs are observed. Although staining revealed similar proportions of OPCs, the number of CC1⁺ was decreased in OS-WWOX-KO compared to OS-WT. We next examined OLs and OPCs at week 20, the first time point in which myelin is expected by staining for the myelin protein CNP and the OPCs marker NG2. We found a prominent decrease in CNP⁺ cells, which was not apparent in NG2⁺ cells, suggesting both diminished numbers of OLs and hypomyelination. Finally, we examined week 30 OS, a time point in which compact and mature myelin was described.²⁴ In OS-WT, WWOX expression was prominent in Tuj⁺ cells. At this stage, OS-WWOX-KO displayed significantly reduced staining of both MBP and CNP (Fig. 6E), which was also supported by transcript levels. Interestingly, we found RNA levels of earlier markers for OPCs, such as *SOX10,* and *PDGFRα* are slightly increased in OS-WWOX-KO. Furthermore, electron micrograph images indicated more myelinated axons in OS-WT compared to the OS-WWOX-KO when examined at 37 weeks (Fig. 6F, G). Overall, these findings in our human model are in agreement with the mouse data regarding the implications of WWOX loss in the brain, supporting its critical role in the physiological development of myelin and the pathological development of epilepsy.

### Discussion

Several lines of evidence suggest that WWOX plays an important role in maintaining brain homeostasis, however the precise cellular roles of WWOX in CNS remain to be elucidated. In the current study, we unveiled a previously unknown cellular role of WWOX that is attributed to the complex phenotype.

WWOX expression is found in neurons, oligodendrocytes and astrocytes in the CNS.⁴⁶ To identify the type of cells contributing to the defects in the *Wwox* null mice we decided to systematically mutate the gene in different neural populations. Conditional deletion of WWOX in either neural stem cells and progenitors or matured neurons reproduced the *Wwox* null phenotypes including growth retardation, epileptic seizures, ataxia and premature death. Consistent with the documented EEG recording from WWOX patients^{10, 13-18}, we found that neuronal deletion of WWOX is associated with hyperexcitability and spontaneous epileptic activity in the neocortex.

One of most striking finding in the analysis of neuronal deletion of WWOX is the marked hypomyelination phenotype. This remarkable phenotype was initially observed using bulk RNA-seq and snRNA-seq, the later limited to abundant nuclear transcripts. These observations were further confirmed using immunofluorescence and electron micrographs of different tissue compartments. Overall, these results are in accordance with the documented delayed myelination at the white matter tracks and thin corpus callosum in most WOREE children observed by MRI images.^{10, 13-18} At the cellular level, we found that this myelination defect is the result of reduced number of matured OLs. This reduction likely represents a differentiation defect since we also detected an increased number of OPCs in neuronal-specific *Wwox* knockout brains. Intriguingly, we present evidence, both *in vitro* and *in vivo,* of the non-cell autonomous function of WWOX in positively regulating differentiation of OPCs into mature and myelinating OLs.

To further validate that WWOX function is mostly critical for neurons we ablated *Wwox* specifically in oligodendrocytes or astrocytes. Interestingly, *Wwox* deletion in either cell type did not lead to any of these phenotypes. Together these findings indicate that a non-cell autonomous role of WWOX in neurons somehow impacts the differentiation of OPC into oligodendrocytes, though we cannot exclude the cell autonomous functions of WWOX in oligodendrocytes or astrocytes in other neurological disorders. Altogether our findings underscore the crucial and novel role of neuronal WWOX in CNS biology.

Our results clearly demonstrate that neuronal hyperexcitability and hypomyelination are major defects in *Wwox* mutant mice. These two phenotypes are not necessarily mutually exclusive. In a recent review of white matter imaging in epilepsy, it was proposed that neurological disorders associated with an abnormal myelin content are accompanied by a higher susceptibility to epileptic seizures.⁴⁷ Demyelination or hypomyelination is indeed a common finding in intractable pediatric epilepsies and in animal epilepsy models.⁴⁸ Why hypo- or demyelination should cause local hyperexcitability remains unclear. We believe that WWOX deficiency could impact several functions of the CNS, including imbalance of neuronal activity and myelination leading to the observed complex phenotype of WOREE syndrome.

Differentiation of oligodendrocytes from OPCs is orchestrated by a multitude of intrinsic and extrinsic factors in the CNS.⁴⁹⁻⁵¹ Increasing evidence shows that neuronal activity and glutamate signaling can promote OPC migration, proliferation, differentiation, and myelination during development.^{52, 53} It remains to be seen if neuronal WWOX impacts oligodendrocytes differentiation by neuronal activity or by an alternative mechanism. WWOX was found to regulate many signaling pathways including Wnt/β-catenin⁵⁴⁻⁵⁶, TGFβ/SMAD^{57, 58} and DNA damage response^{6, 59} through its physical interactions with key proteins so whether WWOX loss of function could deregulate these critical pathways and affect CNS homeostasis remains to be explored.

In recent years, brain organoids have gained a lot of interest by the scientific community due to their capability to model human diseases.⁶⁰⁻⁶⁴ Modeling WWOX deficiency in brain organoids recapitulated several of the phenotypes observed in *Wwox* mutant mice. This allowed us to model the epileptiform activity, which presented as increased power at the low-frequency ranges. This range corresponds to the delta and theta waves, which are implicated in epilepsy.⁴⁵ Cell-patch recordings revealed a depolarized RMP in OS-WWOX-KO neurons implying delayed development, and possibly accounting for the increased excitation. Interestingly, the neuronal changes were observed as early as week 15, a time point that was described in the literature to be still lacking myelin. These data suggest that the role of WWOX in regulating hyperexcitability, supporting the notion of an important role of neuronal WWOX expression in disease development.

WWOX deficient brain organoids also reproduced the hypomyelination defect observed in Wwox-mutant mice. The phenotype was found to be progressive, and eventually resulted in apparent diminished myelin staining and more unmyelinated axons, suggestive of hypomyelination. Overall, our results in this system further support the function of WWOX in OL and myelination in humans.

Altogether, our findings indicate that neuronal WWOX deficiency results in hyperexcitability and myelination defects.

### References

1. Abu-Remaileh M, Dodson EJ, Schueler-Furman O, Aqeilan RI. Pleiotropic Functions of Tumor Suppressor WWOX in Normal and Cancer Cells. J Biol Chem. Oct 23 2015;doi:10.1074/jbc.R115.676346
2. Bednarek AK, Laflin KJ, Daniel RL, Liao Q, Hawkins KA, Aldaz CM. WWOX, a novel WW domain-containing protein mapping to human chromosome 16q23.3-24.1, a region frequently affected in breast cancer. Cancer Res. Apr 15 2000;60(8):2140-5.
3. Aqeilan RI, Abu-Remaileh M, Abu-Odeh M. The common fragile site FRA16D gene product WWOX: roles in tumor suppression and genomic stability. Cell Mol Life Sci. Dec 2014;71(23):4589-99. doi:10.1007/s00018-014-1724-y
4. Gardenswartz A, Aqeilan RI. WW domain-containing oxidoreductase's role in myriad cancers: Clinical significance and future implications. Experimental Biology and Medicine. Mar 1 2014;239(3):253-63. doi:10.1177/1535370213519213
5. Salah Z, Aqeilan R, Huebner K. WWOX gene and gene product: tumor suppression through specific protein interactions. Future oncology. Feb 2010;6(2):249-59. doi:10.2217/fon.09.152
6. Abu-Odeh M, Hereema NA, Aqeilan RI. WWOX modulates the ATR-mediated DNA damage checkpoint response. Oncotarget. Jan 26 2016;7(4):4344-55. doi:10.18632/oncotarget.6571
7. Aldaz CM, Ferguson BW, Abba MC. WWOX at the crossroads of cancer, metabolic syndrome related traits and CNS pathologies. Review. Biochimica et biophysica acta. Jun 14 2014;doi:10.1016/j.bbcan.2014.06.001
8. Chen ST, Chuang JI, Wang JP, Tsai MS, Li H, Chang NS. Expression of WW domain-containing oxidoreductase WOX1 in the developing murine nervous system. Neuroscience. 2004;124(4):831-9. doi:10.1016/j.neuroscience.2003.12.036
9. Nunez MI, Ludes-Meyers J, Aldaz CM. WWOX protein expression in normal human tissues. J Mol Histol. May 2006;37(3-4):115-25.
10. Piard J, Hawkes L, Milh M, et al. The phenotypic spectrum of WWOX-related disorders: 20 additional cases of WOREE syndrome and review of the literature. Genet Med. Jun 2019;21(6):1308-1318. doi:10.1038/s41436-018-0339-3
11. Mallaret M, Synofzik M, Lee J, et al. The tumour suppressor gene WWOX is mutated in autosomal recessive cerebellar ataxia with epilepsy and mental retardation. Brain. Feb 2014;137(Pt 2):411-9. doi:10.1093/brain/awt338 awt338 [pii]
12. Kothur K, Holman K, Farnsworth E, et al. Diagnostic yield of targeted massively parallel sequencing in children with epileptic encephalopathy. Seizure. Jul 2018;59:132-140. doi:10.1016/j.seizure.2018.05.005
13. Shaukat Q, Hertecant J, El-Hattab AW, Ali BR, Suleiman J. West syndrome, developmental and epileptic encephalopathy, and severe CNS disorder associated with WWOX mutations. Epileptic Disord. Oct 1 2018;20(5):401-412. doi:10.1684/epd.2018.1005
14. Ehaideb SN, Al-Bu Ali MJ, Al-Obaid JJ, Aljassim KM, Alfadhel M. Novel Homozygous Mutation in the WWOX Gene Causes Seizures and Global Developmental Delay: Report and Review. Transl Neurosci. 2018;9:203-208. doi:10.1515/tnsci-2018-0029
15. Johannsen J, Kortum F, Rosenberger G, et al. A novel missense variant in the SDR domain of the WWOX gene leads to complete loss of WWOX protein with early-onset epileptic encephalopathy and severe developmental delay. Neurogenetics. Aug 2018;19(3):151-156. doi:10.1007/s10048-018-0549-5
16. Tarta-Arsene O, Barca D, Craiu D, Iliescu C. Practical clues for diagnosing WWOX encephalopathy. Epileptic Disord. Sep 1 2017;19(3):357-361. doi:10.1684/epd.2017.0924
17. Abdel-Salam G, Thoenes M, Afifi HH, Korber F, Swan D, Bolz HJ. The supposed tumor suppressor gene WWOX is mutated in an early lethal microcephaly syndrome with epilepsy, growth retardation and retinal degeneration. Orphanet J Rare Dis. 2014;9:12. doi:10.1186/1750-1172-9-12 1750-1172-9-12 [pii]
18. Mignot C, Lambert L, Pasquier L, et al. WWOX-related encephalopathies: delineation of the phenotypical spectrum and emerging genotype-phenotype correlation. J Med Genet. Jan 2015;52(1):61-70. doi:10.1136/jmedgenet-2014-102748 jmedgenet-2014-102748 [pii]
19. Aqeilan RI, Trapasso F, Hussain S, et al. Targeted deletion of Wwox reveals a tumor suppressor function. Proc Natl Acad Sci USA. March 6, 2007 2007;104(10):3949-3954.
20. Aqeilan RI, Hassan MQ, de Bruin A, et al. The WWOX tumor suppressor is essential for post-natal survival and normal bone metabolism. J Biol Chem. Aug 1 2008;283(31):21629-39.
21. Suzuki H, Katayama K, Takenaka M, Amakasu K, Saito K, Suzuki K. A spontaneous mutation of the Wwox gene and audiogenic seizures in rats with lethal dwarfism and epilepsy. Genes, brain, and behavior. May 20 2009;doi:GBB502 [pii] 10.1111/j.1601-183X.2009.00502.x
22. Abdeen SK, Del Mare S, Hussain S, et al. Conditional inactivation of the mouse Wwox tumor suppressor gene recapitulates the null phenotype. Research Support, N.I.H., Extramural Research Support, Non-U.S. Gov't. Journal of cellular physiology. Jul 2013;228(7):1377-82. doi:10.1002/jcp.24308
23. Elazar N, Vainshtein A, Golan N, et al. Axoglial Adhesion by Cadm4 Regulates CNS Myelination. Neuron. Jan 16 2019;101(2):224-231 e5. doi:10.1016/j.neuron.2018.11.032
24. Madhavan M, Nevin ZS, Shick HE, et al. Induction of myelinating oligodendrocytes in human cortical spheroids. Nat Methods. Sep 2018;15(9):700-706. doi:10.1038/s41592-018-0081-4
25. Mansour AA, Goncalves JT, Bloyd CW, et al. An in vivo model of functional and vascularized human brain organoids. Nat Biotechnol. Jun 2018;36(5):432-441. doi:10.1038/nbt.4127
26. Yang HJ, Vainshtein A, Maik-Rachline G, Peles E. G protein-coupled receptor 37 is a negative regulator of oligodendrocyte differentiation and myelination. Nat Commun. Mar 10 2016;7:10884. doi:10.1038/ncomms10884
27. Tronche F, Kellendonk C, Kretz O, et al. Disruption of the glucocorticoid receptor gene in the nervous system results in reduced anxiety. Nat Genet. Sep 1999;23(1):99-103. doi:10.1038/12703
28. Zhu Y, Romero MI, Ghosh P, et al. Ablation of NF1 function in neurons induces abnormal development of cerebral cortex and reactive gliosis in the brain. Genes & development. Apr 1 2001;15(7):859-76. doi:10.1101/gad.862101
29. Lu QR, Sun T, Zhu Z, et al. Common developmental requirement for Olig function indicates a motor neuron/oligodendrocyte connection. Cell. Apr 5 2002;109(1):75-86. doi:10.1016/s0092-8674(02)00678-5
30. Gregorian C, Nakashima J, Le Belle J, et al. Pten deletion in adult neural stem/progenitor cells enhances constitutive neurogenesis. J Neurosci. Feb 11 2009;29(6):1874-86. doi:10.1523/JNEUROSCI.3095-08.2009
31. Cahoy JD, Emery B, Kaushal A, et al. A transcriptome database for astrocytes, neurons, and oligodendrocytes: a new resource for understanding brain development and function. J Neurosci. Jan 2 2008;28(1):264-78. doi:10.1523/JNEUROSCI.4178-07.2008
32. Hansen KF, Sakamoto K, Pelz C, Impey S, Obrietan K. Profiling status epilepticus-induced changes in hippocampal RNA expression using high-throughput RNA sequencing. Sci Rep. Nov 6 2014;4:6930. doi:10.1038/srep06930
33. Vejar S, Oyarzun JE, Retamal MA, Ortiz FC, Orellana JA. Connexin and Pannexin-Based Channels in Oligodendrocytes: Implications in Brain Health and Disease. Front Cell Neurosci. 2019;13:3. doi:10.3389/fncel.2019.00003
34. Dugas JC, Tai YC, Speed TP, Ngai J, Barres BA. Functional genomic analysis of oligodendrocyte differentiation. J Neurosci. Oct 25 2006;26(43):10967-83. doi:10.1523/JNEUROSCI.2572-06.2006
35. Rosenberg SS, Powell BL, Chan JR. Receiving mixed signals: uncoupling oligodendrocyte differentiation and myelination. Cell Mol Life Sci. Dec 2007;64(23):3059-68. doi:10.1007/s00018-007-7265-x
36. Emery B, Agalliu D, Cahoy JD, et al. Myelin gene regulatory factor is a critical transcriptional regulator required for CNS myelination. Cell. Jul 10 2009;138(1):172-85. doi:10.1016/j.cell.2009.04.031
37. Habib N, Li Y, Heidenreich M, et al. Div-Seq: Single-nucleus RNA-Seq reveals dynamics of rare adult newborn neurons. Science. Aug 26 2016;353(6302):925-8. doi:10.1126/science.aad7038
38. Habib N, Avraham-Davidi I, Basu A, et al. Massively parallel single-nucleus RNA-seq with DroNc-seq. Nat Methods. Oct 2017;14(10):955-958. doi:10.1038/nmeth.4407
39. Zeisel A, Munoz-Manchado AB, Codeluppi S, et al. Brain structure. Cell types in the mouse cortex and hippocampus revealed by single-cell RNA-seq. Science. Mar 6 2015;347(6226):1138-42. doi:10.1126/science.aaa1934
40. Marques S, Zeisel A, Codeluppi S, et al. Oligodendrocyte heterogeneity in the mouse juvenile and adult central nervous system. Science. Jun 10 2016;352(6291):1326-1329. doi:10.1126/science.aaf6463
41. Artegiani B, Lyubimova A, Muraro M, van Es JH, van Oudenaarden A, Clevers H. A Single-Cell RNA Sequencing Study Reveals Cellular and Molecular Dynamics of the Hippocampal Neurogenic Niche. Cell Rep. Dec 12 2017;21(11):3271-3284. doi:10.1016/j.celrep.2017.11.050
42. Barak B, Zhang Z, Liu Y, et al. Neuronal deletion of Gtf2i, associated with Williams syndrome, causes behavioral and myelin alterations rescuable by a remyelinating drug. Nat Neurosci. May 2019;22(5):700-708. doi:10.1038/s41593-019-0380-9
43. Cicciarelli J, Terasaki PI. DR phenotype matching and transfusion in patients treated with cyclosporine. Transplant Proc. Dec 1988;20(6):1082-3.
44. Pre D, Nestor MW, Sproul AA, et al. A time course analysis of the electrophysiological properties of neurons differentiated from human induced pluripotent stem cells (iPSCs). PLoS One. 2014;9(7):e103418. doi:10.1371/journal.pone.0103418
45. Clemens B. Pathological theta oscillations in idiopathic generalised epilepsy. Clin Neurophysiol. Jun 2004;115(6):1436-41. doi:10.1016/j.clinph.2004.01.018
46. Tochigi Y, Takamatsu Y, Nakane J, Nakai R, Katayama K, Suzuki H. Loss of Wwox Causes Defective Development of Cerebral Cortex with Hypomyelination in a Rat Model of Lethal Dwarfism with Epilepsy. Int J Mol Sci. Jul 23 2019;20(14)doi:10.3390/ijms20143596
47. Drenthen GS, Backes WH, Aldenkamp AP, Vermeulen RJ, Klinkenberg S, Jansen JFA. On the merits of non-invasive myelin imaging in epilepsy, a literature review. J Neurosci Methods. May 15 2020;338:108687. doi:10.1016/j.jneumeth.2020.108687
48. Gibson EM, Geraghty AC, Monje M. Bad wrap: Myelin and myelin plasticity in health and disease. Dev Neurobiol. Feb 2018;78(2): 123-135. doi:10.1002/dneu.22541
49. Zuchero JB, Barres BA. Intrinsic and extrinsic control of oligodendrocyte development. Curr Opin Neurobiol. Dec 2013;23(6):914-20. doi:10.1016/j.conb.2013.06.005
50. Simons M, Trajkovic K. Neuron-glia communication in the control of oligodendrocyte function and myelin biogenesis. J Cell Sci. Nov 1 2006;119(Pt 21):4381-9. doi:10.1242/jcs.03242
51. Emery B. Regulation of oligodendrocyte differentiation and myelination. Science. Nov 5 2010;330(6005):779-82. doi:10.1126/science.1190927
52. Barres BA, Raff MC. Proliferation of oligodendrocyte precursor cells depends on electrical activity in axons. Nature. Jan 21 1993;361(6409):258-60. doi:10.1038/361258a0
53. Bergles DE, Roberts JD, Somogyi P, Jahr CE. Glutamatergic synapses on oligodendrocyte precursor cells in the hippocampus. Nature. May 11 2000;405(6783):187-91. doi:10.1038/35012083
54. Bouteille N, Driouch K, Hage PE, et al. Inhibition of the Wnt/beta-catenin pathway by the WWOX tumor suppressor protein. Oncogene. May 25 2009;doi:onc2009120 [pii] 10. 103 8/onc.2009.120
55. Abu-Odeh M, Bar-Mag T, Huang H, et al. Characterizing WW Domain Interactions of Tumor Suppressor WWOX Reveals Its Association with Multiprotein Networks. The Journal of biological chemistry. Mar 28 2014;289(13):8865-80. doi:10.1074/jbc.M113.506790
56. Wang HY, Juo LI, Lin YT, et al. WW domain-containing oxidoreductase promotes neuronal differentiation via negative regulation of glycogen synthase kinase 3beta. Cell death and differentiation. Jun 2012;19(6):1049-59. doi:10.1038/cdd.2011.188
57. Khawaled S, Nigita G, Distefano R, et al. Pleiotropic tumor suppressor functions of WWOX antagonize metastasis. Signal Transduct Target Ther. Apr 17 2020;5(1):43. doi:10.1038/s41392-020-0136-8
58. Ferguson BW, Gao X, Zelazowski MJ, et al. The cancer gene WWOX behaves as an inhibitor of SMAD3 transcriptional activity via direct binding. Meta-Analysis Research Support, N.I.H., Extramural. BMC cancer. 2013;13:593. doi:10.1186/1471-2407-13-593
59. Abu-Odeh M, Salah Z, Herbel C, Hofmann TG, Aqeilan RI. WWOX, the common fragile site FRA16D gene product, regulates ATM activation and the DNA damage response. Proc Natl Acad Sci U S A. Nov 4 2014;111(44):E4716-25. doi:10.1073/pnas.1409252111
60. Lancaster MA, Knoblich JA. Organogenesis in a dish: modeling development and disease using organoid technologies. Science. Jul 18 2014;345(6194):1247125. doi:10.1126/science.1247125
61. Lancaster MA, Renner M, Martin CA, et al. Cerebral organoids model human brain development and microcephaly. Nature. Sep 19 2013;501(7467):373-9. doi:10.1038/nature12517
62. Sidhaye J, Knoblich JA. Brain organoids: an ensemble of bioassays to investigate human neurodevelopment and disease. Cell Death Differ. Jun 1 2020;doi:10.1038/s41418-020-0566-4
63. Aqeilan RI. Engineering organoids: a promising platform to understand biology and treat diseases. Cell Death Differ. Jan 2021;28(1):1-4. doi:10.1038/s41418-020-00680-0
64. Steinberg DJ, Saleem A, Repudi SR, et al. Modeling Genetic Epileptic Encephalopathies using Brain Organoids. bioRxiv. 2020:2020.08.23.263236. doi:10.1101/2020.08.23.263236

### Example 2: Neonatal neuronal WWOX gene therapy rescues Wwox null phenotypes

In recent years, evidence linking WWOX function to the regulation of homeostasis of the central nervous system (CNS) has been proposed.^{9,10} Germline recessive mutations (missense, nonsense and partial/complete deletions) in the *WWOX* gene were found to be associated with two major phenotypes, namely SCAR12 (spinocerebellar ataxia, autosomal recessive -12, OMIM 614322) and WOREE syndrome (WWOX-related epileptic encephalopathy), the latter also known as developmental and epileptic encephalopathy-28 (DEE28, OMIM 616211).⁹ WOREE is a complex and devastating neurological disorder observed in children harboring an early premature stop codon or complete loss of WWOX.¹¹ The clinical spectrum of WOREE includes severe developmental delay, early-onset of severe epilepsy with variable seizure manifestations (tonic, clonic, tonic-clonic, myoclonic, infantile spasms and absence). Most of the affected patients make no eye contact and are not able to sit, speak, or walk.⁹ WOREE syndrome is refractory to current anticonvulsant drugs, hence there is an urgent need to develop alternative treatments to help children with WOREE syndrome. Children with SCAR12, mostly due to missense mutations in WWOX, display a milder phenotype including ataxia and epilepsy.¹² Epilepsy in SCAR12 can be treated with anticonvulsant drugs, though children still display ataxia and are intellectually disabled. Moreover, WWOX mutations have been documented in patients with West Syndrome, which is characterized by epileptic spasms with hypsarrhythmia.¹³ Brains of the children carrying *WWOX* gene mutations are found to be abnormal, as assessed by magnetic resonance imaging (MRI). Brain abnormalities such as hypoplasia of the corpus callosum, progressive cerebral atrophy, delayed myelination and optic nerve atrophy have been documented in most cases. It is largely unknown how mutations in *WWOX* or loss of WWOX function could lead to these CNS-associated abnormalities.

There is a marked similarity between human WWOX (hWWOX) and murine Wwox (*mWwox*). In fact, the human WWOX protein sequence is 93% identical and 95% similar to the murine WWOX protein sequence. Remarkably, targeted loss of *Wwox* function in rodent models (mice and rats) phenocopies the complex human neurological phenotypes, including severe epileptic seizures, growth retardation, ataxia and premature death.^{12,14,15} Wwox null mice also exhibit phenotypes associated with impaired bone metabolism and steroidogenesis.^{16,17} Example 1 of this disclosure shows that conditional ablation of murine Wwox in either neural stem cells and progenitors (N-KO) or neuronal cells (S-KO mice) resulted in severe epilepsy, ataxia and premature death at 3-4 weeks, recapitulating the phenotypes observed in the Wwox-null mice. These results highlight the significant role of WWOX in neuronal function and prompted us to test whether restoring WWOX expression in the neuronal compartment of *Wwox* null mice could reverse the observed phenotypes. To this end, we used an adeno-associated virus (AAV) vector to restore WWOX expression. We demonstrated that an AAV vector harboring the mW wox or hWWOX open reading frame and driven by the human neuronal Synapsin I promoter could reverse Wwox null phenotypes. A single intracerebroventricular (ICV) injection of AAV9-Synapsin I-WWOX rescued the growth retardation, epileptic seizures, ataxia and premature death of Wwox null mice. In addition, WWOX restoration improved myelination and reversed the abnormal behavioral changes of *Wwox* null mice. Overall, these remarkable results indicate that WWOX gene therapy could be a promising cure approach for children with WOREE and SCAR12.

### Results

### Restoration of neuronal WWOX rescues growth retardation and post-natal lethality of Wwox null mutant mice

In example 1, we show that conditional ablation of WWOX in neurons phenocopies the *Wwox* null mice including growth retardation, spontaneous epileptic seizures, ataxia and premature death at 3-4 weeks. These results implied that WWOX is a key neuronal gene regulating homeostasis of the CNS. Prompted by these remarkable findings, we wanted to address whether neuronal-specific expression of WWOX in *Wwox*-null mice could rescue lethality of these mice and their associated phenotypes. We designed an adeno-associated viral (AAV) vector to express murine *Wwox (mWwox),* or human WWOX (hWWOX), cDNA driven by a human Synapsin-I (hSynI) promoter (Fig. 8A) and packaged this into an AAV9 serotype, which has high CNS tropism and has been used in CNS-based gene therapy trials.^{22,24} An *IRES-EGFP* sequence was cloned downstream of the *Wwox*/*WWOX* sequence to allow tracking of expression. Successful delivery of AAV9-hSynI-*mWwox*-IRES-EGFP (*AAV9-hSynI-mWwox*) should lead to expression of intact WWOX protein in Synapsin-I-positive non-dividing/matured neurons. As control, AAV9-hSynI-EGFP was used. Expression of WWOX and GFP was initially validated by infecting primary *Wwox*-null dorsal root ganglion (DRGs) neurons with the viral particles *in vitro.*

We then evaluated the expression and function of the AAVs *in vivo.* Viral particles (2×10¹⁰/hemisphere) of AAV9-hSynI-mWwox or AAV9-hSynI-EGFP were injected into the intracerebroventricular region of *Wwox* null mice at birth (P0), to achieve widespread transduction of neurons throughout the brain.^{25,26} Successful expression of the transgene in neurons, but not in oligodendrocytes (CC1-positive cells), was validated by immunofluorescence using anti-NeuN and anti-WWOX antibodies.

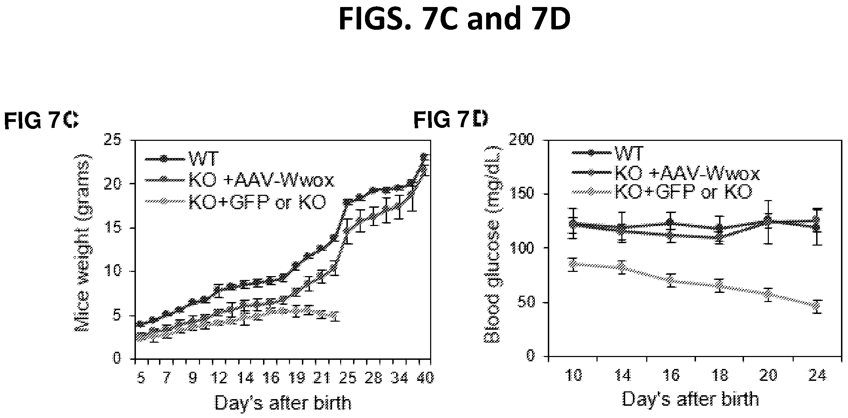

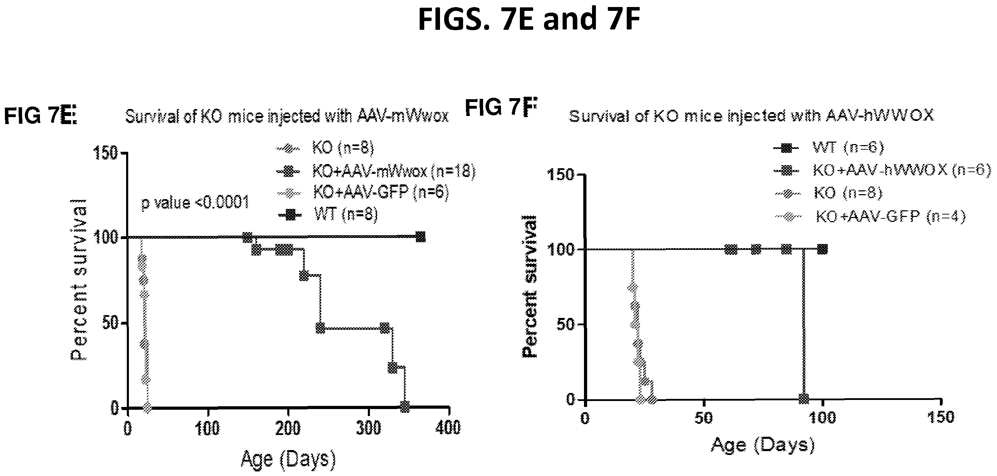

Monitoring of the treated mice revealed that mice injected with AAV9-hSynI-mWwox grew normally (Fig. 7B), gradually gained weight (Fig. 7C) and were indistinguishable from wild type by the age of 6-8 weeks. AAV9-hSynI-EGFP-injected mice exhibited similar phenotypes of *Wwox* null mice (Fig. 7C). Of note, *Wwox* null and AAV9-hSynI-EGFP-injected mice were hypoglycemic from the second week until they died, while AAV9-hSynI-mWwox-injected mice had normal blood glucose levels when compared to the wild type mice (Fig. 7D). Remarkably, all rescued mice lived longer with a median survival of 240 days compared to the *Wwox* null or AAV9-hSynI-EGFP-injected *Wwox* null mice (p value <0.0001) (Fig. 7E). Similar results and outcomes were obtained when replacing mWwox with hWWOX cDNA, though we have only followed these mice for up to 100 days so far (Fig. 7F). Notably, the rescued mice were active and both males and females were fertile. Since *Wwox* null mice were previously shown to lack testicular Leydig cells,¹⁶ we next determined if WWOX neuronal restoration rescues this phenotype and indeed found intact Leydig cells in P17 *AAV9-hSynI-mWwox*-treated mice. Bone growth defects were also formerly documented in *Wwox* mutant mice^{17,27-30}, and hence we examined bones of rescued mice and observed that cortical bones were of comparable size and thickness to WT mice. These results imply that neuronal restoration of WWOX could be sufficient to rescue the abnormal phenotypes of *Wwox* null mice.

### Neuronal restoration of WWOX decreases hyperexcitability of Wwox null mice

*Wwox* null mutants display spontaneous recurrent seizures.^{12,14,18,31} As we did not observe any spontaneous seizures in rescued mice, we determined next the epileptic activity in brains of P21-22 wild type (WT), *Wwox* null (KO) and AAV9-hSynI-mWwox-injected *Wwox* null mice (KO*+AAV9-Wwox*) by performing cell-attached electrophysiology recordings. As expected, the KO pups exhibited severe hyperactivity. Representative traces with spontaneous firing of action potentials are shown in Fig. 8A. A clear hyperexcitability can be noted from the representative traces (WT, KO+*AAV9-Wwox,* and KO). The activity of the KO brains usually resulted in bursts of action potentials and overall there was a drastic increase in the firing rate. The average firing rate over 20 WT, 20 KO+AAV-Wwox and 30 KO recorded neurons was about 6-fold higher in KO pups compared to the WT pups (p=2.6e-7). No significant difference in average firing rate was observed between the KO+AAV-Wwox and the WT pups.

Since KO mice died within less than 4 weeks, we could not perform *in vivo* recordings in adult KO mice. We therefore performed cell attached *in vivo* recordings only in adult WT and KO+AAV9-Wwox mice (Fig. 8B). Representative traces are shown in for WT and KO+AAV9-mWwox and the average firing rate over 60 WT and 60 KO+*AAV9-mWwox* neurons are presented (Fig. 8B). There were no significant differences in the firing rate of adult WT and KO+AAV9-mWwox cortical neurons. These findings indicate that AAV9-hSynI-mWwox could prevent epileptic seizures resulting from WWOX loss.

### Neuronal restoration of WWOX enhances myelination in Wwox null mice likely by promoting OPC differentiation

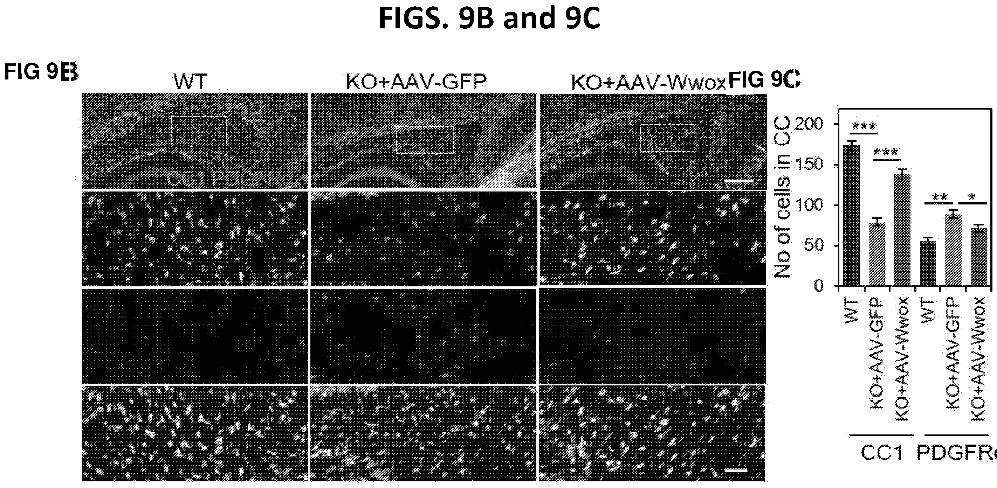

Example 1 linked WWOX loss with hypomyelination. In fact, it was shown that neuronal WWOX ablation results in a non-cell autonomous function impairing differentiation of oligodendrocyte progenitors (OPCs). Hence we next tested whether neuronal restoration of WWOX, using AAV, could rescue the hypomyelination phenotype in *Wwox* null mice. Immunofluorescence analysis of P17 sagittal brain tissues with anti-MBP antibody revealed improved myelination in all parts (cortex, hippocampus and cerebellum) of the rescued *AAV9-hSynI-mWwox* treated-mice brain compared to *Wwox* null mice injected with control virus (Fig. 9A). In addition, we tested whether this improved myelination is associated with increased differentiation of OPCs to matured oligodendrocytes by a non-cell autonomous function of neuronal WWOX. As expected, AAV9-mediated WWOX expression in neurons increased the differentiation of OPCs to matured oligodendrocytes as assessed by immunostaining with CC1 (marker for matured oligodendrocytes) and anti-PDGFRα (marker for OPCs) (Fig. 9B). Quantification of CC1 and OPCs in the corpus callosum showed significantly increased number of matured oligodendrocytes in rescued mice compared to the KO mice injected with control virus on P17 (Fig. 9C).

To further validate the finding of improved myelination after neuronal restoration of WWOX, we performed electron microscopy (EM) analysis for corpus callosum on P17 and in adult mice. Remarkably, neuronal restoration of WWOX using AAV9-hSynI-mWwox increased the number of myelinated axons compared to KO at P17 in the corpus callosum. Furthermore, calculated g-ratios indicated increased myelin thickness upon neuronal WWOX restoration compared to control KO mice. In addition, EM images of the corpus callosum and optic nerves of adult (6 months) rescued mice showed improved myelination. Of note, when comparing myelin thickness of KO+AAV-Wwox and WT corpus callosum at P17 and 6 months, we observed some differences in g-ratio.

### WWOX neuronal restoration decreases anxiety and improves motor functions

We next explored the behavioral changes in *Wwox* null mice after restoration of WWOX in neurons. Unfortunately, we could not assess behavior of *Wwox* null mice due to their poor conditions and premature death. We performed open field, elevated plus maze (EPM) and rotarod tests to examine anxiety and motor coordination in rescued mice (Fig. 10A-E). Remarkably, at 8-10 weeks we observed similar tracking patterns in the open field in the rescued mice (males and females) to those seen in the wild type, indicating that WWOX restoration reduces anxiety in *Wwox* null mice. In addition, the velocity and total distance travelled in the open field tracks were very similar to that of the WT mice (Fig. 10B, C). Moreover, rescued female and male mice exhibited near normal behavior in the EPM (Fig. 10D). Rotarod test was performed to check the motor coordination in rescued mice. Our results revealed that rescued mice had similar motor coordination to WT mice in trial 3, an indicative of their learning ability. Altogether, these results suggest that neuronal WWOX re-expression in *Wwox* null mice restores activity and normal behavior.

### Discussion

We aimed in this Example to restore WWOX in neurons and assess the therapeutic potential of this restoration. In this study, we utilized an AAV9 vector for targeted gene delivery of WWOX to mature neurons to treat the complex neuropathy in the Wwox-null mouse model. We injected mWwox or hWWOX cDNA under the neuronal promotor Synapsin-I into the brains of newborn *Wwox* null mice and showed that this treatment was able to reverse the phenotypes of WWOX deficiency.

The role of WWOX in regulating CNS homeostasis is emerging as a key function of the WWOX gene. Deficiency of WWOX has been linked to a number of neurological disorders.^{9,10} Of particular interest is WOREE syndrome, a devastating complex neurological disease causing premature death with a median survival of 1-4 years.^{9,10} WOREE children are refractory to the current antiepileptic drugs (AEDs) hence challenging the medical and scientific communities to develop new therapeutic strategies. We believe that delivering AAV9-WWOX into the brain of WOREE syndrome patients could be a novel gene therapy approach that would help these patients.

The effects of delivering AAV9-SynI-WWOX into the brains of *Wwox* null mice were remarkable. Firstly, WWOX neuronal delivery restored normal growth and survival of mice with no occurrence of spontaneous seizures and ataxia. In addition, we showed that neuronal restoration of WWOX reduced hyperexcitability in cell-attached recordings. Secondly, neuronal WWOX restoration improved myelination of all regions of the brain further confirming the previous observations of WWOX neuronal non-cell autonomous function on OPC maturation (Example 1). Of note, there are still some differences between rescued and WT mice which could be attributed to an oligodendrocyte-specific WWOX function in regulating the myelination process. Thirdly, WWOX restoration improved the overall behavior of the rescued mice. These findings might suggest that WWOX's proposed role in regulating autism^{9-11,33,34} and perhaps other behavior-associated disorders is driven by proper neuronal function of WWOX.

Another intriguing consequence of neuronal WWOX delivery is the reversibility of hypoglycemia associated with WWOX deficiency in *Wwox* null mice.^{35,36} These results are consistent with a central role of WWOX in the CNS regulation of metabolism of glucose and likely other metabolic functions.³⁷⁻⁴⁰ Interestingly, targeted deletion of *Wwox* in skeletal muscle resulted in impaired glucose homeostasis⁴¹ and this effect was linked to cell-autonomous functions of WWOX.

Another peculiar observation is that the rescued mice were also fertile and able to breed. Given that *Wwox* null mice were shown to display impaired steroidogenesis^{16,29,42}, our current findings imply that WWOX's function in the CNS is superimposing its tissue level function. Altogether, these findings suggest that WWOX could have pleotropic function both at the organ level and at the organism level.

WWOX is ubiquitously expressed in all brain regions.^{10,43,44} Our current observations do not imply that WWOX expression in other brain cell types, such as astrocytes and oligodendrocyte, are dispensable. Evidence linking WWOX function with oligodendrocyte pathology is starting to emerge⁴⁵⁻⁴⁹, however less is known about the cell-autonomous functions of WWOX in oligodendrocytes. The fact that WWOX expression in neurons regulates oligodendrocyte maturation and antagonizes astrogliosis⁵⁰ suggests a complex function of WWOX in CNS physiology and pathophysiology that warrants further in-depth analysis.

The WWOX gene was initially cloned as a putative tumor suppressor.^{51,52} Indeed a plethora of research work in various animal models (reviewed in¹⁵) and observations in human cancer patients^{1,27,39,53-57} proposed WWOX as a tumor suppressor. Given that our restoration of WWOX is limited to brain, we assumed other tissues lacking WWOX expression would be more susceptible to tumor development. Of note, we didn't detect gross tumor formation in the limited number of adult *Wwox*-null mice treated with AAV9-hSynI-mWwox that we examined (age 6-8month, n=6). This was not surprising given that Wwox somatic deletion in several tissues required other hits to promote tumor formation in animal models.^{28,39,58,59}

The limited life-span and poor conditions of *Wwox* null mice forced us to treat these mice very early on in their life (P0). Nevertheless, attempts to treat post-natal *Wwox*-null mice should be explored in the future. Our current findings indicate that WWOX restoration in neonatal mice using an AAV vector could reverse the phenotypes associated with WWOX deficiency. We envisage that this proof-of-concept will lay down the groundwork for a possible gene therapy clinical trial on children suffering from the devastating and often refractory WOREE syndrome.

### Materials and Methods

### Plasmid vectors

Murine *Wwox* or human WWOX cDNA was cloned under the promoter of human *Synapsin I* in pAAV and this vector was packaged into AAV9 serotype (Vector Biolabs, Philadelphia, USA). Custom-made AAV9-hSynI-mWwox-IRES-EGFP, AAV9-hSynI-hWWOX-2A-EGFP and AAV9-hSynI-EGFP viral particles were obtained either from Vector Biolabs or from the Vector Core Facility at Hebrew University of Jerusalem.

### Mice

Generation of *Wwox* null ^{(-/-)} mice (KO) was previously reported¹⁶ and these mice were maintained in an FVB background. Heterozygote ^{(+/-)} mice were used for breeding to get the *Wwox* null mice. Animals were maintained in a SPF unit in a 12 h-light/dark cycle with *ad libitum* access to the food and water. All animal-related experiments were performed in accordance and with prior approval of the Hebrew University-Institutional Animal Care Use Committee (HU-IACUC).

### Intracerebroventricular (ICV) injection of AAV particles in to P0 Wwox null mice

Free-hand intracranial injections of either AAV9-hSynI-mWwox-IRES-EGFP (AAV9-WWOX) or AAV9-hSynI-EGFP (AAV9-GFP) into the *Wwox* null mice were done following a published protocol.²⁵ Briefly, when neonates were born, they were PCR genotyped to identify *Wwox* null mice. *Wwox* null neonates were anesthetized by placing on a dry, flat, cold surface. The anesthetized pup head was gently wiped with a cotton swab soaked in 70% ethanol. Trypan blue 0.1% was added to the virus to enable visualization of the dispensed liquid. An injection site was located at 2/5 of the distance from the lambda suture to each eye. Holding the syringe (preloaded with virus) perpendicular to the surface of the skull, the needle was inserted to a depth of approximately 3 mm. Approximately 1 µl (2 x 10¹⁰ GC/hemisphere) virus was dispensed using a NanoFil syringe with a 33G beveled needle (World Precision Instruments). The other hemisphere was injected in the same way. Injected pups were placed on the warming pad until they were awake, then transferred to the mother's cage. Each injected mouse was carefully monitored for growth, mobility, seizures, ataxia and general condition to assess phenotypes.

### Weight and blood glucose levels

Mice were weighed regularly as indicated in the Figures. To monitor the blood glucose, the tip of the mouse tail was ruptured with scissors and a tiny drop of blood collected for measurement (mg/dL) using an Accu-Check glucometer (Roche Diagnostics, Mannheim, Germany).

### Immunofluorescence

Mice from different genotypes and treatment groups (P17-P18) were euthanized by CO₂ and transcardially perfused with 2% PFA/PBS. Dissected brains were postfixed on ice for 30 min then incubated in 30% sucrose at 4°C overnight. They were then embedded in OCT and sectioned (12-14 µm) using a cryostat. Sagittal sections were washed with PBS and blocked with 5% goat serum containing 0.5% Triton X-100 then incubated for 1 h at room temperature followed by incubation with primary antibodies overnight at 4°C. Then, sections were washed with PBS and incubated with corresponding secondary antibodies tagged with Alexa fluorophore for 1 h at room temperature followed by washing with PBS and mounting with mounting medium.

### Surgical procedures for electrophysiology

Mice were anesthetized using ketamine/medetomidine (i.p; 100 and 83 mg/kg, respectively). The effectiveness of anesthesia was confirmed by the absence of toe-pinch reflexes. Supplemental doses were administered every ~1 h with a quarter of the initial dosage to maintain anesthesia during the electrophysiology procedures. During all surgeries and experiments, body temperature was maintained using a heating pad (37°C). The skin was removed to expose the skull. A custom-made metal pin was affixed to the skull using dental cement and connected to a custom stage. A small hole (3 mm diameter craniotomy) was made in the skull using a biopsy punch (Miltex, PA).

### Cell attached recordings

Cell-attached recordings were obtained with blind patch-clamp recording. Electrodes (~7 MOhm) were pulled from filamented, thin-walled, borosilicate glass (outer diameter, 1.5 mm; inner diameter, 0.86 mm; Hilgenberg GmbH, Malsfeld, Germany) on a vertical two-stage puller (PC-12, Narishige, EastMeadow, NY). The electrodes were filled with internal solution containing the following: 140 mM K-gluconate, 10 mM KCl, 10 mM HEPES, 10 mM Na₂-phosphocreatine, and 0.5 mM EGTA, adjusted to pH 7.25 with KOH. The electrode was inserted at a 45 degrees angle and reached a depth of 300 µm. The electrode positioning was targeted on the brain surface, positioned at 1.6-2 mm posterior to the bregma and 4 mm lateral to the midline. While positioning the electrode, an increase of the pipette resistance to 10-200 MOhm resulted in most cases in the appearance of action potentials (spikes). The detection of a single spike was the criteria to start the recording. All recordings were acquired with an intracellular amplifier in current clamp mode (Multiclamp 700B, Molecular Devices), acquired at 10 kHz (CED Micro 1401-3, Cambridge Electronic Design Limited) and filtered with a high pass filter. For calculation of the average firing rate, the firing rate over a 4 min recording period was calculated for each of the recorded cells. A two-sample t-test was used to assess statistical significance between the recorded groups.

### Electron microscopy

Mice were anesthetized and perfused with a fixative containing 2% paraformaldehyde and 2.5% glutaraldehyde (EM grade) in 0.1 M sodium cacodylate buffer, pH 7.3. Brains were isolated and incubated in the same fixative for 2 h at room temperature then stored in 4°C until they were processed. Collected tissues (corpus callosum, optic nerve) were washed four times with sodium cacodylate and postfixed for 1 h with 1% osmium tetroxide, 1.5% potassium ferricyanide in sodium cacodylate, and washed four times with the same buffer. Then, tissue samples were dehydrated with graded series of ethanol solutions (30, 50, 70, 80, 90, 95%) for 10 min each and then 100% ethanol three times for 20 min each, followed by two changes of propylene oxide. Tissue samples were then infiltrated with series of epoxy resin (25, 50, 75, 100%) for 24 h each and polymerized in the oven at 60°C for 48 h. The blocks were sectioned by an ultramicrotome (Ultracut E, Riechert-Jung), and sections of 80 nm were obtained and stained with uranyl acetate and lead citrate. Sections were observed using a Jeol JEM 1400 Plus transmission electron microscope and pictures were taken using a Gatan Orius CCD camera. EM micrographs were analyzed using computer-assisted ImageJ analysis software. To calculate g-ratio, myelinated axons (~300, 100 axons per mouse, n = 3 per genotype) from EM images from corpus callosum were analyzed by dividing inner axonal diameter over the total axonal diameter.

### Open field test

The open field test was performed following the previously published protocol.⁶⁰ Briefly, mice were placed in the corner of a 50 x 50 x 33 cm arena, and allowed to freely explore for 6 min. The center of the arena was defined as a 25 x 25 cm square in the middle of the arena. Velocity and time spent in the center and arena circumference were measured. Mice tested in the open field were recorded using a video camera connected to a computer having tracking software (Ethovision 12).

### Elevated plus maze test

The test apparatus consisted of two open arms (30 x 5 cm) bordered by a 1 cm high rim across from each other and perpendicular to two closed arms bordered by a rim of 16 cm, all elevated 75 cm from the floor. Mice were put into the maze and were allowed to explore it for 5 min. Duration of visits in both the open and closed arms were recorded.⁶⁰

### Rotarod test

Each animal was placed on a rotating rod whose revolving speed increased from 5 rounds per min (rpm) to 40 rpm for 99 s. The test for each animal consisted of three trials separated by 20 min. Time to fall from device (latency) was recorded for each trial for each animal. If the animal did not fall from the device by 240 s from the beginning of the trial, the trial was terminated.⁶¹

### Image acquisition and analysis

Immunostained sections were imaged using a panoramic digital slide scanner or an Olympus FV1000 confocal laser scanning microscope or Nikon A1R+ confocal microscope. The acquired images were processed using the associated microscope software programs, namely CaseViewer, F-10-ASW viewer, and NIS elements respectively. Images were analyzed using ImageJ software. Images were analyzed while blinded to the genotype and the processing included the global changes of brightness and contrast.

### Statistical analysis

All graphs and statistical analyses was preformed using either Excel or GraphPad Prism 5. Results of the experiments were presented as mean ± SEM. The two-tailed unpaired Student's t-test was used to test the statistical significance. Results were considered significant when the p <0.05, otherwise they were represented as ns (no significance). Data analysis was performed while blinded to the genotype. Sample size and p value is indicated in the figure legends.

### References

1. Aldaz, C.M., Ferguson, B.W., and Abba, M.C. (2014). WWOX at the crossroads of cancer, metabolic syndrome related traits and CNS pathologies. Biochimica et biophysica acta 1846, 188-200. 10.1016/j.bbcan.2014.06.001S0304-419X(14)00050-X [pii].
2. Del Mare, S., Salah, Z., and Aqeilan, R.I. (2009). WWOX: its genomics, partners, and functions. J Cell Biochem 108, 737-745. 10.1002/jcb.22298.
3. Salah, Z., Alian, A., and Aqeilan, R.I. (2012). WW domain-containing proteins: retrospectives and the future. Frontiers in bioscience : a journal and virtual library 17, 331-348.
4. Salah, Z., Aqeilan, R., and Huebner, K. (2010). WWOX gene and gene product: tumor suppression through specific protein interactions. Future oncology 6, 249-259. 10.2217/fon.09.152.
5. Chang, N.S., Hsu, L.J., Lin, Y.S., Lai, F.J., and Sheu, H.M. (2007). WW domain-containing oxidoreductase: a candidate tumor suppressor. Trends Mol Med 13, 12-22.
6. Abu-Remaileh, M., Dodson, E.J., Schueler-Furman, O., and Aqeilan, R.I. (2015). Pleiotropic Functions of Tumor Suppressor WWOX in Normal and Cancer Cells. J Biol Chem. 10.1074/jbc.R115.676346.
7. Aqeilan, R.I., Abu-Remaileh, M., and Abu-Odeh, M. (2014). The common fragile site FRA16D gene product WWOX: roles in tumor suppression and genomic stability. Cell Mol Life Sci 71, 4589-4599. 10.1007/s00018-014-1724-y.
8. Hazan, I., Hofmann, T.G., and Aqeilan, R.I. (2016). Tumor Suppressor Genes within Common Fragile Sites Are Active Players in the DNA Damage Response. PLoS Genet 12, e1006436. 10.1371/journal.pgen.1006436.
9. Banne, E., Abudiab, B., Abu-Swai, S., Repudi, S.R., Steinberg, D.J., Shatleh, D., Alshammery, S., Lisowski, L., Gold, W., Carlen, P.L., and Aqeilan, R.I. (2021). Neurological Disorders Associated with WWOX Germline Mutations-A Comprehensive Overview. Cells 10, 824.
10. Aldaz, C.M., and Hussain, T. (2020). WWOX Loss of Function in Neurodevelopmental and Neurodegenerative Disorders. Int J Mol Sci 21. 10.3390/ijms21238922.
11. Piard, J., Hawkes, L., Milh, M., Villard, L., Borgatti, R., Romaniello, R., Fradin, M., Capri, Y., Heron, D., Nougues, M.C., Nava, C., et al. (2019). The phenotypic spectrum of WWOX-related disorders: 20 additional cases of WOREE syndrome and review of the literature. Genet Med 21, 1308-1318. 10.1038/s41436-018-0339-3.
12. Mallaret, M., Synofzik, M., Lee, J., Sagum, C.A., Mahajnah, M., Sharkia, R., Drouot, N., Renaud, M., Klein, F.A., Anheim, M., Tranchant, C., et al. (2014). The tumour suppressor gene WWOX is mutated in autosomal recessive cerebellar ataxia with epilepsy and mental retardation. Brain 137, 411-419. 10.1093/brain/awt338awt338 [pii].
13. Shaukat, Q., Hertecant, J., El-Hattab, A.W., Ali, B.R., and Suleiman, J. (2018). West syndrome, developmental and epileptic encephalopathy, and severe CNS disorder associated with WWOX mutations. Epileptic Disord 20, 401-412. 10.1684/epd.2018.1005.
14. Suzuki, H., Katayama, K., Takenaka, M., Amakasu, K., Saito, K., and Suzuki, K. (2009). A spontaneous mutation of the Wwox gene and audiogenic seizures in rats with lethal dwarfism and epilepsy. Genes, brain, and behavior 8, 650-660. 10.1111/j.1601-183X.2009.00502.xGBB502 [pii].
15. Tanna, M., and Aqeilan, R.I. (2018). Modeling WWOX Loss of Function in vivo: What Have We Learned? Front Oncol 8, 420. 10.3389/fonc.2018.00420.
16. Aqeilan, R.I., Hagan, J.P., de Bruin, A., Rawahneh, M., Salah, Z., Gaudio, E., Siddiqui, H., Volinia, S., Alder, H., Lian, J.B., Stein, G.S., et al. (2009). Targeted ablation of the WW domain-containing oxidoreductase tumor suppressor leads to impaired steroidogenesis. Endocrinology 150, 1530-1535. en.2008-1087 [pii]10.1210/en.2008-1087.
17. Aqeilan, R.I., Hassan, M.Q., de Bruin, A., Hagan, J.P., Volinia, S., Palumbo, T., Hussain, S., Lee, S.H., Gaur, T., Stein, G.S., Lian, J.B., et al. (2008). The WWOX tumor suppressor is essential for post-natal survival and normal bone metabolism. J Biol Chem 283, 21629-21639.
18. Repudi, S.R., Steinberg, D.J., Elazar, N., Breton, V.L., Aquilino, M.S., Saleem, A., Abu-Swai, S., Vainshtein, A., Eshed-Eisenbach, Y., Vijayaragavan, B., Behar, O., et al. (2021). Neuronal deletion of Wwox, associated with WOREE syndrome, causes epilepsy and myelin defects. Brain In press.
19. Sun, S., and Schaffer, D.V. (2018). Engineered viral vectors for functional interrogation, deconvolution, and manipulation of neural circuits. Curr Opin Neurobiol 50, 163-170. 10.1016/j.conb.2017.12.011.
20. Aguti, S., Malerba, A., and Zhou, H. (2018). The progress of AAV-mediated gene therapy in neuromuscular disorders. Expert Opin Biol Ther 18, 681-693. 10.1080/14712598.2018.1479739.
21. Pierce, E.A., and Bennett, J. (2015). The Status of RPE65 Gene Therapy Trials: Safety and Efficacy. Cold Spring Harb Perspect Med 5, a017285. 10.1101/cshperspect.a017285.
22. Wang, D., Tai, P.W.L., and Gao, G. (2019). Adeno-associated virus vector as a platform for gene therapy delivery. Nat Rev Drug Discov 18, 358-378. 10.1038/s41573-019-0012-9.
23. Nakai, H., Yant, S.R., Storm, T.A., Fuess, S., Meuse, L., and Kay, M.A. (2001). Extrachromosomal recombinant adeno-associated virus vector genomes are primarily responsible for stable liver transduction in vivo. J Virol 75, 6969-6976. 10.1128/JVI.75.15.6969-6976.2001.
24. Balakrishnan, B., and Jayandharan, G.R. (2014). Basic biology of adeno-associated virus (AAV) vectors used in gene therapy. Curr Gene Ther 14, 86-100. 10.2174/1566523214666140302193709.
25. Kim, J.Y., Grunke, S.D., Levites, Y., Golde, T.E., and Jankowsky, J.L. (2014). Intracerebroventricular viral injection of the neonatal mouse brain for persistent and widespread neuronal transduction. J Vis Exp, 51863. 10.3791/51863.
26. Gholizadeh, S., Tharmalingam, S., Macaldaz, M.E., and Hampson, D.R. (2013). Transduction of the central nervous system after intracerebroventricular injection of adeno-associated viral vectors in neonatal and juvenile mice. Hum Gene Ther Methods 24, 205-213. 10.1089/hgtb.2013.076.
27. Kurek, K.C., Del Mare, S., Salah, Z., Abdeen, S., Sadiq, H., Lee, S.H., Gaudio, E., Zanesi, N., Jones, K.B., DeYoung, B., Amir, G., et al. (2010). Frequent attenuation of the WWOX tumor suppressor in osteosarcoma is associated with increased tumorigenicity and aberrant RUNX2 expression. Cancer Res 70, 5577-5586. 0008-5472.CAN-09-4602 [pii]10.1158/0008-5472.CAN-09-4602.
28. Del Mare, S., Husanie, H., Iancu, O., Abu-Odeh, M., Evangelou, K., Lovat, F., Volinia, S., Gordon, J., Amir, G., Stein, J., Stein, G.S., et al. (2016). WWOX and p53 Dysregulation Synergize to Drive the Development of Osteosarcoma. Cancer Res 76, 6107-6117. 10.1158/0008-5472.CAN-16-0621.
29. Ludes-Meyers, J.H., Kil, H., Parker-Thornburg, J., Kusewitt, D.F., Bedford, M.T., and Aldaz, C.M. (2009). Generation and characterization of mice carrying a conditional allele of the Wwox tumor suppressor gene. PLoS One 4, e7775. 10.1371/journal.pone.0007775.
30. Abdeen, S.K., Del Mare, S., Hussain, S., Abu-Remaileh, M., Salah, Z., Hagan, J., Rawahneh, M., Pu, X.A., Russell, S., Stein, J.L., Stein, G.S., et al. (2013). Conditional inactivation of the mouse Wwox tumor suppressor gene recapitulates the null phenotype. Journal of cellular physiology 228, 1377-1382. 10.1002/jcp.24308.
31. Cheng, Y.Y., Chou, Y.T., Lai, F.J., Jan, M.S., Chang, T.H., Jou, I.M., Chen, P.S., Lo, J.Y., Huang, S.S., Chang, N.S., Liou, Y.T., et al. (2020). Wwox deficiency leads to neurodevelopmental and degenerative neuropathies and glycogen synthase kinase 3beta-mediated epileptic seizure activity in mice. Acta Neuropathol Commun 8, 6. 10.1186/s40478-020-0883-3.
32. Pattali, R., Mou, Y, and Li, X.J. (2019). AAV9 Vector: a Novel modality in gene therapy for spinal muscular atrophy. Gene Ther 26, 287-295. 10.1038/s41434-019-0085-4.
33. Peter, B., Dinu, V., Liu, L., Huentelman, M., Naymik, M., Lancaster, H., Vose, C., and Schrauwen, I. (2019). Exome Sequencing of Two Siblings with Sporadic Autism Spectrum Disorder and Severe Speech Sound Disorder Suggests Pleiotropic and Complex Effects. Behav Genet 49, 399-414. 10.1007/s10519-019-09957-8.
34. Mignot, C., Lambert, L., Pasquier, L., Bienvenu, T., Delahaye-Duriez, A., Keren, B., Lefranc, J., Saunier, A., Allou, L., Roth, V., Valduga, M., et al. (2015). WWOX-related encephalopathies: delineation of the phenotypical spectrum and emerging genotype-phenotype correlation. J Med Genet 52, 61-70. 10.1136/jmedgenet-2014-102748jmedgenet-2014-102748 [pii].
35. Abu-Remaileh, M., Seewaldt, V.L., and Aqeilan, R.I. (2014). WWOX loss inactivates aerobic glycolysis. Molecular and cellular oncology.
36. Abu-Remaileh, M., and Aqeilan, R.I. (2014). Tumor suppressor WWOX regulates glucose metabolism via HIF1alpha modulation. Cell death and differentiation 21, 1805-1814. 10.1038/cdd.2014.95.
37. Iatan, I., Choi, H.Y., Ruel, I., Reddy, M.V., Kil, H., Lee, J., Abu Odeh, M., Salah, Z., Abu-Remaileh, M., Weissglas-Volkov, D., Nikkola, E., et al. (2014). The WWOX Gene Modulates HDL and Lipid Metabolism. Circulation. Cardiovascular genetics. 10.1161/CIRCGENETICS.113.000248.
38. Lee, J.C., Weissglas-Volkov, D., Kyttala, M., Dastani, Z., Cantor, R.M., Sobel, E.M., Plaisier, C.L., Engert, J.C., van Greevenbroek, M.M., Kane, J.P., Malloy, M.J., et al. (2008). WW-domain-containing oxidoreductase is associated with low plasma HDL-C levels. Am J Hum Genet 83, 180-192. S0002-9297(08)00399-6 [pii]10.1016/j.ajhg.2008.07.002.
39. Abu-Remaileh, M., Khalaileh, A., Pikarsky, E., and Aqeilan, R.I. (2018). WWOX controls hepatic HIF1alpha to suppress hepatocyte proliferation and neoplasia. Cell Death Dis 9, 511. 10.1038/s41419-018-0510-4.
40. Abu-Remaileh, M., and Aqeilan, R.I. (2015). The tumor suppressor WW domain-containing oxidoreductase modulates cell metabolism. Exp Biol Med (Maywood) 240, 345-350. 10.1177/1535370214561956.
41. Abu-Remaileh, M., Abu-Remaileh, M., Akkawi, R., Knani, I., Udi, S., Pacold, M.E., Tam, J., and Aqeilan, R.I. (2019). WWOX somatic ablation in skeletal muscles alters glucose metabolism. Mol Metab 22, 132-140. 10.1016/j.molmet.2019.01.010.
42. Saluda-Gorgul, A., Seta, K., Nowakowska, M., and Bednarek, A.K. (2011). WWOX oxidoreductase--substrate and enzymatic characterization. Zeitschrift fur Naturforschung. C, Journal of biosciences 66, 73-82.
43. Chen, S.T., Chuang, J.I., Wang, J.P., Tsai, M.S., Li, H., and Chang, N.S. (2004). Expression of WW domain-containing oxidoreductase WOX1 in the developing murine nervous system. Neuroscience 124, 831-839. 10.1016/j.neuroscience.2003.12.036.
44. Chiang, M.F., Chen, S.T., Lo, C.P., Sze, C.I., Chang, N.S., and Chen, Y.J. (2013). Expression of WW domain-containing oxidoreductase WOX1 in human nervous system tumors. Anal Cell Pathol (Amst) 36, 133-147. 10.3233/ACP-140087.
45. Jakel, S., Agirre, E., Mendanha Falcao, A., van Bruggen, D., Lee, K.W., Knuesel, I., Malhotra, D., Ffrench-Constant, C., Williams, A., and Castelo-Branco, G. (2019). Altered human oligodendrocyte heterogeneity in multiple sclerosis. Nature 566, 543-547. 10.1038/s41586-019-0903-2.
46. International Multiple Sclerosis Genetics, C., Beecham, A.H., Patsopoulos, N.A., Xifara, D.K., Davis, M.F., Kemppinen, A., Cotsapas, C., Shah, T.S., Spencer, C., Booth, D., Goris, A., et al. (2013). Analysis of immune-related loci identifies 48 new susceptibility variants for multiple sclerosis. Nat Genet 45, 1353-1360. 10.1038/ng.2770.
47. Ziliotto, N., Marchetti, G., Scapoli, C., Bovolenta, M., Meneghetti, S., Benazzo, A., Lunghi, B., Balestra, D., Laino, L.A., Bozzini, N., Guidi, I., et al. (2019). C6orf10 Low-Frequency and Rare Variants in Italian Multiple Sclerosis Patients. Front Genet 10, 573. 10.3389/fgene.2019.00573.
48. International Multiple Sclerosis Genetics, C. (2019). Multiple sclerosis genomic map implicates peripheral immune cells and microglia in susceptibility. Science 365. 10.1126/science.aav7188.
49. Matsushita, T., Madireddy, L., Sprenger, T., Khankhanian, P., Magon, S., Naegelin, Y., Caverzasi, E., Lindberg, R.L., Kappos, L., Hauser, S.L., Oksenberg, J.R., et al. (2015). Genetic associations with brain cortical thickness in multiple sclerosis. Genes, brain, and behavior 14, 217-227. 10.1111/gbb.12190.
50. Hussain, T., Kil, H., Hattiangady, B., Lee, J., Kodali, M., Shuai, B., Attaluri, S., Takata, Y., Shen, J., Abba, M.C., Shetty, A.K., et al. (2019). Wwox deletion leads to reduced GABA-ergic inhibitory interneuron numbers and activation of microglia and astrocytes in mouse hippocampus. Neurobiol Dis 121, 163-176. 10.1016/j.nbd.2018.09.026.
51. Bednarek, A.K., Laflin, K.J., Daniel, R.L., Liao, Q., Hawkins, K.A., and Aldaz, C.M. (2000). WWOX, a novel WW domain-containing protein mapping to human chromosome 16q23.3-24.1, a region frequently affected in breast cancer. Cancer Res 60, 2140-2145.
52. Ried, K., Finnis, M., Hobson, L., Mangelsdorf, M., Dayan, S., Nancarrow, J.K., Woollatt, E., Kremmidiotis, G., Gardner, A., Venter, D., Baker, E., et al. (2000). Common chromosomal fragile site FRA16D sequence: identification of the FOR gene spanning FRA16D and homozygous deletions and translocation breakpoints in cancer cells. Hum Mol Genet 9, 1651-1663.
53. Abdeen, S.K., and Aqeilan, R.I. (2019). Decoding the link between WWOX and p53 in aggressive breast cancer. Cell Cycle 18, 1177-1186. 10.1080/15384101.2019.1616998.
54. Khawaled, S., Nigita, G., Distefano, R., Oster, S., Suh, S.S., Smith, Y., Khalaileh, A., Peng, Y., Croce, C.M., Geiger, T., Seewaldt, V.L., et al. (2020). Pleiotropic tumor suppressor functions of WWOX antagonize metastasis. Signal Transduct Target Ther 5, 43. 10.1038/s41392-020-0136-8.
55. Khawaled, S., Suh, S.S., Abdeen, S.K., Monin, J., Distefano, R., Nigita, G., Croce, C.M., and Aqeilan, R.I. (2019). WWOX Inhibits Metastasis of Triple-Negative Breast Cancer Cells via Modulation of miRNAs. Cancer Res 79, 1784-1798. 10.1158/0008-5472.CAN-18-0614.
56. Gardenswartz, A., and Aqeilan, R.I. (2014). WW domain-containing oxidoreductase's role in myriad cancers: Clinical significance and future implications. Experimental Biology and Medicine 239, 253-263. 10.1177/1535370213519213.
57. Baryla, I., Styczen-Binkowska, E., and Bednarek, A.K. (2015). Alteration of WWOX in human cancer: a clinical view. Exp Biol Med (Maywood) 240, 305-314. 10.1177/1535370214561953.
58. Abdeen, S.K., Salah, Z., Khawaled, S., and Aqeilan, R.I. (2013). Characterization of WWOX inactivation in murine mammary gland development. Journal of cellular physiology 228, 1391-1396. 10.1002/jcp.24310.
59. Ferguson, B.W., Gao, X., Kil, H., Lee, J., Benavides, F., Abba, M.C., and Aldaz, C.M. (2012). Conditional Wwox deletion in mouse mammary gland by means of two Cre recombinase approaches. PloS one 7, e36618. 10.1371/journal.pone.0036618.
60. Wolf, G., Lifschytz, T., Ben-Ari, H., Tatarskyy, P., Merzel, T.K., Lotan, A., and Lerer, B. (2018). Effect of chronic unpredictable stress on mice with developmental under-expression of the Ahi1 gene: behavioral manifestations and neurobiological correlates. Transl Psychiatry 8, 124. 10.1038/s41398-018-0171-1.
61. Greenbaum, L., Lifschytz, T., Zozulinsky, P., Broner, E.C., Slonimsky, A., Kohn, Y., and Lerer, B. (2012). Alteration in RGS2 expression level is associated with changes in haloperidol induced extrapyramidal features in a mutant mouse model. Eur Neuropsychopharmacol 22, 379-386. 10.1016/j.euroneuro.2011.09.006.

### Example 3: Modeling genetic epileptic encephalopathies using brain organoids

### Introduction

Epilepsy is a neurological disorder characterized by a chronic predisposition for the development of recurrent seizures (Fisher *et al,* 2014; Aaberg *et al,* 2017). Epilepsy affects around 50 million people worldwide and is considered the most frequent chronic neurological condition in children (Aaberg *et al,* 2017; Blumcke *et al,* 2017). Approximately 40% of seizures in the early years of life are accounted for by developmental and epileptic encephalopathy (DEE), previously known as early infantile epileptic encephalopathies (EIEEs) (Howell *et al,* 2021). These are pathologies of the developing brain, characterized by intractable epileptiform activity and impaired cerebral and cognitive functions (Lado *et al,* 2013; Shao & Stafstrom, 2016; Nashabat *et al,* 2019; Howell *et al,* 2021). Several genes have been implicated in causing DEEs (McTague *et al,* 2016). In recent years, autosomal recessive mutations in WWOX gene are increasingly recognized for their role in the pathogenesis of DEE (Piard *et al,* 2018; Nashabat *et al,* 2019). WWOX, a tumor suppressor that spans the chromosomal fragile site FRA16D, is highly expressed in the brain, suggesting an important role in central nervous system (CNS) homeostasis (Abu-Remaileh *et al,* 2015). In 2014, WWOX was implicated in the autosomal recessive spinocerebellar ataxia-12 (*SCAR12*) (Gribaa *et al,* 2007; Mallaret *et al,* 2014) and in the *WWOX-*related epileptic encephalopathy (WOREE syndrome, also termed DEE28) (Abdel-Salam *et al,* 2014; Ben-Salem *et al,* 2015; Mignot *et al,* 2015). Both disorders are associated with a wide variety of neurological symptoms, including seizures, intellectual disability, growth retardation, and spasticity, but differ by severity, onset, and underlying types of mutations. The WOREE syndrome is considered more aggressive, appearing as early as 1.5 months and associating with more extreme genetic changes (Banne *et al,* 2021). This observation may imply that both syndromes can be considered as a continuum. Alongside seizures, patients with WOREE syndrome may present with global developmental delay, progressive microcephaly, atrophy of specific CNS components, and premature death. However, it is important to note that the phenotypic spectrum of WOREE syndrome is wide, with different patients exhibiting different symptoms. For example, although microcephaly is seen in some patients, many other do not exhibit this condition (Piard *et al,* 2018). Although modeling WWOX loss of function in rodents has shed some lights on the roles of WWOX in the mammalian brain (Aqeilan *et al,* 2007, 2008; Suzuki *et al,* 2009; Mallaret *et al,* 2014; Tanna & Aqeilan, 2018; Tochigi *et al,* 2019), the genetic background and brain development of a specific patient cannot be modeled in a mouse, but is inherent and retained in patient-derived induced pluripotent stem cells (iPSCs). In an effort to bypass the comprehensible lack of availability of DEE brain samples, including those of patients with *WWOX* mutations, we utilized genome editing and reprogramming technologies to recapitulate the genetic changes seen in patients with WOREE and SCAR12 syndromes in human PSCs. We then generated brain organoids, 3D neuronal cultures, that recapitulate much of the brain's spatial organization and cell type formation, and have neuronal functionality *in vitro* (Amin & Pa 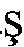 ca, 2018; Sidhaye & Knoblich, 2020). This allowed us to model features of the development and maturation of the CNS and its complex circuitry, in a system that is more representative of the *in vivo* human physiology than 2D cell cultures. Using this platform, we identified severe defects in neural cell populations, cortical formation, and electrical activity, and tested possible rescue strategies. This approach has resulted in a deeper understanding of WWOX physiology and pathophysiology in the CNS, laying the foundation for developing more appropriate treatments, and supports the concept of using human brain organoids for modeling other human epileptic diseases.

### Results

### Generation and characterization of WWOX knockout cerebral organoids

To shed light on the pathogenesis of DEE, we studied the WOREE syndrome as a prototype model using brain organoids. The role of WWOX in the development of the human brain in a controlled genetic background was investigated by generating WWOX knockout (KO) clones of the WiBR3 hESC line using the CRISPR/Cas9 system (Abdeen *et al,* 2018). Immunoblot analysis was used to assess WWOX expression in these lines. Two clones that showed consistent undetectable protein levels of WWOX throughout our validations were picked for the continuation of the study-WWOX-KO line 1B (WKO-1B, from here on KO1) and WKO-A2 (from here on KO2). These clones were assessed for genetic stability and pluripotency using karyotype analysis and teratoma assays, respectively. Sanger sequencing confirmed editing of *WWOX* at exon 1. Furthermore, to confirm cell-autonomous function of WWOX, we restored *WWOX* cDNA into the endogenous AAVS locus of WWOX-KO1 hESC line and examined reversibility of the phenotypes. The KO1-AAV4 line was selected for generating COs for having a strong and stable expression of WWOX throughout our validations, and from here on is referred as W-AAV. These lines were practically indistinguishable from the parental cell line (WiBR3 WT) in terms of morphology and proliferation throughout the culture period.

To investigate how depletion of WWOX affects cerebral development in a 3D context, we differentiated our hESCs into cerebral organoids (COs), using an established protocol (Lancaster *et al,* 2013; Lancaster & Knoblich, 2014). COs from all genotypes showed comparable gross morphology and development at all stages. Next, we investigated the expression pattern of WWOX in the developing brain at different time points by co-staining with markers of the two major populations found in the organoids-neuronal progenitor cells and neurons. In week 10, WWOX expression was specifically localized to the ventricular-like zone (VZ), which is composed of SOX2⁺ cells, corresponding to radial glial cells (RGs), the progenitors of the brain, and not in the surrounding cells. This finding is in concordance with previous work showing limited WWOX expression during early steps of mouse cortical development (Chen *et al,* 2004). To confirm the identity of the WWOX-expressing cells in the VZ, we co-stained for crystallin alpha B (CRYAB), which is specifically expressed in ventricular radial glial cells (vRGs) (Pollen *et al,* 2015), and confirmed WWOX expression in these cells (Fig. 11B). Furthermore, even in later time points, such as week 24, when the VZ structure is lost, WWOX expression is found mainly in SOX2⁺ cells. Importantly, WWOX expression was not seen in COs generated from the WWOX-KO lines, although similar levels of expression of the other markers such as SOX2 and neuron-specific class III β-tubulin (TUBB3 or TUJ1) were observed (Fig. 11B). Interestingly, the W-AAV COs, in which WWOX expression is driven by human ubiquitin promoter (UBP), exhibited high WWOX levels in the VZ, as expected, though other cellular populations also showed prominent WWOX expression.

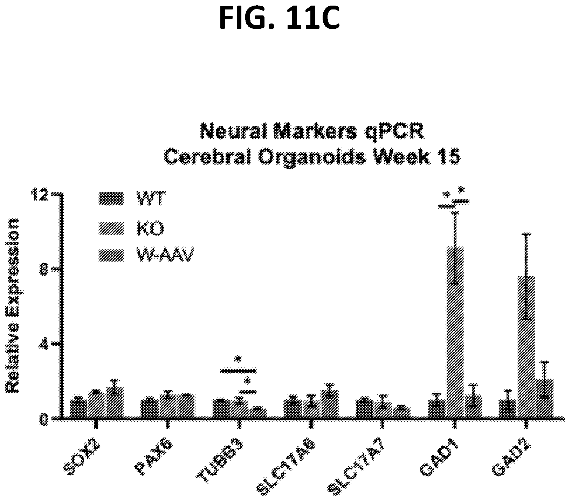

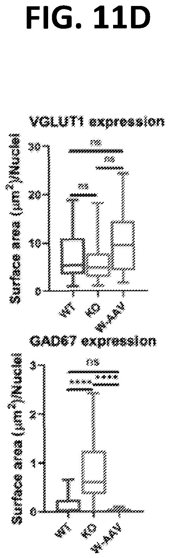

Next, to address the microcephalic phenotype observed in some patients, we measured the diameter of our organoids through the culture period, which showed no significant difference. This led us to examine the development of the histological cerebral structures. In WT organoids, the VZ, which is composed of SOX2⁺ cells, is surrounded by intermediate progenitors (IP; TBR2⁺ cells, also known as EOEMS), marking the presence of the subventricular zone (SVZ). Outside this layer is the cortical plate (CP), composed mainly of neurons (NeuN⁺ cells). In week 10 COs, no visible differences in the composition or formation of the VZ and the surrounding structures were observed (Fig. 11C), suggesting similar proportions of these populations. This was also supported by measuring RNA expression levels of progenitor markers (SOX2 and PAX6) and the neuronal marker *TUBB3* (Fig. 11C). This surprising observation led us to further examine the two major neuronal subpopulations found in COs-glutamatergic (marked by vesicular glutamate transporter 1, VGLUT1) and GABAergic neurons (marked by glutamic acid decarboxylase 67, GAD67). Immunostaining revealed that although VGLUT1 expression remained similar, a marked increase in expression of GAD67 was observed in KO COs compared with WT. In contrast, WWOX restoration (W-AAV) significantly reversed this imbalance (Fig. 11D). RNA levels of *SLC17A6 (VGLUT2), SLC17A7 (VGLUTI), GAD1 (GAD67)* and *GAD2 (GAD65)* followed the same trend.

These findings suggest that during human embryonic development, WWOX expression is limited to the cells of the apical layer of the VZ, and that WWOX depletion did not affect the VZ-SVZ-CP architecture but did disrupt the balance between glutamatergic and GABAergic neurons.

### WWOX-depleted cerebral organoids exhibited hyperexcitability and epileptiform activity

Cerebral organoids give rise to neurons that have previously shown electrophysiological functionality (Trujillo *et al,* 2019). To characterize the functional properties of the WWOX-KO COs, we performed local field potential recordings (LFP) on 7-week CO slices. Electrodes were positioned 150 µm away from the edge of the slice (data not shown) to avoid areas potentially damaged by slice preparation. Sample traces of the WT and KO COs revealed visible differences between the two lines under baseline conditions (Fig. 12A, left). The mean spectral power of field recordings further showed an overall increase in power of the KO COs in the 0.25-1 Hz, typically labelled as slow-wave oscillations (SWO, < 1 Hz) (Fig. 12B), and a decrease in the 30- to 79.9-Hz high-frequency range. The oscillatory power (OP) was quantified by the area under the curve, which was significantly higher than the WT line, under baseline conditions (Fig. 12C). Over time, the OP of the KO lines decreased significantly, while the WT line's OP stayed the same, suggesting a developmental delay in the KO line.

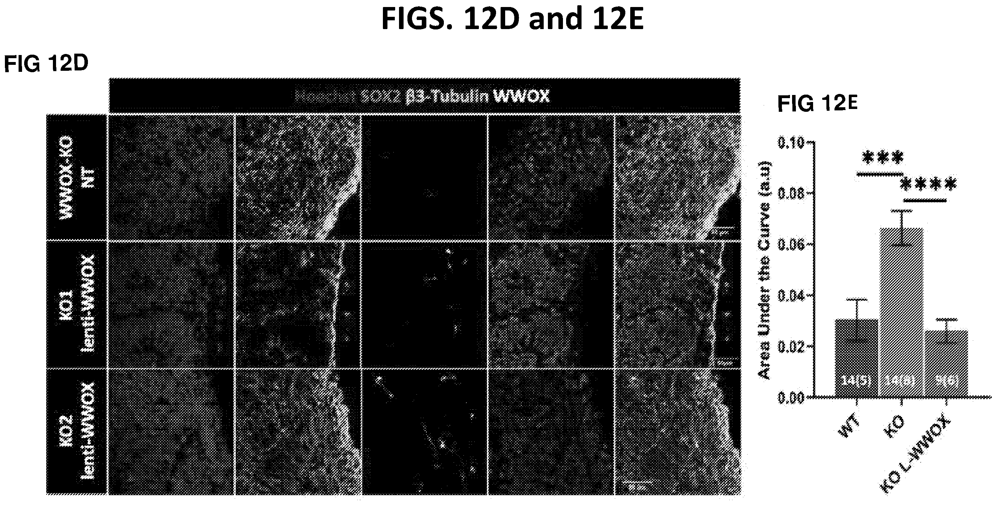

To further measure the hyperexcitability of the KO line, 100 µM 4-AP, a commonly used convulsant for seizure induction, was applied to the slices during recordings. While 4-AP did show changes in LFP recordings for both WT and KO lines (Fig. 12A, right), the KO line showed significantly increased activity, which was otherwise absent in WT traces. The effect of 4-AP on spectral power became evident 5 min after its addition. Cross-frequency coupling of the sample traces for both WT and KO lines in the presence of 4-AP revealed an increase in the δ: HFO frequency pairs-an attribute that has previously been used to characterize and classify seizure substates (Guirgis *et al,* 2013). Importantly, transduction of a lentivirus containing *WWOX* cDNA resulted in recovery of the KO line, with respect to the mean power spectral density (Fig. 12D and E).

### WWOX-depleted cerebral organoids exhibited impaired astrogenesis and DNA damage response

It is widely accepted that an imbalance between excitatory and inhibitory activity in the brain is a leading mechanism for seizures, but this does not necessarily mean neurons are the only population involved. It is well known that brain samples from epileptic patients show signs of inflammation, astrocytic activation, and gliosis (Cohen-Gadol *et al,* 2004; Thom, 2009), which can be a sole histopathological finding in some instances (Blumcke *et al,* 2017). Whether this phenomenon is a result of the acute insult or a cause of the seizures is still debatable (Vezzani *et al,* 2011; Robel *et al,* 2015; Rossini *et al,* 2017; Patel *et al,* 2019). Furthermore, recent work has demonstrated astrogliosis in the brain of Wwox-null mice (Hussain *et al,* 2019).

To address this, we used immunofluorescence staining to visualize the astrocytic markers glial fibrillary acidic protein (GFAP) and S100 calcium-binding protein B (S100β) in week 15 and week 24 COs (Fig. 13A-C). This revealed a marked increase in astrocytic cells in WWOX-KO COs that progressed through time, and was partially reversed in W-AAV COs. This was further supported by immunoblot analysis of week 20 COs. It is notable that GFAP also marks RGs (Middeldorp *et al,* 2010), which are abundant in week 15, but are reduced in number in week 24, which could be a source of noise at early stages.

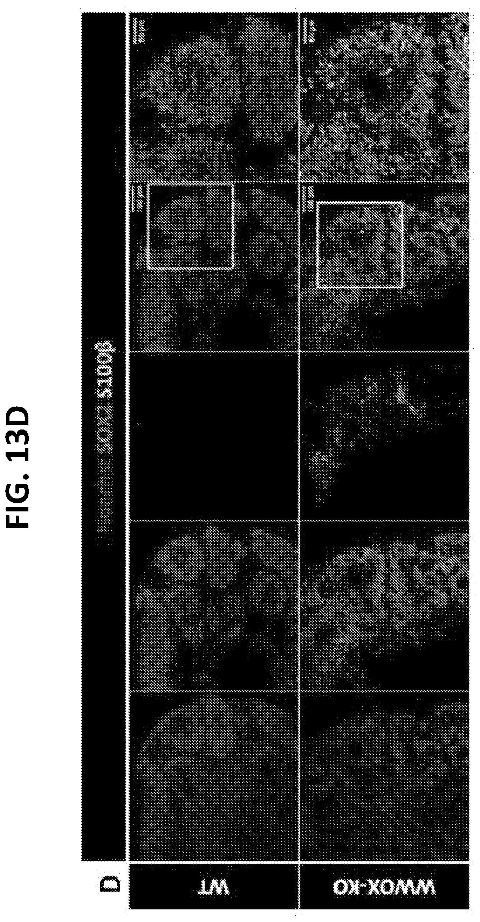

Astrocytes arise from two distinct populations of cells in the brain: the RG cells, switching from neurogenesis to astrogenesis, or the astrocyte progenitor cells (APCs) (Zhang *et al,* 2016; Blair *et al,* 2018). To track back these differences in astrocyte markers, we compared 6- and 10-week-old COs. We found that in week 6 COs, where no astrocytic markers were detected in WT organoids, a significant expression of S100β was observed in the VZ (Fig. 13D). At week 10, we observed expression of S100β in both WT and KO organoids, but when co-staining is performed with the cell proliferation marker Ki67, we did not detect a significant difference in double-positive cells suggesting similar glial proliferation. Quantification of Ki67⁺ nuclei, together with SOX2⁺ nuclei, revealed that although the proportions of SOX2 remained intact in WT compared with WWOX-KO (18.5% in WT, 95% CI = 14.2-22.81; 19.5% in KO, 95% CI = 15.29-22.81), the proportions of proliferating cells (9.5% in WT, 95% CI = 6.63-12.35; 4.9% in KO, 95% CI = 2.76- 7.13) and Ki67⁺/SOX2⁺ double-positive cells were diminished (51.83% in WT, 95% CI = 41.18-62.48; 27.09% in KO, 95% CI = 14.1-40.1). These findings imply that although overall proliferation of SOX2⁺ cells is decreased upon loss of WWOX, the total amount of RGs outside the VZ is not affected, raising a question in regard to the source of the astrocytic cells.

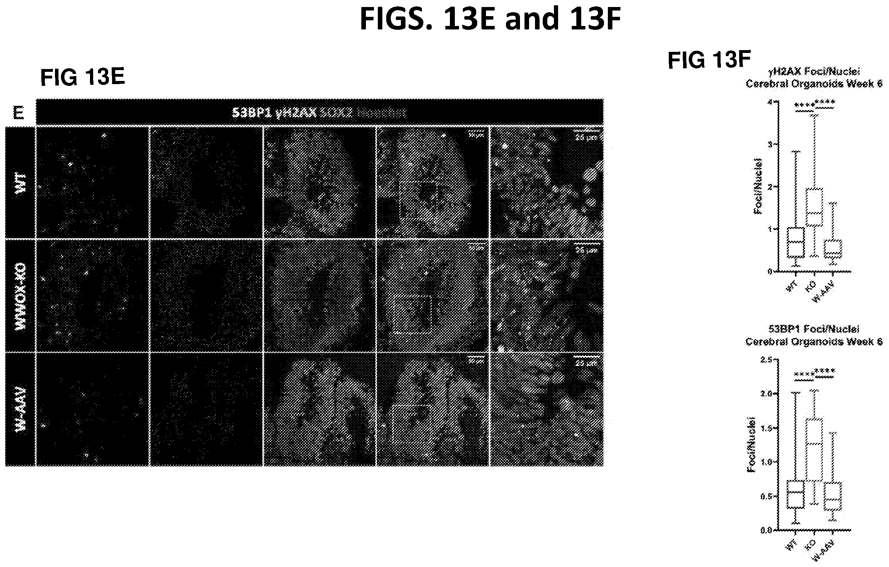

This peculiar behavior of the vRGs in KO COs led us to take a closer look at their functionality by examining their physiological DNA damage response (DDR), a signaling pathway in which WWOX is known to be directly involved (Abu-Odeh *et al,* 2014b; Abu-odeh *et al,* 2016). To this end, we stained for γH2AX and 53BP1, surrogate markers for DNA double-strand breaks. We found a marked accumulation of γH2AX and 53BP1 foci in the nuclei of SOX2⁺ cells in the innermost layer of the VZ, averaging 1.5 foci/nuclei [95% CI =1.33-1.74] and 1.2 foci/nuclei [95% CI = 1-1.38] in WWOX-KO, respectively. This is in comparison with 0.78 γH2AX foci/nuclei [95% CI = 0.55-1.02] and 0.62 53BP1 foci/nuclei in the age-matched WT COs, and with 0.58 foci/nuclei [95% CI = 0.38-0.77] and 0.56 foci/nuclei [95% CI = 0.37-0.76] in the age-matched W-AAV COs (Fig. 13E and F). These findings are consistent with WWOX direct role in DDR signaling (Aqeilan *et al,* 2014; Hazan *et al,* 2016). Importantly, W-AAV COs presented with improved DDR. Intriguingly, a higher number of γH2AX foci were found in highly proliferating cells in the VZ, which was observed by co-staining with Ki67-18.6% of SOX2⁺ cells were double-positive in KO COs [95% CI = 15-22%] compared with 11.9% [95% CI = 8-15%]. This led us to speculate that the continued proliferation of damaged cells might be accompanied by diminished apoptosis, which might indicate loss of checkpoint inhibition. This hypothesis was addressed by staining for cleaved caspase-3 in the VZ, which revealed a decline in apoptosis of these cells upon WWOX-KO, and was rescued in the W-AAV COs.

In conclusion, WWOX-KO COs present with a progressive increase in astrocytic number, likely due to enhanced differentiating RGs, and with increased DNA damage in neural progenitor cells.

### RNA-sequencing of WWOX-depleted cerebral organoids revealed major differentiation defects

In an effort to examine the molecular profiles, we performed whole-transcriptome RNA-sequencing (RNA-seq) analysis on week 15 WT and KO COs. Albeit the known heterogeneity of brain organoids, principal component analysis (PCA) separated the sample into two distinct clusters. The analysis revealed 15,370 differentially expressed genes, of which 1,246 genes were upregulated in WWOX-KO COs and showed both a fold change greater than 1.2 (FC > 1.2) and a significant P-value (P < 0.01), and 1,021 genes were downregulated (FC < 1/1.2, *P <* 0.01). Among the top 100 upregulated genes, we found genes related to neural populations such as GABAergic neurons (*GAD1, GRM7, LHX5)* and astrocytes (*AGT, S100A1, GJA1, OTX2*), and to neuronal processes such as calcium signaling (*HRC, GRIN2A, ERBB3, P2RX3, HTR2C, PDGFRA*) and axon guidance (*GATA3, DRGX, ATOH1, NTN1, SHH, RELN, OTX2, SLIT3, GBX2, LHX5*)*.* In the top 100 downregulated genes were genes related to GABA receptors (*GABRB3, GABRB2),* autophagy (*IFI16, MDM2, RB1, PLAT, RB1CC1*), and the mTOR pathway (*EIF4EBP1, PIK3CA, RB1CC1*).

Gene set enrichment analysis (GSEA) and gene ontology (GO) enrichment analysis of the top 3,000 differentially expressed genes revealed, among others, inhibition of processes related to ATP synthesis-coupled electron transport and oxidative phosphorylation, all of which are consistent with previous reported functions of WWOX in mouse models (Abu-Remaileh & Aqeilan, 2014, 2015; Abu-Remaileh *et al,* 2018). Downregulation of genes related to negative regulation of cell cycle was seen, consistent with the previously reported diminished checkpoint inhibition (Abu-Odeh *et al,* 2014b; Abu-odeh *et al,* 2016). On the other hand, marked enrichment was seen in pathways related to regionalization, neuron fate commitment and specification, axis specification (ventral-dorsal and anterior-posterior), and glycolysis and gluconeogenesis, some of which are also supported by past studies (Wang *et al,* 2012; Abu-Remaileh & Aqeilan, 2014). As could be anticipated, upregulated genes were related to the development pathways such as Wnt pathway (*e*.*g*., WNT1, WNT2B, WNT3, WNT3A, WNT5A, WNT8B, LEF1, AXIN2, GBX2, ROR2, LRP4, NKD1, IRX3, CDH1) and the Shh pathway (e.g., SHH, GLI1, LRP2, PTCH1, HHIP, PAX1, PAX2).

Since WWOX has been previously implicated in the Wnt signaling pathway (Bouteille *et al,* 2009; Wang *et al,* 2012; Abu-Odeh *et al,* 2014a; Cheng *et al,* 2020; Khawaled *et al,* 2020), we set to further explore this in our CO models. First, we used our RNA-seq data to inspect the expression of different members of the WNT signaling pathway (such as WNT1, WNT3, WNT5A, WNT8B), of canonical targets (such as Axin2, TCF7L2, LEF1, TCF7L1), of brain-specific targets (IRX3, ITGA9, GATA2, FRAS1, SP5), and of receptors (ROR2, FZD2, FZD10, FZD1). We next validated some of these genes using qPCR (Fig. 14A). We also observed downregulation of some of the Wnt-related genes in W-AAV COs, including WNT3, WNT3A, WNT1, and ROR2 (Fig. 14A). Additionally, to prove Wnt activation, we demonstrated β-catenin translocation into the nucleus-a hallmark of the canonical Wnt pathway. To this end, week 16 COs were subfractionated into cytoplasmic and nuclear fractions and immunoblotted (Fig. 14B). We found an approximate 1.7-fold increase in the normalized intensity of β-catenin in the nucleus of WWOX-KO COs, supporting the notion of Wnt pathway activation after the loss of WWOX. This was also supported by examining kinetic expression of several Wnt-related genes (WNT3, AXIN2, LEF1, and TCF7) at weeks 6-24, which revealed a chronic Wnt activation in KO COs as compared to progressive decrease in WT COs.

Recent evidence has demonstrated that activation of Wnt in forebrain organoids during development causes a disruption of neuronal specification and cortical layer formation (Qian *et al,* 2020). To address whether this occurs in WWOX-KO COs, we examined the expression levels of cortical layer markers (Qian *et al,* 2016) in our RNA-seq data. Interestingly, changes were observed in all six layers, with layers I-IV (marked by TBR1, BCL11B, SATB2, POU3F2) showing decreased expression and superficial layers V-VI (marked by CUX1 and RELN) exhibiting marked increase. This pattern was also confirmed by qPCR. Intriguingly, when we examined protein levels using immunofluorescent staining, we also observed impaired expression patterns and layering, with TBR1⁺, CTIP2⁺ (BCL11B), and SATB2⁺ neurons intermixing in WWOX-KO COs (Fig. 17D and E). This defect was progressive, worsening at week 24. Surprisingly, when examining the effect of ectopic WWOX expression, a less clear phenotype was observed; although CTIP2⁺ cell and SATB2⁺ cell numbers recovered and layering improved in W-AAV COs compared with WWOX-KO COs, RNA levels did only partially. In contrast, expression of the superficial layer markers CUX1 and RELN, which was upregulated in WWOX-KO, decreased in W-AAV, together with the upper layer marker POU3F2 (BRN2). Importantly, when examining the expression of dorsal and ventral genes in COs, we observed that the organoids are of dorsal identity, as assessed by high RNA read counts. Of note, we did not observe any statistically significant differences between WT and KO in the expression of these markers.

Overall, RNA-seq reveled impaired spatial patterning, axis formation, and cortical layering in WWOX-KO COs, which is correlated with disruption of cellular pathways and activation of Wnt signaling. The reintroduction of WWOX prevents these changes to some extent, further supporting its possible implication in gene therapy.

### Brain organoids of patient-derived WWOX-related developmental and epileptic encephalopathies

Although disease modeling using CRISPR-edited cell is a widely used tool, critiques argue against it for not modeling the full genetic background of the human patients. Therefore, we reprogrammed peripheral blood mononuclear cells (PBMCs) donated from two families with WWOX-related diseases, differing in their severity: The first family carries a c.517-2A>G splice site mutation (Weisz-Hubshman *et al,* 2019) that results in the WOREE syndrome (DEE28) phenotype in the homozygous patient (referred to as WSM family); and the second family carries a c.1114G>C (G372R) mutation (Mallaret *et al,* 2014) that results in the SCAR12 phenotype in the homozygous patient (referred to as WPM family). All the iPSC lines showed normal morphology for primed hPSCs and self-renewal capabilities and were evaluated for expression of pluripotent markers.

We then proceeded to generate COs from iPSCs isolated from the healthy, heterozygote parents of the WSM family (the father, referred to as WSM F1; and the mother, referred to as WSM M2, collectively referred to as WSM P), and from the sick homozygote son (lines WSM S2 and S5, referred to collectively as WSM S). Additionally, we employed the rescue approach descried for W-AAV COs and reintroduced *WWOX* into the WSM S5 line (referred to as WSM S5 W-AAV3 and W-AAV6, and collectively as WSM S W-AAV). These organoids were then validated for neuronal differentiation and VZ formation (Fig. 15A). Similar to the hESC-derived COs, WWOX was mainly expressed in the VZ in WSM F1 and WSM M2. Although β3-Tubulin⁺-positive cells and SOX2⁺-positive cells were comparable in numbers, WSM S COs showed no detectable levels of WWOX, and WSM S W-AAV COs expressed WWOX globally.

Next, to evaluate the neuronal hyperexcitability of the WSM S COs, we performed cell-attached recordings from 41 WSM S COs' neurons along with 24 neurons from WSM P COs and 40 neurons from WSM S W-AAV organoids. Sample traces with the spontaneous firing of action potentials from the WSM S, WSM P, and WSM S W-AAV COs revealed visible differences between the three groups recorded under the same conditions. WSM S COs demonstrated bursts of action potentials and overall elevated neuronal activity compared with the WSM P and WSM S W-AAV organoids (Fig. 15B). The WSM S COs' neurons showed a drastic increase in the firing rate (about a fourfold) compared with the WSM P (*P <* 0.0001) and WSM S W-AAV (*P* < 0.0001) COs' neurons (Fig. 15C). There was no significant difference between the firing rate of WSM P and WSM S W-AAV COs' neurons (*P* = 0.7681). Importantly, no significant difference was observed when comparing COs from WSM F1 line to WSM M2 line (P = 0.0952). Overall, our results demonstrate neuronal hyperexcitability in 7-week-old organoids derived from the patient with WOREE syndrome compared with his or her parents.

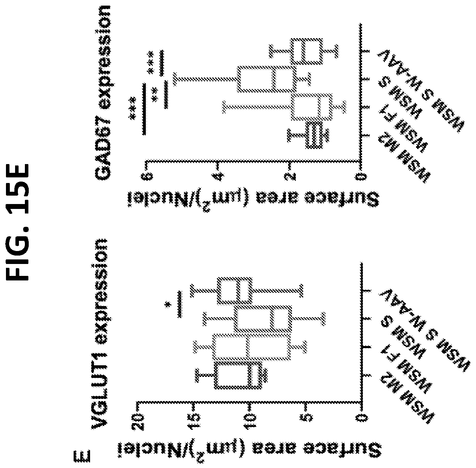

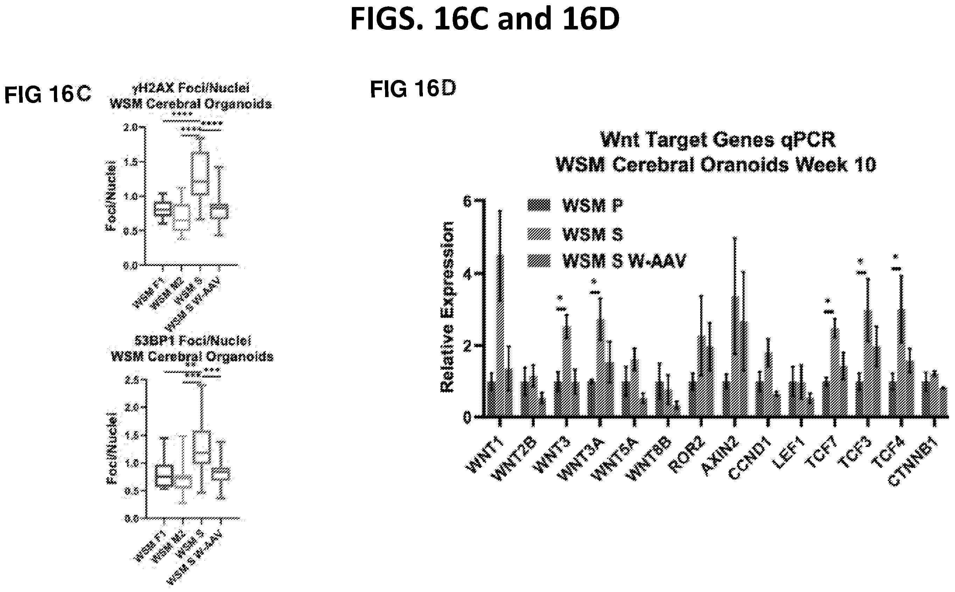

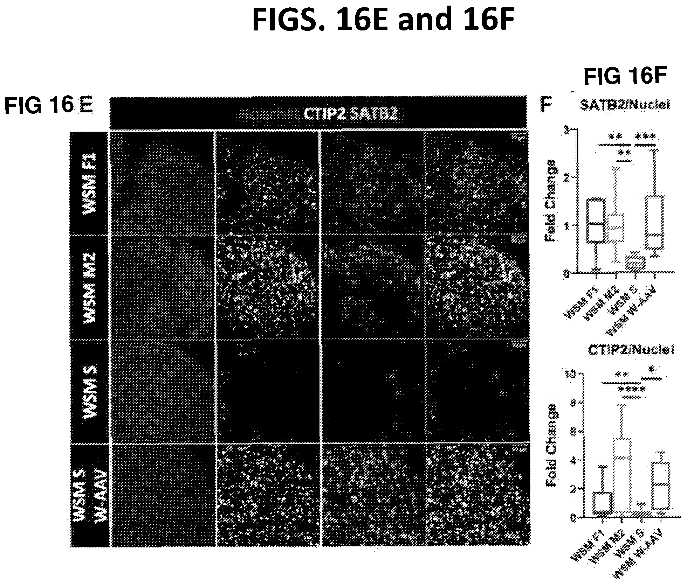

Consistent with our other findings, week 10 WSM S COs exhibited increased expression of GAD67 compared with WSM F1 and WSM M2, a phenotype that was reversed in WSM S W-AAV COs (Fig. 15D and E). The expression of VGLUT1 remained unchanged between the different lines. We next assessed the expression of astrocytic markers and found increased expression of GFAP and S100β in WSM S compared with age-matched controls (Fig. 16A). DDR defects in vRGs of WSM S COs were also apparent (Fig. 16B and C). The status of the Wnt pathway was also assessed using qPCR, with findings suggestive of activation in week 10 WSM S COs compared with the age-matched WSM P and WSM S W-AAV (Fig. 16D). Finally, we evaluated cortical lamination using immunofluorescence and found decreased expression of cortical markers CTIP2⁺ and SATB2⁺ in WSM S COs (Fig. 16E and F).

Since a major part of the phenotype was observed in the cortical areas of the COs, and given the demonstrated role for *WWOX* in the cortex, we decided to employ a cortex-specific protocol and generate forebrain organoids (FOs) (Qian *et al,* 2016, 2018). First, to validate reproducibility, we generated FOs from WSM F1 and WSM S5 and found comparable phenotypes for WSM COs.

We next sought to study whether the WPM SCAR12 family, whose patients have a milder phenotype, present with similar phenotypes to our COs and FOs of WOREE syndrome. We generated FOs from the healthy heterozygous father and mother (WPM F2 and WPM M3) and their affected homozygous daughter and son (WPM D1 and WPM S1). As expected, FOs were indistinguishable in terms of morphology, growth, and expression of β3-Tubulin and SOX2, but while in the VZ of WPM F2 and WPM M3, WWOX was detected, barely any signal was observed in WPM D1 and S1, consistent with WWOX levels in the iPSCs. Dorsal forebrain identity was validated through staining for PAX6. Surprisingly, transcript expression levels of neuronal markers did not show any clear difference in the ratio between glutamatergic and GABAergic neurons. Although some differences were seen between FOs from lines with similar genotypes, the comparable levels of cortical layers' marker expression between the healthy iPSC lines (WPM F2 and WPM M3) and the disease-bearing lines (WPM D1 and WPM S1) supported the notion of normal neuronal and cortical development. Interestingly, RNA levels of Wnt genes did show a pattern suggestive of the Wnt pathway activation, which raises a question regarding its role in the pathogenesis of the milder disease. Furthermore, immunostaining and qPCR analyses for astrocytic levels did not reveal significant differences. Lastly, upon analyzing the DDR signaling in the FOs' VZ, we did not observe major differences in accumulation of DNA damage foci between healthy and sick SCAR12 individuals. Altogether, these data suggest different developmental outcomes between SCAR12 and WOREE syndrome-derived organoids.

### Discussion

DEEs are a group of severe neurological syndromes whose underlying molecular pathology is unknown (Howell *et al,* 2021). Together with the lack of accessibility of human samples, it is not surprising that the current medical treatment is lacking. Our study set out to utilize the major technological advances in developmental biology, together with the role of WWOX in the severe WOREE syndrome, to model human refractory DEEs in a tissue-relevant context. By utilizing genetic manipulations and reprogramming, along with electro-physiology, we observed hyperexcitability in both *WWOX* CRISPR-edited and patient-derived brain organoids, therefore successfully demonstrating epileptiform activity. We then further examined the cellular and molecular changes highlighting possible mechanisms for the disease pathophysiology. First, although the neuronal population was largely intact in terms of quantity, we noticed a marked increase in GABAergic markers. This finding is even more surprising when considering the decrease in GABA receptor components seen by RNA-seq. This can indicate a disruption in development of normal and balanced neuronal networks, supporting the increased electrical activity observed in these organoids. It should be noted that several lines of evidence implicate that during development, GABAergic synapses have a depolarizing effect (Obata *et al,* 1978; Ben-Ari *et al,* 2007; Murata & Colonnese, 2020). Seizure dynamics in developmental epilepsies are known to be dependent on depolarizing GABA responses, particularly due to an accumulation of intracellular chloride resulting in a depolarized chloride reversal potential, thereby causing increased excitability, instead of hyperpolarization upon activation of GABA_{A} receptors (Khalilov *et al,* 2005; Ben-Ari *et al,* 2007). The evidence of increased mean spectral power in WWOX-depleted COs and WSM FOs, and its recovery in the presence of lentivirus containing WWOX, further strengthens the idea that depolarizing GABA plays a key role in seizure susceptibility. These findings shed a new light on the lack of efficacy of common anticonvulsant therapies on immature neurons (Khalilov *et al,* 2005; Murata & Colonnese, 2020)-making WWOX-depleted COs a useful model to test and study novel therapies targeting excitatory GABAergic responses. An increase in SWO has previously been linked to various stages of the seizure cycle-onset, throughout the seizure, and termination (Bragin & Engel, 2008). Previous studies have also demonstrated that SWOs modulate cortical excitability (Vanhatalo *et al,* 2004) and can localize the seizure-onset zone during the preictal period (Miller *et al,* 2007). Furthermore, slow waves have been identified as characteristic of EEG ictal activity in full-term and preterm infants (Patrizi *et al,* 2003). The mechanism for SWOs during seizure development is poorly understood. However, a few hypotheses suggest an increase in extracellular potassium, changes in the pH, glial cell dysfunction, and/or blood-brain barrier functions (Bragin & Engel, 2008). While exploring the mechanism is beyond the scope of this paper, these are interesting directions to explore in the future.

On the other hand, Fig 12A-C shows a decrease in higher frequency activity-beta (12-30 Hz) and gamma (> 30 Hz) oscillations. Although higher frequency activity-particularly gamma oscillations-is known to increase prior to seizure onset at specific focal zones and has been studied as a possible determinant of epileptogenesis (Medvedev *et al,* 2011), our data do not support this for WWOX-related seizures. One possible explanation for this is the maturity of the 7-week-old COs. Gamma oscillations are associated with functional connectivity and integrate neural networks within and across brain structures (Kheiri *et al,* 2013; Ahnaou *et al,* 2017). Lower power in these high-frequency ranges may be due to the delayed development and lower functional connectivity in WWOX-KO organoids. An interesting observation in our week 12 WSM FOs was that the activity in WSM S5 FOs was enhanced at high-frequency ranges. This contrast could be due to age, development protocol, or underlying mechanism for detected epileptiform activity. These-particularly the underlying mechanism-would need to be investigated further using in-depth single-cell analysis and through the use of targeted channel blockers and drugs.

Secondly, we closely examined other populations seen in brain organoids and found an increase in astrocytic markers, while the RG population, which express high levels of WWOX, seemed to maintain normal proportions. This pattern was detected early on and appeared to stem from the vRGs, and not from the APCs. A possible explanation is the impaired DDR signaling observed in WWOX-depleted organoids; previous studies in both ESC-derived and primary murine neural stem cells (NSCs) found that accumulation of DNA damage foci, either in the nuclear or in the mitochondrial DNA, causes NSCs to shift to astrocytic differentiation (Wang *et al,* 2011; Schneider *et al,* 2013). In the CNS, physiological DNA breaks can be formed by replicative stress (mainly in dividing progenitor cells), by oxidative and metabolic stress as a result of accumulation of reactive oxygen species (ROS), and even by neuronal activity (as part of developmental processes and learning) (Suberbielle *et al,* 2013; Madabhushi *et al,* 2014; Madabhushi *et al,* 2015). Impaired repair of these breaks is linked with CNS pathology and neurode-generation (Suberbielle *et al,* 2013; Madabhushi *et al,* 2014; Shanbhag *et al,* 2019). Our findings suggest a homeostatic role for WWOX in the vRGs, in which WWOX maintains proper DDR signaling in physiological conditions and prevents accumulation of DNA damage associated with impaired differentiation. It is important to note that although we chose to focus on vRGs for the DDR analysis, as they highly express WWOX, our data do not suggest these breaks accumulate specifically in vRGs and could possibly persist in the progenies.

Although the ability of brain organoids to develop functional synapses and complex neural network dynamics is rapidly being established through intensive research (Trujillo *et al,* 2019; Sidhaye & Knoblich, 2020), the capability to model epileptiform activity is only recently being studied (preprint: Samarasinghe *et al,* 2019; Sun *et al,* 2019). Sun *et al* (2019) utilized brain organoids to model Angelman syndrome using UBE3A-KO hESCs, recapitulating hyperactive neuronal firing, aberrant network synchronization, and the underlying channelopathy, which was observed in 2D and mouse models (Sun *et al,* 2019). Samarasinghe *et al* (2019) took advantage of the organoid fusion method and generated organoids enriched with inhibitory interneurons from Rett syndrome patient's iPSCs. In the disease-bearing organoids, they observed susceptibility to hyperexcitability, reductions in the microcircuit clusters, recurring epileptiform spikes, and altered frequency oscillations, which were traced back to dysfunctional inhibitory neurons (preprint: Samarasinghe *et al,* 2019). Furthermore, the model was used to test treatment options by treating the mutated organoids with valproic acid (VPA) or with the TP53 inhibitor, pifithrin-α (PFT), showing improved neuronal activity compared with the treatment with vehicle, with better results using PFT rather than VPA. Although pioneering, these studies focused on the electrophysiological changes seen in the disease-modeling organoids. Considering the lack of gross neurohistological changes in epileptic patients to direct the mechanistic research (Blumcke *et al,* 2017), our study sought to strengthen the utilization of brain organoids for the molecular study of epilepsy. This end was highlighted by bulk RNA-seq analysis, showing defective regional identity acquisition, cortical layer disruption, and Wnt signaling activation. The latter is of particular interest in light of the purposed role for Wnt signaling pathway as a regulator of seizure-induced brain consequences, and therefore a possible target for treatment (Yang *et al,* 2016; Qu *et al,* 2017; Hodges & Lugo, 2018). The forementioned cortical dyslamination is reminiscent of cortical dysplasia, which have a well-recognized role in the pathogenesis of drug-resistant epilepsy (Tassi *et al,* 2002; Fauser *et al,* 2006; Kobow *et al,* 2019).

In agreement with our findings, a recent study that examined the brain histology of a fetus suffering from the WOREE syndrome reported anomalous migration of the external granular layer within the molecular layer of the cortex, a phenotype that was validated also in a rat model with spontaneous WWOX mutations (Iacomino *et al,* 2020). This observation is further supported by the transcriptomic analysis performed by Kosla *et al* (2019) on human neuronal progenitor cells (hNPCs) after silencing WWOX using shRNA. This study found that knocking down WWOX causes hNPCs to lose the enrichment of genes related to neural crest differentiation and migration and to cell-cell adhesion, present in WT hNPCs. The authors also reported decreased mitochondrial redox potential, enhanced cellular adhesion to the growth surface, and reduced expression of MMP2 and MMP9. Iacomino *et al* (2020) reanalyzed this transcriptomic data, focusing on genes associated with neuronal migration and differentiation, and found reduced expression of some neural migration-related genes, such as microtubule proteins and kinesin family proteins. Notably, cortical layering was found to be affected by the status of the Wnt pathway (Qian *et al,* 2020), a pathway in which WWOX has been implicated through its binding partners. For example, WWOX was found to bind the Dishevelled proteins Dvl1 and Dvl2, with the latter being inhibited by WWOX, therefore attenuating the Wnt pathway (Bouteille *et al,* 2009; Abu-Odeh *et al,* 2014a). Our study further highlights a possible crosstalk between Wnt activation and DNA damage, a phenomenon that was previously described (Elyada *et al,* 2011). This is very much in line with the previously described pleiotropic functions of WWOX (Abu-Remaileh *et al,* 2015) and with the reduced negative regulation of cell cycle and MDM2 levels seen in our RNA-seq. We found accumulation of DNA breaks in Ki67⁺ cells in the VZ of KO COs, which might be explained by Wnt activation, promoting proliferation and likely replicative stress.

In addition to disease modeling in brain organoids, we attempted to rescue the phenotypes seen by reintroducing WWOX to the hESCs genome. This resulted in supraphysiological expression of WWOX in all cell populations seen in COs and a partial rescue. These results provide a proof of concept for successful reintroduction of WWOX as a mean for correcting the phenotype and possibly for therapeutic intervention. Yet, our findings suggest the importance of optimizing population-targeted delivery and fine-tuning of expression levels for successful genetic therapy approaches in patients with WOREE syndrome.

Lastly, we generated FOs from patients suffering from WOREE syndrome (WSM) and the relatively milder phenotype-SCAR12 (WPM). Our findings indicate that both brain organoid culture protocols (COs and FOs) result in similar outcomes, validating the phenotype of WWOX deficiency and that it stems from cortex. Intriguingly, when modeling the family of SCAR12, we did not observe the same developmental abnormalities as in WOREE organoids. SCAR12 FOs exhibited very mild, if any, differences in the forebrain neuronal population development, astrocyte development, and DDR signaling. This strengthens the system's ability to model the differences seen between the syndromes, and points out the need of closer examination of the rare SCAR12 syndrome and the pleiotropic functions of WWOX (Abu-Remaileh *et al,* 2015; Banne *et al,* 2021). It is noteworthy that although there is a marked difference in WWOX expression in the healthy heterozygote parents from different families, there is a very minor difference in the levels observed in the affected homozygote patients. These results raise the question whether the disease severity is correlated with the functional levels of WWOX rather than the total expression levels.

Overall, our data demonstrate the ability of brain organoids to model childhood epileptic encephalopathies, while elucidating the pathological changes seen in patients with germline mutations of *WWOX* and possible approaches for treatment development.

### Materials and Methods

### Cell culture and plasmids

WiBR3 hES cell line and the generated iPS cell lines were maintained in 5% CO₂ conditions on irradiated DR4 mouse embryonic fibroblast (MEF) feeder layers in FGF/KOSR conditions: DMEM/F12 (Gibco; 21331-020 or Biological Industries; 01-170-1A) supplemented with 15% knockout serum replacement (KOSR, Gibco; 10828-028), 1% GlutaMAX (Gibco; 35050-038), 1% MEM nonessential amino acids (NEAA, Biological Industries; 01-340-1B), 1% sodium pyruvate (Biological Industries; 03-042-1B), 1% penicillin-streptomycin (Biological Industries; 03-031-113), and 8 ng/ml bFGF (PeproTech; 100-18B). Medium was changed daily, and cultures were passaged every 5-7 days either manually or by trypsinization with trypsin type C (Biological Industries; 03-053-1B). Rho-associated kinase inhibitor (ROCKi, also known as Y27632) (Cayman; 10005583) was added for the first 24-48 h after passaging at a 10 µM concentration.

For transfection of hESCs, cells were cultured in 10 µM ROCKi 24h before electroporation. Cells were detached using trypsin C solution and resuspended in PBS (with Ca²⁺ and Mg²⁺) mixed with a total of 100 µg DNA constructs, and electroporated in Gene Pulser Xcell System (Bio-Rad; 250 V, 500 µF, 0.4-cm cuvettes). Cells were subsequently plated on MEF feeder layers in FGF/KOSR medium supplemented with ROCKi. For WWOX-KO, px330 plasmid containing the sgRNA targeting exon 1 was co-electroporated in 1:5 ratio with pNTK-GFP, and 48hr later, GFP-positive cells were sorted and subsequently plated sparsely (2,000 cells per 10-cm plate) on MEF feeder plates for colony isolation, ~10 days later. For WWOX reintroduction, pAAVS-2aNeo-UBp-IRES-GFP plasmid cloned to carry the WWOX coding sequence was co-electroporated with px330 targeting the AAVS1 locus (Guernet *et al,* 2016), sorted for GFP, and selected with 0.5 µg/ml puromycin for colony isolation. Gene editing was validated via Western blot. sgRNA sequences are noted in Table EV3.

For RNA or protein isolation, hPSCs were passaged onto Matrigel-coated plates (Corning; 356231) as indicated above and were cultured in NutriStem hPSC XF Medium (Biological Industries; 05-100-1A).

### Cerebral organoid generation, culture, and lentiviral infection

Cerebral organoids were generated from hESCs as previously described (Lancaster *et al,* 2013; Lancaster & Knoblich, 2014; Bagley *et al,* 2017; Lancaster *et al,* 2018), with the following changes:
Human WiBR3 cells and WSM iPSCs were maintained on mitotically inactivated MEFs. 4-7 days before protocol initiation, cells were passaged onto 60-mm plates coated with either MEFs or Matrigel (Corning; FAL356231) and grown until 70-80% confluency was reached. On day 0, hESC colonies were detached from MEFs with 0.7 mg/ml collagenase D solution (Sigma; 11088858001) and dissociated to single-cell suspension using a quick 2-min treatment with trypsin type C. For cells cultured on Matrigel, collagenase D treatment was skipped, and cells were immediately dissociated with trypsin type C, with no other variations in protocols from this point forward. Although only empirically observed, no major differences were seen in final outcome; however, MEF-cultured hPSCs seemed to have better success rates of neural induction and therefore were preferentially used.

After dissociation, cells were counted and suspended in hESC medium, composed of DMEM/F12-supplemented 20% KOSR, 3% USDA-certified hESC-quality FBS (Biological Industries), 1% GlutaMAX, 1% NEAA, 100 µM 2-mercaptoethanol (Sigma; M3148), 4 ng/ml bFGF, and 10 µM Rocki. For embryoid body (EB) formation 9,000 cells were seeded in each well of an ultra-low attachment V-bottom 96-well plates (S-Bio Prime; MS-9096VZ). EBs were fed every other day for another 5 days, in which fresh bFGF and ROCKi were added in the first change. At day 6, the medium was replaced with Neural Induction (NI) medium (Bagley *et al,* 2017), composed of DMEM/F12, 1% N2 supplement (Gibco; 17502048), 1% GlutaMAX, 1% MEM-NEAA, and 1 µg/ml heparin solution (Sigma; H3149). NI medium was changed every other day until establishment of neuroepithelium (usually on days 11-12), where quality control was performed as indicated (Lancaster & Knoblich, 2014; Bagley *et al,* 2017), and well-developed EBs were embedded in Matrigel droplets (Lancaster & Knoblich, 2014; Bagley *et al,* 2017). Droplets were transferred to 90-mm sterile, non-treated, culture dishes (Miniplast; 825-090-15-017) with Cerebral Differentiation Medium (CDM) composed of 1:1 mixture of DMEM/F12 and Neuro-basal Medium (Gibco; 21103049 or Biological Industries; 06-1055110-1A), 0.5% N2 supplement, 1% B27 supplement without vitamin A (Gibco; 12587010), 1% GlutaMax, 1% penicillin/streptomycin, 0.5% NEAA, 50 µM 2-mercaptoethanol, 2.5 µg/ml human recombinant Insulin (Biological Industries; 41-975-100), and 3 µM CHIR-99021 (Axon Medchem; 1386). The medium was changed every other day. From day 16 onward, organoids were cultured on an orbital shaker at 37°C and 5% CO₂ in Cerebral Maturation Medium (CMM) (Lancaster *et al,* 2018) composed similar to CDM, with B27 supplement changed to B27 supplement containing vitamin A (Gibco; 17504044), without CHIR-99021, and containing 400 µM vitamin C (Sigma; A4403) and 12.5 mM HEPES buffer (Biological Industries; 03-025-1B). Medium was changed every 2-4 days. From week 6, 1% Matrigel was added to the medium. To reduce chances of contamination, every 30 days the organoids were moved to fresh sterile plates. All of the described media were filtered through a 0.22-µm filter and stored at 4°C until usage. For all analyses, organoids from the same batch were used, unless stated otherwise.

Lentiviral transduction of WWOX was carried as previously published (Deverman *et al,* 2016; Khawaled *et al,* 2019). Briefly, viruses carrying WWOX were generated from pDEST12.2TM destination vector (Gateway Cloning Technology). After ultracentrifugation, titer was determined empirically by infecting 293T cells. At day 35 of culture, individual COs were transferred to an Eppendorf tube containing CMM with 1:100 of virus-containing medium and 5 µg/ml polybrene (Merck; TR-1003-6) and incubated overnight. The day after, organoids were put back on shaking culture with fresh medium.

### Reprogramming of somatic cells

Blood samples from families affected by WOREE and SCAR12 syndromes were donated under the approval of the Kaplan Medical Center Helsinki Committee for research purposes only, with informed consent obtained from all human subjects, and all the experiments conformed to the principles set out in the WMA Declaration of Helsinki and the Department of Health and Human Services Belmont Report.

Derivation of iPSCs directly from PBMCs was conducted by infection with the Yamanaka factors and Sendai virus CytoTune-iPS 2.0 Kit according to the manufacturer's instructions. Briefly, blood samples from PBMCs were isolated by Ficoll gradient and were cultured with StemPro-34^{™} medium (Gibco; 10639-011) supplemented with StemPro-34 Nutrient Supplement (Gibco; 10639-011), 100 ng/ml human SCF (PeproTech; 300-07), 100 ng/ml human FLT-3 ligand (R&D Systems; 308-FKE), 20 ng/ml human IL-3 (PeproTech; 200-03), and 10 ng/ml Human IL-6 (PeproTech; 200-06). After 24 h, half of the medium was replaced. After additional 24 h, day 0 of the protocol, cells were transferred to 6-well plates, reprogramming virus mixture was added, and the plates were centrifuged at 1,000×g for 30 min at room temperature. Cells were resuspended and placed back in the incubator overnight. The next day, to get rid of the remaining virus, the cells were centrifuged washed and resuspended in fully supplemented StemPro-34 medium, with extra medium addition on day 2. On day 3, cells were transferred to 10 cm MEF-coated plates, with half the medium replaced with complete StemPro-34 without cytokines and half medium changes every other day. By day 7, cells in different phases of reprogramming were seen, and the medium was gradually changed into mTeSR supplemented with 10 µM ROCKi to prevent reprogramming-related apoptosis. On day 16, colonies with normal morphology and growth rate were picked, expanded, validated for expression of pluripotency markers, and sequenced for WWOX mutations.

### Forebrain organoid generation and culture

Forebrain organoids were generated from iPSCs as previously described (Qian *et al,* 2016, 2018), with the changes noted below:
iPSC cells were maintained on mitotically inactivated MEFs. 4-7 days before protocol initiation, cells were passaged onto MEF-coated 60 mm plates and were cultured up to 70-80% confluency. On day 0, iPSC colonies were detached, dissociated, and counted the same as for COs, and resuspended in hPSC medium containing DMEM/F12, 20% KOSR, 1% GlutaMax, 1% MEM-NEAA, 1% penicillin/streptomycin, and 100 µM 2-mercaptoethanol. 9,000 cells per well were seeded in V-bottom 96-well plate. On day 1, medium was changed to Neuroectoderm Medium (NEM), which is hPSC medium freshly supplemented with 2 µM A83 (Axon Medchem; 1421) and 100 nM LDN-193189 (Axon Medchem; 1527), which was changed every other day. On days 5 and 6, half of the medium was aspirated and replaced by Neural Induction Medium (NIM) composed of DMEM/F12, 1% N2 supplement, 1% GlutaMax, 1% penicillin/streptomycin, 1% NEAA, 10 µg/ml heparin, 1 µM CHIR-99021 (Axon Medchem; 1386), and 1 µM SB-431542 (Sigma; S4317). On day 7, quality control and Matrigel embedding were performed as indicated (Qian *et al,* 2018), and EBs were continued to be cultured in NIM with medium changes every other day. At day 14, Matrigel removal was preformed (Qian *et al,* 2018), medium was changed to Forebrain Differentiation Medium (FDM) composed of DMEM/F12, 1% N2 supplement, 1% B27 with vitamin A, 1% NEAA, 1% GlutaMax, 1% penicillin/streptomycin, 50 µM 2-mercaptoethanol, and 2.5 µg/ml insulin, and transferred to an orbital shaker at 37°C and 5% CO₂. Medium was changed every 2-3 days. On day 71, the medium was changed to Forebrain Maturation Medium (FMM), containing Neurobasal medium, 1% B27 supplement with vitamin A, 1% GlutaMax, 1% penicillin/streptomycin, 50 µM 2-mercaptoethanol, 200 µM vitamin C, 20 ng/ml human recombinant BDNF (Pepro-Tech; 450-02), 20 ng/ml human recombinant GDNF (PeproTech; 450-10), 1 µM dibutyryl-cAMP (Sigma; D0627), and 1 ng/mL TGF-β1 (PeproTech; 100-21C). Medium was changed every 2-3 days.

### Immunofluorescence

Organoid fixation and immunostaining were performed as previously described (Mansour *et al,* 2018). Briefly, organoids were washed three times in PBS, then transferred for fixation in 4% ice-cold paraformaldehyde for 45 min, washed three times in cold PBS, and cryoprotected by overnight equilibration in 30% sucrose solution. The next day, organoids were embedded in OCT, snap-frozen on dry ice, and sectioned at 10 µm by Leica CM1950 cryostats.

For immunofluorescent staining, sections were warmed to room temperature and washed in PBS for rehydration, permeabilized in 0.1% Triton X-100 in PBS (PBT), and then blocked for 1 hr in a blocking buffer containing 5% normal goat serum (NGS) and 0.5% BSA in PBT. The sections were then incubated at 4°C overnight with primary antibodies diluted in the blocking solution. The day after, sections were then washed in three times while shaking in PBS containing 0.05% Tween-20 (PBST) and incubated with secondary antibodies and Hoechst 33258 solution diluted in blocking buffer for 1.5 h at RT. Slides were washed four times in PBST while shaking, and coverslips were mounted using Immunofluorescence Mounting Medium (Dako; s3023). Sections were imaged with Olympus FLUOVIEW FV1000 confocal laser scanning microscope and processed using the associated Olympus FLUOVIEW software. γH2AX-positive nuclei were manually counted using NIH ImageJ and statistically analyzed as later described.

### Electrophysiological recordings

Organoids were embedded in 3% low-temperature gelling agarose (at ~36°C) and incubated on ice for 5 min, after which they were sliced to 400 µm using a Leica 1200S Vibratome in sucrose solution (in mM: 87 NaCl, 25 NaHCO₃, 2.5 KCl, 25 glucose, 0.5 CaCl₂, 7 MgCl₂, 1.25 NaHPO₄, and 75 sucrose) at 4°C. Slices were incubated in artificial cerebrospinal fluid (ACSF, in mM: 125 NaCl, 25 NaHCO₃, 2.5 KCl, 10 glucose, 2.5 CaCl₂, 1.5 MgCl₂, pH 7.38, and 300 mOsm) for 30 min at 37°C, followed by 1 h at RT. During recordings, slices were incubated in the same ACSF at 37°C with perfused carbogen (95% O₂, 5% CO₂), in baseline condition. Local field potential (LFP) and whole-cell patch-clamp recordings were done using electrodes pulled from borosilicate capillary glass and positioned 150-µm deep from the outer rim of each slice. LFP electrodes were filled with ACSF, while patch electrodes were filled with internal solution. Data were recorded using MultiClamp software at a sampling rate of 25,000 Hz. Data were analyzed using MATLAB software. Traces were filtered using (i) 60 notch filter (with 5 harmonics) to eliminate noise and (ii) 0.1-Hz high-pass IIR filter to eliminate fluctuations from the recording setup. The detrended feature (using the hamming window) was then used to eliminate large variations in the signal, and the normalized spectral power was calculated using the fast Fourier transform. The area under the curve of the power spectral density plots was calculated by taking the sum of binned frequencies over specific frequency ranges.

### Cell-attached recordings

Cell-attached recordings were obtained with blind patch-clamp recordings. We recorded spontaneous neuronal activity from organoids' neuronal populations. Electrodes (~7 MOhm) were pulled from filamented, thin-walled, borosilicate glass (outer diameter, 1.5 mm; inner diameter, 0.86 mm; Hilgenberg GmbH) on a vertical two-stage puller (PC-12, Narishige). The electrodes were filled with an internal solution that contained the following (in mM): 140 K-gluconate, 10 KCl, 10 HEPES, 10 Na₂-phosphocreatine, and 0.5 EGTA, and adjusted to pH 7.25 with KOH.

The electrodes were inserted at 45° to the organoid's surface. During the recordings, the organoids were kept in CMM without Matrigel at 35°C. An increase in the pipette resistance to 10-200 MOhm resulted in most cases in the appearance of spikes. The detection of a single spike was the criteria to start the recording. All recordings were acquired with an intracellular amplifier in current-clamp mode (MultiClamp 700B, Molecular Devices), acquired at a sampling rate of 10 kHz (CED Micro 1401-3, Cambridge Electronic Design Limited), and filtered with a high-pass filter to eliminate field potentials and retain neuronal spikes.

Data analysis of the cell-attached recordings was carried out with custom-written code in MATLAB (The MathWorks). Spikes recorded in the cell-attached mode were extracted from raw voltage traces by applying a threshold (the spikes' threshold was placed well above the peaks in the background noise level). For calculating the average firing rate, the firing rate over a 4-min recording period was calculated for each recorded cell.

### Immunoblot analysis and subcellular fractionation

For total protein, organoids were homogenized in lysis buffer containing 50 mM Tris (pH 7.5), 150 mM NaCl, 10% glycerol, and 0.5% Nonidet P-40 (NP-40) that was supplemented with protease and phosphatase inhibitors. For separation of cytoplasmic fraction, organoids were grinded in a hypotonic lysis buffer [10 mmol/l HEPES (pH 7.9), 10 mmol/l KCl, 0.1 mmol/l EDTA] supplemented with 1 mmol/l DTT and protease and phosphatase inhibitors. The cells were allowed to swell on ice for 15 min, then 0.5% NP-40 was added, and cells were lysed by vortex. After centrifugation, the cytoplasmic fraction was collected. Afterwards, nuclear fraction was obtained by incubating remaining pellet in a hypertonic nuclear extraction buffer [20 mmol/l HEPES (pH 7.9), 0.42 mol/l KCl, 1 mmol/l EDTA] supplemented with 1 mmol/l DTT for 15 min at 4°C while shaking. The samples were centrifuged, and liquid phase was collected.

Western blotting was performed under standard conditions, with 40-50 µg protein used for each sample. Blots were repeated and quantified 2-3 times per experiment in Bio-Rad's Image Lab software.

### RNA extraction, reverse transcription-PCR, and qPCR

Total RNA was isolated using Bio-Tri reagent (Biolab; 9010233100) as described by the manufacturer for phenol/chloroform-based method. 0.5-1 µg of RNA was used to synthesize cDNA using a qScript cDNA Synthesis Kit (QuantaBio; 95047). qRT-PCR was performed using Power SYBR Green PCR Master Mix (Applied Biosystems; AB4367659). All measurements were performed in triplicate and were standardized to the levels of either HPRT or UBC.

### Library preparation and RNA sequencing

Library preparation and RNA sequencing was performed by the Genomic Applications Laboratory in the Hebrew University's Core Research Facility following the standard procedures. Briefly, RNA quality was assessed by using RNA ScreenTape Kit (Agilent Technologies; 5067-5576), D1000 ScreenTape Kit (Agilent Technologies; 5067-5582), Qubit(r) RNA HS Assay Kit (Invitrogen; Q32852), and Qubit(r) DNA HS Assay Kit (Invitrogen; 32854).

For mRNA library preparation, 1 µg of RNA per sample was processed using KAPA Stranded mRNA-Seq Kit with mRNA Capture Beads (Kapa Biosystems; KK8421). Library was eluted in 20 µl of elution buffer and adjusted to 10 mM, and then, 10 µl (50%) from each sample was collected and pooled in one tube. Multiplex sample pool (1.5pM including PhiX 1.5%) was loaded in NextSeq 500/550 High Output v2 Kit (75 cycles) cartridge (Illumina; FC-404-1005) and loaded on NextSeq 500 System Machine (Illumina), with 75 cycles and single-read sequencing conditions.

For library quality control, Fastq files were tested with *FastQC* (ver.0.11.8) and trimmed for residual adapters, low-quality bases (Q = 20), and read length (20 bases). Trimming was performed with *trim galore* (ver.0.6.1). Read counts were high around 30-50 M per sample and decreased negligibly after filtering. Transcriptome mapping was performed with *salmon* (ver.1.2.1) in its mapping-based mode, turning on both validate mapping mode and gc-bias correction. Prior to alignment, a salmon index was created based on HS GRCh38 CDNA release 99 (Nov 2019) using kmer size of 25. Salmon mapping reports both raw transcripts count and TPM counts. Resulting mapping rates are high between 80% and 90%. A total of 8 CO samples were sequenced (4 WT COs and 4 KO COs)-one WT sample failed our preliminary quality control (low read count and low transcriptome mapping rate). Another WT sample that did not cluster with any of the other samples (neither WWOX-KO nor WT) was apparent in both PCA and dendrogram analysis. These two samples were extracted from further analysis, giving a total of six samples used for further analysis. For differentially expressed gene determination (KO versus WT), raw transcript counts were filtered for minimal overall count of 10 on all six samples and imported with R package *tximport* (ver.1.16.1) for analysis with *DEeq2* (ver.1.28.1). Counts were normalized by DESeq2, and differentially expressed genes were filtered, setting alpha to 0.01. Mean-based fold change was calculated, as well as a shrink-based fold change, based on *apeglm* (ver.1.10.0).

For the preparation of the heatmaps shown in Fig. 16A and C, the list of differentially expressed genes was separated to upregulated (WWOX-KO expression was higher than WT expression) and downregulated sublists. Each sublist was sorted by fold change values, and top 100 genes were selected from each sublist. For each of the selected genes, log2-normalized counts were scaled and presented in a heatmap using heatmap.2 from R package *gplots* (ver.3.0.3).

For the heatmap seen in Fig. 14F, log2-normalized counts for each of the six cortical layer gene markers were scaled and presented in a heatmap form using heatmap.2 from R package *gplots.* Gene set enrichment analysis was performed with Broad Institute *GSEA* software (ver.4.0.3). Input included 15,348 genes ranked by log2 of fold change. GO sets are Broad Institute set c5.all.v7.0. Permissible sets are those with at least 15 genes and no more than 500 genes. Gene sets are GO biological processes. Permissible sets in this analysis are those with at least 10 genes and no more than 500 genes. PCA plot of first two components was calculated and plotted with base R functions. Calculation is based on log2-transformed and log2-normalized counts adding pseudo count of 1.

### Statistics

Results of the experiments were expressed either as mean ± SEM or in a boxplot indicating the 1^{st} and 3^{rd} quartiles, minimum and maximum values, and the median. First, the Wilk-Shapiro test was used to determine normality: For normally distributed samples, a two-tailed unpaired Student's t-test with Welch's correction was used to compare the values of the test and control samples. For non-normally distributed samples, the non-parametric Mann-Whitney test was used. For comparisons between more than two samples, one-way ANOVA was used, correcting for the multiple comparisons with Tukey's multiple comparisons test. For samples that were not normally distributed, the Kruskal-Wallis test was used with Dunn's multiple comparisons test. For the kinetic experiments, the analysis was corrected for multiple t-tests using the Holm- Šídák method, without assuming equal SD. P-value cutoff for statistically significant results was as follows: n.s (non-significant), **P* ≤ 0.05, ***P* ≤ 0.01, ****P* ≤ 0.001, and *****P* ≤ 0.0001. Statistical analysis and visual data presentation were preformed using GraphPad Prism 8. No randomization or blinding was applied in this study. The experiments were performed on several biological replicates, with at least two hPSC lines used for each genotype (with the exception of the WiBR3 WT line). Unless stated otherwise, the experiments were performed on multiple batches of organoids.

### References

Aaberg KM, Gunnes N, Bakken IJ, Lund Søraas C, Berntsen A, Magnus P, Lossius MI, Stoltenberg C, Chin R, Surén P (2017) Incidence and prevalence of childhood epilepsy: a nationwide cohort study. Pediatrics 139: 1-9

Abdeen SK, Ben-David U, Shweiki A, Maly B, Aqeilan RI (2018) Somatic loss of WWOX is associated with TP53 perturbation in basal-like breast cancer. Cell Death Dis 9 https://doi.org/10.1038/s41419-018-0896-z

Abdel-Salam G, Thoenes M, Afifi HH, Körber F, Swan D, Bolz H (2014) The supposed tumor suppressor gene WWOX is mutated in an early lethal microcephaly syndrome with epilepsy, growth retardation and retinal degeneration. Orphanet J Rare Dis 9: 12-18

Abu-Odeh M, Bar-Mag T, Huang H, Kim T, Salah Z, Abdeen SK, Sudol M, Reichmann D, Sidhu S, Kim PM et al (2014a) Characterizing WW domain interactions of tumor suppressor WWOX reveals its association with multiprotein networks. J Biol Chem 289: 8865-8880

Abu-odeh M, Hereema NA, Aqeilan RI (2016) WWOX modulates the ATR-mediated DNA damage checkpoint response. Oncotarget 7: 4344-4355

Abu-Odeh M, Salah Z, Herbel C, Hofmann TG, Aqeilan RI (2014b) WWOX, the common fragile site FRA16D gene product, regulates ATM activation and the DNA damage response. Proc Natl Acad Sci USA 111: E4716-E4725

Abu-Remaileh M, Aqeilan RI (2014) Tumor suppressor WWOX regulates glucose metabolism via HIF1α modulation. Cell Death Differ 21: 1805-1814

Abu-Remaileh M, Aqeilan RI (2015) The tumor suppressor WW domain-containing oxidoreductase modulates cell metabolism. Exp Biol Med 240: 345-350

Abu-Remaileh M, Joy-Dodson E, Schueler-Furman O, Aqeilan RI (2015) Pleiotropic functions of tumor suppressor WWOX in normal and cancer cells. J Biol Chem 290: 30728-30735

Abu-Remaileh M, Khalaileh A, Pikarsky E, Aqeilan RI (2018) WWOX controls hepatic HIF1α to suppress hepatocyte proliferation and neoplasia article. Cell Death Dis 9 https://doi.org/10.1038/s41419-018-0510-4

Ahnaou A, Huysmans H, Van De Casteele T, Drinkenburg WHIM (2017) Cortical high gamma network oscillations and connectivity: a translational index for antipsychotics to normalize aberrant neurophysiological activity. Transl Psychiatry 7: 1285

Amin ND, Pa 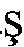 ca SP (2018) Building models of brain disorders with three-dimensional organoids. *Neuron* 100: 389-405

Aqeilan RI, Abu-Remaileh M, Abu-Odeh M (2014) The common fragile site FRA16D gene product WWOX: roles in tumor suppression and genomic stability. Cell Mol Life Sci 71: 4589-4599

Aqeilan RI, Hassan MQ, de Bruin A, Hagan JP, Volinia S, Palumbo T, Hussain S, Lee S-H, Gaur T, Stein GS et al (2008) The WWOX tumor suppressor is essential for postnatal survival and normal bone metabolism. J Biol Chem 283: 21629-21639

Aqeilan RI, Trapasso F, Hussain S, Costinean S, Marshall D, Pekarsky Y, Hagan JP, Zanesi N, Kaou M, Stein GS et al (2007) Targeted deletion of Wwox reveals a tumor suppressor function. Proc Natl Acad Sci USA 104: 3949-3954

Bagley JA, Reumann D, Bian S, Lévi-Strauss J, Knoblich JA (2017) Fused cerebral organoids model interactions between brain regions. Nat Methods 14: 743-751

Banne E, Abudiab B, Abu-Swai S, Repudi SR, Steinberg DJ, Shatleh D, Alshammery S, Lisowski L, Gold W, Carlen PL et al (2021) Neurological disorders associated with WWOX germline mutations-a comprehensive overview. Cells 10: 824

Ben-Ari Y, Gaiarsa J-L, Tyzio R, Khazipov R (2007) GABA: a pioneer transmitter that excites immature neurons and generates primitive oscillations. Physiol Rev 87: 1215-1284

Ben-Salem S, Al-Shamsi AM, John A, Ali BR, Al-Gazali L (2015) A novel whole exon deletion in WWOX gene causes early epilepsy, intellectual disability and optic atrophy. J Mol Neurosci 56: 17-23

Blair JD, Hockemeyer D, Bateup HS (2018) Genetically engineered human cortical spheroid models of tuberous sclerosis. Nat Med 24: 1568-1578

Blumcke I, Spreafico R, Haaker G, Coras R, Kobow K, Bien CG, Pfäfflin M, Elger C, Widman G, Schramm J et al (2017) Histopathological findings in brain tissue obtained during epilepsy surgery. N Engl J Med 377: 1648-1656

Bouteille N, Driouch K, El HP, Sin S, Formstecher E, Camonis J, Lidereau R, Lallemand F (2009) Inhibition of the Wnt/β-catenin pathway by the WWOX tumor suppressor protein. Oncogene 28: 2569-2580

Bragin A, Engel J (2008) Slow waves associated with seizure activity. In Computational neuroscience in epilepsy, Soltesz I, Staley K (eds), pp 440-453. San Diego: Elsevier Chen S, Chuang J, Wang J, Tsai M, Li H, Chang N (2004) Expression of WW domain-containing oxidoreductase WOX1 in the developing murine nervous system. Neuroscience 124: 831-839

Cheng Y-Y, Chou Y-T, Lai F-J, Jan M-S, Chang T-H, Jou I-M, Chen P-S, Lo J-Y, Huang S-S, Chang N-S et al (2020) Wwox deficiency leads to neurodevelopmental and degenerative neuropathies and glycogen synthase kinase 3β-mediated epileptic seizure activity in mice. Acta Neuropathol Commun 8: 6

Cohen-Gadol AA, Pan JW, Kim JH, Spencer DD, Hetherington HH (2004) Mesial temporal lobe epilepsy: a proton magnetic resonance spectroscopy study and a histopathological analysis. J Neurosurg 101: 613-620

Deverman BE, Pravdo PL, Simpson BP, Kumar SR, Chan KY, Banerjee A, Wu W-L, Yang B, Huber N, Pasca SP et al (2016) Cre-dependent selection yields AAV variants for widespread gene transfer to the adult brain. Nat Biotechnol 34: 204-209

Elyada E, Pribluda A, Goldstein RE, Morgenstern Y, Brachya G, Cojocaru G, Snir-Alkalay I, Burstain I, Haffner-Krausz R, Jung S et al (2011) CKIα ablation highlights a critical role for p53 in invasiveness control. Nature 470: 409-413

Fauser S, Huppertz HJ, Bast T, Strobl K, Pantazis G, Altenmueller DM, Feil B, Rona S, Kurth C, Rating D et al (2006) Clinical characteristics in focal cortical dysplasia: a retrospective evaluation in a series of 120 patients. Brain 129: 1907-1916

Fisher RS, Acevedo C, Arzimanoglou A, Bogacz A, Cross JH, Elger CE, Engel J, Forsgren L, French JA, Glynn M et al (2014) ILAE official report: a practical clinical definition of epilepsy. Epilepsia 55: 475-482

Gribaa M, Salih M, Anheim M, Lagier-Tourenne C, H'mida D, Drouot N, Mohamed A, Elmalik S, Kabiraj M, Al-Rayess M et al (2007) A new form of childhood onset, autosomal recessive spinocerebellar ataxia and epilepsy is localized at 16q21-q23. Brain 130: 1921-1928

Guernet A, Mungamuri S, Cartier D, Sachidanandam R, Jayaprakash A, Adriouch S, Vezain M, Charbonnier F, Rohkin G, Coutant S et al (2016) CRISPR-barcoding for intratumor genetic heterogeneity modeling and functional analysis of oncogenic driver mutations. Mol Cell 63: 526-538

Guirgis M, Chinvarun Y, Carlen PL, Bardakjian BL (2013) The role of delta-modulated high frequency oscillations in seizure state classification. In Proceedings of the Annual International Conference of the IEEE Engineering in Medicine and Biology Society, EMBS pp 6595-6598

Hazan I, Hofmann TG, Aqeilan RI (2016) Tumor suppressor genes within common fragile sites are active players in the DNA damage response. PLoS Genet 12: 1-19

Hodges SL, Lugo JN (2018) Wnt/β-catenin signaling as a potential target for novel epilepsy therapies. Epilepsy Res 146: 9-16

Howell KB, Freeman JL, Mackay MT, Fahey MC, Archer J, Berkovic SF, Chan E, Dabscheck G, Eggers S, Hayman M et al (2021) The severe epilepsy syndromes of infancy: a population-based study. Epilepsia 62: 358-370

Hussain T, Kil H, Hattiangady B, Lee J, Kodali M, Shuai B, Attaluri S, Takata Y, Shen J, Abba MC et al (2019) Wwox deletion leads to reduced GABA-ergic inhibitory interneuron numbers and activation of microglia and astrocytes in mouse hippocampus. Neurobiol Dis 121: 163-176

Iacomino M, Baldassari S, Tochigi Y, Kośla K, Buffelli F, Torella A, Severino M, Paladini D, Mandarà L, Riva A *et al* (2020) Loss of Wwox perturbs neuronal migration and impairs early cortical development. *Front Neurosci* 14 https://doi.org/10.3389/fnins.2020.00644

Khalilov I, Le Van QM, Gozlan H, Ben-Ari Y (2005) Epileptogenic actions of GABA and fast oscillations in the developing hippocampus. Neuron 48: 787-796

Khawaled S, Nigita G, Distefano R, Oster S, Suh S-S, Smith Y, Khalaileh A, Peng Y, Croce CM, Geiger T et al (2020) Pleiotropic tumor suppressor functions of WWOX antagonize metastasis. Signal Transduct Target Ther 5: 1-10

Khawaled S, Suh SS, Abdeen SK, Monin J, Distefano R, Nigita G, Croce CM, Aqeilan RI (2019) WWOX inhibits metastasis of triple-negative breast cancer cells via modulation of miRNAs. Cancer Res 79: 1784-1798

Kheiri F, Bragin A, Engel J (2013) Functional connectivity between brain areas estimated by analysis of gamma waves. J Neurosci Methods 214: 184-191

Kobow K, Ziemann M, Kaipananickal H, Khurana I, Mühlebner A, Feucht M, Hainfellner JA, Czech T, Aronica E, Pieper T et al (2019) Genomic DNA methylation distinguishes subtypes of human focal cortical dysplasia. Epilepsia 60: 1091-1103

Kośla K, P 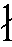 uciennik E, Styczeń-Binkowska E, Nowakowska M, Orzechowska M, Bednarek AK (2019) The WWOX gene influences cellular pathways in the neuronal differentiation of human neural progenitor cells. *Front Cell Neurosci* 13: 391

Lado FA, Rubboli G, Capovilla P, Avanzini G, Moshé SL (2013) Pathophysiology of epileptic encephalopathies. Epilepsia 54: 6-13

Lancaster MA, Corsini NS, Wolfinger S, Gustafson EH, Phillips AW, Burkard TR, Otani T, Livesey FJ, Knoblich JA (2018) Guided self-organization and cortical plate formation in human brain organoids. Nat Biotechnol 35: 659-666

Lancaster MA, Knoblich JA (2014) Generation of cerebral organoids from human pluripotent stem cells. Nat Protoc 9: 2329-2340

Lancaster MA, Renner M, Martin C, Wenzel D, Bicknell LS, Hurles ME, Homfray T, Penninger JM, Jackson AP, Knoblich JA (2013) Cerebral organoids model human brain development and microcephaly. Nature 501: 373-379

Madabhushi R, Gao F, Pfenning A, Pan L, Yamakawa S, Seo J, Rueda R, Phan TX, Yamakawa H, Pao P-C et al (2015) Activity-induced DNA breaks govern the expression of neuronal early-response genes. Cell 161: 1592-1605

Madabhushi R, Pan L, Tsai L-H (2014) DNA damage and its links to neurodegeneration. Neuron 83: 266-282

Mallaret M, Synofzik M, Lee J, Sagum CA, Mahajnah M, Sharkia R, Drouot N, Renaud M, Klein FAC, Anheim M et al (2014) The tumour suppressor gene WWOX is mutated in autosomal recessive cerebellar ataxia with epilepsy and mental retardation. Brain 137: 411-419

Mansour AA, Gonçalves JT, Bloyd CW, Li H, Fernandes S, Quang D, Johnston S, Parylak SL, Jin X, Gage FH (2018) An in vivo model of functional and vascularized human brain organoids. Nat Biotechnol 36: 432-441

McTague A, Howell KB, Cross JH, Kurian MA, Scheffer IE (2016) The genetic landscape of the epileptic encephalopathies of infancy and childhood. Lancet Neurol 15: 304-316

Medvedev AV, Murro AM, Meador KJ (2011) Abnormal interictal gamma activity may manifest a seizure onset zone in temporal lobe epilepsy. Int J Neural Syst 21: 103-114

Middeldorp J, Boer K, Sluijs JA, De FL, Encha-razavi F, Vescovi AL, Swaab DF, Aronica E, Hol EM (2010) GFAPdelta in radial glia and subventricular zone progenitors in the developing human cortex. Development 321: 313-321

Mignot C, Lambert L, Pasquier L, Bienvenu T, Delahaye-Duriez A, Keren B, Lefranc J, Saunier A, Allou L, Roth V et al (2015) WWOX-related encephalopathies: delineation of the phenotypical spectrum and emerging genotype-phenotype correlation. J Med Genet 52: 61-70

Miller JW, Kim W, Holmes MD, Vanhatalo S (2007) Ictal localization by source analysis of infraslow activity in DC-coupled scalp EEG recordings. NeuroImage 35: 583-597

Murata Y, Colonnese MT (2020) GABAergic interneurons excite neonatal hippocampus in vivo. Sci Adv 6: eaba1430

Nashabat M, Al Qahtani XS, Almakdob S, Altwaijri W, Ba-Armah DM, Hundallah K, Al Hashem A, Al Tala S, Maddirevula S, Alkuraya FS et al (2019) The landscape of early infantile epileptic encephalopathy in a consanguineous population. Seizure 69: 154-172

Obata K, Oide M, Tanaka H (1978) Excitatory and inhibitory actions of GABA and glycine on embryonic chick spinal neurons in culture. Brain Res 144: 179-184

Patel DC, Tewari BP, Chaunsali L, Sontheimer H (2019) Neuron-glia interactions in the pathophysiology of epilepsy. Nat Rev Neurosci 20: 282-297

Patrizi S, Holmes GL, Orzalesi M, Allemand F (2003) Neonatal seizures: characteristics of EEG ictal activity in preterm and full-term infants. Brain Dev 25: 427-437

Piard J, Hawkes L, Milh M, Villard L, Borgatti R, Romaniello R, Fradin M, Capri Y, Héron D, Nougues M-C et al (2018) The phenotypic spectrum of WWOX-related disorders: 20 additional cases of WOREE syndrome and review of the literature. Genet Med 21: 1308-1318

Pollen A, Nowakowski T, Chen J, Retallack H, Sandoval-Espinosa C, Nicholas C, Shuga J, Liu S, Oldham M, Diaz A et al (2015) Molecular identity of human outer radial glia during cortical development. Cell 163: 55-67

Qian X, Jacob F, Song MM, Nguyen HN, Song H, Ming GL (2018) Generation of human brain region-specific organoids using a miniaturized spinning bioreactor. Nat Protoc 13: 565-580

Qian X, Nguyen H, Song M, Hadiono C, Ogden S, Hammack C, Yao B, Hamersky G, Jacob F, Zhong C et al (2016) Brain-region-specific organoids using mini-bioreactors for modeling ZIKV exposure. Cell 165: 1238-1254

Qian X, Su Y, Adam CD, Deutschmann AU, Pather SR, Goldberg EM, Su K, Li S, Lu L, Jacob F et al (2020) Sliced human cortical organoids for modeling distinct cortical layer formation. Cell Stem Cell 26: 766-781.e9

Qu Z, Su F, Qi X, Sun J, Wang H, Qiao Z, Zhao H, Zhu Y (2017) Wnt/β-catenin signalling pathway mediated aberrant hippocampal neurogenesis in kainic acid-induced epilepsy. Cell Biochem Funct 35: 472-476

Repudi S, Steinberg DJ, Elazar N, Breton VL, Aquilino MS, Saleem A, Abu-Swai S, Vainshtein A, Eshed-Eisenbach Y, Vijayaragavan B et al (2021) Neuronal deletion of Wwox, associated with WOREE syndrome, causes epilepsy and myelin defects. Brain awab174

Robel S, Buckingham SC, Boni JL, Campbell SL, Danbolt NC, Riedemann T, Sutor B, Sontheimer H (2015) Reactive astrogliosis causes the development of spontaneous seizuresreactive astrogliosis causes the development of spontaneous seizures. J Neurosci 35: 3330-3345

Rossini L, Garbelli R, Gnatkovsky V, Didato G, Villani F, Spreafico R, Deleo F, Lo Russo G, Tringali G, Gozzo F et al (2017) Seizure activity per se does not induce tissue damage markers in human neocortical focal epilepsy. Ann Neurol 82: 331-341

Samarasinghe RA, Miranda OA, Mitchell S, Ferando I, Watanabe M, Buth JE, Kurdian A, Golshani P, Plath K, Lowry WE et al (2019) Identification of neural oscillations and epileptiform changes in human brain organoids. bioRxiv https://doi.org/10.1101/820183 [PREPRINT]

Schneider L, Pellegatta S, Favaro R, Pisati F, Roncaglia P, Testa G, Nicolis SK, Finocchiaro G, D'Adda Di Fagagna F (2013) DNA damage in mammalian neural stem cells leads to astrocytic differentiation mediated by BMP2 signaling through JAK-STAT. Stem Cell Rep 1: 123-138

Shanbhag NM, Evans MD, Mao W, Nana AL, Seeley WW, Adame A, Rissman RA, Masliah E, Mucke L (2019) Early neuronal accumulation of DNA double strand breaks in Alzheimer's disease. Acta Neuropathol Commun 7: 1 - 18

Shao L, Stafstrom CE (2016) Pediatric epileptic encephalopathies: pathophysiology and animal models. Semin Pediatr Neurol 23: 98-107

Sidhaye J, Knoblich JA (2020) Brain organoids: an ensemble of bioassays to investigate human neurodevelopment and disease. Cell Death Differ 28: 52-67

Suberbielle E, Sanchez PE, Kravitz AV, Wang X, Ho K, Eilertson K, Devidze N, Kreitzer AC, Mucke L (2013) Physiologic brain activity causes DNA double-strand breaks in neurons, with exacerbation by amyloid-β. Nat Neurosci 16: 613-621

Sun AX, Yuan Q, Fukuda M, Yu W, Yan H, Lim GGY, Nai MH, D' Agostino GA, Tran H-D, Itahana Y et al (2019) Potassium channel dysfunction in human neuronal models of Angelman syndrome. Science 366: 1486-1492

Suzuki H, Katayama K, Takenaka M, Amakasu K, Saito K, Suzuki K (2009) A spontaneous mutation of the Wwox gene and audiogenic seizures in rats with lethal dwarfism and epilepsy. Genes, Brain Behav 8: 650-660

Tanna M, Aqeilan RI (2018) Modeling WWOX loss of function in vivo: what have we learned? Front Oncol 8 https://doi.org/10.3389/fonc.2018.00420

Tassi L, Colombo N, Garbelli R, Francione S, Lo Russo G, Mai R, Cardinale F, Cossu M, Ferrario A, Galli C et al (2002) Focal cortical dysplasia: neuropathological subtypes, EEG, neuroimaging and surgical outcome. Brain 125: 1719-1732

Thom M (2009) Hippocampal sclerosis: progress since sommer. Brain Pathol 19: 565-572

Tochigi Y, Takamatsu Y, Nakane J, Nakai R, Katayama K, Suzuki H (2019) Loss of Wwox causes defective development of cerebral cortex with hypomyelination in a rat model of lethal dwarfism with epilepsy. Int J Mol Sci 20: 3596

Trujillo CA, Gao R, Negraes PD, Gu J, Buchanan J, Preissl S, Wang A, Wu W, Haddad GG, Chaim IA et al (2019) Complex oscillatory waves emerging from cortical organoids model early human brain network development. Cell Stem Cell 25: 558-569.e7

Vanhatalo S, Palva JM, Holmes MD, Miller JW, Voipio J, Kaila K (2004) Infraslow oscillations modulate excitability and interictal epileptic activity in the human cortex during sleep. Proc Natl Acad Sci USA 101: 5053-5057

Vezzani A, French J, Bartfai T, Baram TZ (2011) The role of inflammation in epilepsy. Nat Rev Neurol 7: 31-40

Wang H-Y, Juo L-I, Lin Y-T, Hsiao M, Lin J-T, Tsai C-H, Tzeng Y-H, Chuang Y-C, Chang N-S, Yang C-N et al (2012) WW domain-containing oxidoreductase promotes neuronal differentiation via negative regulation of glycogen synthase kinase 3B. Cell Death Differ 19: 1049-1059

Wang W, Esbensen Y, Kunke D, Suganthan R, Rachek L, Bjørås M, Eide L (2011) Mitochondrial DNA damage level determines neural stem cell differentiation fate. J Neurosci 31: 9746-9751

Weisz-Hubshman M, Meirson H, Michaelson-Cohen R, Beeri R, Tzur S, Bormans C, Modai S, Shomron N, Shilon Y, Banne E et al (2019) Novel WWOX deleterious variants cause early infantile epileptic encephalopathy, severe developmental delay and dysmorphism among Yemenite Jews. Eur J Paediatr Neurol 23: 418-426

Yang J, Zhang X, Wu Y, Zhao Bo, Liu X, Pan Y, Liu Y, Ding Y, Qiu M, Wang Y-Z et al (2016) Wnt/β-catenin signaling mediates the seizure-facilitating effect of post ischemic reactive astrocytes after pentylenetetrazole-kindling. Glia 64: 1083-1091

Zhang Y, Sloan S, Clarke L, Caneda C, Plaza C, Blumenthal P, Vogel H, Steinberg G, Edwards M, Li G et al (2016) Purification and characterization of progenitor and mature human astrocytes reveals transcriptional and functional differences with mouse. Neuron 89: 37-53

### SEQUENCES

SEQ ID No 1: Human WWOX cDNA (coding) sequence (NCBI Reference Sequence NM_016373.4) 
atggc agcgctgcgc tacgcggggc tggacgacac ggacagtgag gacgagctgc
ctccgggctg ggaggagaga accaccaagg acggctgggt ttactacgcc aatcacaccg
aggagaagac tcagtgggaa catccaaaaa ctggaaaaag aaaacgagtg gcaggagatt
tgccatacgg atgggaacaa gaaactgatg agaacggaca agtgtttttt gttgaccata
taaataaaag aaccacctac ttggacccaa gactggcgtt tactgtggat gataatccga
ccaagccaac cacccggcaa agatacgacg gcagcaccac tgccatggaa attctccagg
gccgggattt cactggcaaa gtggttgtgg tcactggagc taattcagga atagggttcg
aaaccgccaa gtcttttgcc ctccatggtg cacatgtgat cttggcctgc aggaacatgg
caagggcgag tgaagcagtg tcacgcattt tagaagaatg gcataaagcc aaggtagaag
caatgaccct ggacctcgct ctgctccgta gcgtgcagca ttttgctgaa gcattcaagg
ccaagaatgt gcctcttcat gtgcttgtgt gcaacgcagc aacttttgct ctaccctgga
gtctcaccaa agatggcctg gagaccacct ttcaagtgaa tcatctgggg cacttctacc
ttgtccagct cctccaggat gttttgtgcc gctcagctcc tgcccgtgtc attgtggtct
cctcagagtc ccatcgattt acagatatta acgactcctt gggaaaactg gacttcagtc
gcctctctcc aacaaaaaac gactattggg cgatgctggc ttataacagg tccaagctct
gcaacatcct cttctccaac gagctgcacc gtcgcctctc cccacgcggg gtcacgtcga
acgcagtgca tcctggaaat atgatgtact ccaacattca tcgcagctgg tgggtgtaca
cactgctgtt taccttggcg aggcctttca ccaagtccat gcaacaggga gctgccacca
ccgtgtactg tgctgctgtc ccagaactgg agggtctggg agggatgtac ttcaacaact
gctgccgctg catgccctca ccagaagctc agagcgaaga gacggcccgg accctgtggg
cgctcagcga gaggctgatc caagaacggc ttggcagcca gtccggctaa
SEQ ID No 2: Human WWOX amino acid sequence
SEQ ID No. 3: Human WWOX cDNA (full) 
GCAGTGCGCAGGCGTGAGCGGTCGGGCCCCGACGCGCGCGGGTCTCGTTTGGAGCGGGAGTGAG
TTCCTGAGCGAGTGGACCCGGCAGCGGGCGATAGGGGGGCCAGGTGCCTCCACAGTCAGCCATG
GCAGCGCTGCGCTACGCGGGGCTGGACGACACGGACAGTGAGGACGAGCTGCCTCCGGGCTGGG
AGGAGAGAACCACCAAGGACGGCTGGGTTTACTACGCCAATCACACCGAGGAGAAGACTCAGTG
GGAACATCCAAAAACTGGAAAAAGAAAACGAGTGGCAGGAGATTTGCCATACGGATGGGAACAA
GAAACTGATGAGAACGGACAAGTGTTTTTTGTTGACCATATAAATAAAAGAACCACCTACTTGG
ACCCAAGACTGGCGTTTACTGTGGATGATAATCCGACCAAGCCAACCACCCGGCAAAGATACGA
CGGCAGCACCACTGCCATGGAAATTCTCCAGGGCCGGGATTTCACTGGCAAAGTGGTTGTGGTC
ACTGGAGCTAATTCAGGAATAGGGTTCGAAACCGCCAAGTCTTTTGCCCTCCATGGTGCACATG
TGATCTTGGCCTGCAGGAACATGGCAAGGGCGAGTGAAGCAGTGTCACGCATTTTAGAAGAATG
GCATAAAGCCAAGGTAGAAGCAATGACCCTGGACCTCGCTCTGCTCCGTAGCGTGCAGCATTTT
 
GCTGAAGCATTCAAGGCCAAGAATGTGCCTCTTCATGTGCTTGTGTGCAACGCAGCAACTTTTG
CTCTACCCTGGAGTCTCACCAAAGATGGCCTGGAGACCACCTTTCAAGTGAATCATCTGGGGCA
CTTCTACCTTGTCCAGCTCCTCCAGGATGTTTTGTGCCGCTCAGCTCCTGCCCGTGTCATTGTG
GTCTCCTCAGAGTCCCATCGATTTACAGATATTAACGACTCCTTGGGAAAACTGGACTTCAGTC
GCCTCTCTCCAACAAAAAACGACTATTGGGCGATGCTGGCTTATAACAGGTCCAAGCTCTGCAA
CATCCTCTTCTCCAACGAGCTGCACCGTCGCCTCTCCCCACGCGGGGTCACGTCGAACGCAGTG
CATCCTGGAAATATGATGTACTCCAACATTCATCGCAGCTGGTGGGTGTACACACTGCTGTTTA
CCTTGGCGAGGCCTTTCACCAAGTCCATGCAACAGGGAGCTGCCACCACCGTGTACTGTGCTGC
TGTCCCAGAACTGGAGGGTCTGGGAGGGATGTACTTCAACAACTGCTGCCGCTGCATGCCCTCA
CCAGAAGCTCAGAGCGAAGAGACGGCCCGGACCCTGTGGGCGCTCAGCGAGAGGCTGATCCAAG
AACGGCTTGGCAGCCAGTCCGGCTAAGTGGAGCTCAGAGCGGATGGGCACACACACCCGCCCTG
TGTGTGTCCCCTCACGCAAGTGCCAGGGCTGGGCCCCTTCCAAATGTCCCTCCAACACAGATCC
GCAAGAGTAAAGGAAATAAGAGCAGTCACAACAGAGTGAAAAATCTTAAGTACCAATGGGAAGC
AGGGAATTCCTGGGGTAAAGTATCACTTTTCTGGGGCTGGGCTAGGCATAGGTCTCTTTGCTTT
CTGGTGGTGGCCTGTTTGAAAGTAAAAACCTGCTTGGTGTGTAGGTTCCGTATCTCCCTGGAGA
AGCACCAGCAATTCTCTTTCTTTTACTGTTATAGAATAGCCTGAGGTCCCCTCGTCCCATCCAG
CTACCACCACGGCCACCACTGCAGCCGGGGGCTGGCCTTCTCCTACTTAGGGAAGAAAAAGCAA
GTGTTCACTGCTCCTTGCTGCATTGATCCAGGAGATAATTGTTTCATTCATCCTGACCAAGACT
GAGCCAGCTTAGCAACTGCTGGGGAGACAAATCTCAGAACCTTGTCCCAGCCAGTGAGGATGAC
AGTGACACCCAGAGGGAGTAGAATACGCAGAACTACCAGGTGGCAAAGTACTTGTCATAGACTC
CTTTGCTAATGCTATGCAAAAAATTCTTTAGAGATTATAACAAATTTTTCAAATCATTCCTTAG
ATACCTTGAAAGGCAGGAAGGGAAGCGTATATACTTAAGAATACACAGGATATTTTGGGGGGCA
GAGAATAAAACGTTAGTTAATCCCTTTGTCTGTCAATCACAGTCTCAGTTCTCTTGCTTTCACA
TTGTACTTAAACCTCCTGCTGTGCCTCGCATCCTATGCTTAATAAAAGAACATGCTTGAATATC
A
SEQ ID No 4: Sequence of Human Synapsin I promoter 
ac tacaaaccga gtatctgcag agggccctgc gtatgagtgc
aagtgggttt taggaccagg atgaggcggg gtgggggtgc ctacctgacg
accgaccccg acccactgga caagcaccca acccccattc cccaaattgc
gcatccccta tcagagaggg ggaggggaaa caggatgcgg cgaggcgcgt
gcgcactgcc agcttcagca ccgcggacag tgccttcgcc cccgcctggc
ggcgcgcgcc accgccgcct cagcactgaa ggcgcgctga cgtcactcgc
cggtcccccg caaactcccc ttcccggcca ccttggtcgc gtccgcgccg
ccgccggccc agccggaccg caccacgcga ggcgcgagat aggggggcac
gggcgcgacc atctgcgctg cggcgccggc gactcagcgc tgcctcagtc
tgcggtgggc agcggaggag tcgtgtcgtg cctgagagcg cagctgtgct
cctgggcacc gcgcagtccg cccccgcggc tcctggccag accaccccta
ggaccccctg ccccaagtcg cagccttcga
SEQ ID No 5: Synapsin 1 / WWOX cDNA construct (bold font-minimal SynI promoter; underline - human WWOX cDNA; regular font - AAV9 vector)

CCTGCAGGCAGCTGCGCGCTCGCTCGCTCACTGAGGCCGCCCGGGCAAAGCCCGGGCGTCGGGC
GACCTTTGGTCGCCCGGCCTCAGTGAGCGAGCGAGCGCGCAGAGAGGGAGTGGCCAACTCCATC
ACTAGGGGTTCCTGCGGCAATTCAGTCGATAACTATAACGGTCCTAAGGTAGCGATTTAAATAC
GCGCTCTCTTAAGGTAGCCCCGGGACGCGTCAATTGACTACAAACCGAGTATCTGCAGAGGGCC
CTGCGTATGAGTGCAAGTGGGTTTTAGGACCAGGATGAGGCGGGGTGGGGGTGCCTACCTGACG
ACCGACCCCGACCCACTGGACAAGCACCCAACCCCCATTCCCCAAATTGCGCATCCCCTATCAG

AGAGGGGGAGGGGAAACAGGATGCGGCGAGGCGCGTGCGCACTGCCAGCTTCAGCACCGCGGAC
AGTGCCTTCGCCCCCGCCTGGCGGCGCGCGCCACCGCCGCCTCAGCACTGAAGGCGCGCTGACG
TCACTCGCCGGTCCCCCGCAAACTCCCCTTCCCGGCCACCTTGGTCGCGTCCGCGCCGCCGCCG
GCCCAGCCGGACCGCACCACGCGAGGCGCGAGATAGGGGGGCACGGGCGCGACCATCTGCGCTG
CGGCGCCGGCGACTCAGCGCTGCCTCAGTCTGCGGTGGGCAGCGGAGGAGTCGTGTCGTGCCTG
AGAGCGCAGCTGTGCTCCTGGGCACCGCGCAGTCCGCCCCCGCGGCTCCTGGCCAGACCACCCC
TAGGACCCCCTGCCCCAAGTCGCAGCCTTCGAGCTAGCGTTTAAACTTAAGCTTGGTACCGGCC
GCTGCGGCCCTCGAGCAAGCTGGCTAGTTAAGCTATCAACAAGTTTGTACAAAAAAGCAGGCTT
TAAAGGAACCAATTCAGTCGAGAATTCGTCGACTGGATCCGGTACCGAGGAGATCTGCCGCCGC
GATCGCCGCAGTGCGCAGGCGTGAGCGGTCGGGCCCCGACGCGCGCGGGTCTCGTTTGGAGCGG
GAGTGAGTTCCTGAGCGAGTGGACCCGGCAGCGGGCGATAGGGGGGCCAGGTGCCTCCACAGTC
AGCCATGGCAGCGCTGCGCTACGCGGGGCTGGACGACACGGACAGTGAGGACGAGCTGCCTCCG
GGCTGGGAGGAGAGAACCACCAAGGACGGCTGGGTTTACTACGCCAATCACACCGAGGAGAAGA
CTCAGTGGGAACATCCAAAAACTGGAAAAAGAAAACGAGTGGCAGGAGATTTGCCATACGGATG
GGAACAAGAAACTGATGAGAACGGACAAGTGTTTTTTGTTGACCATATAAATAAAAGAACCACC
TACTTGGACCCAAGACTGGCGTTTACTGTGGATGATAATCCGACCAAGCCAACCACCCGGCAAA
GATACGACGGCAGCACCACTGCCATGGAAATTCTCCAGGGCCGGGATTTCACTGGCAAAGTGGT
TGTGGTCACTGGAGCTAATTCAGGAATAGGGTTCGAAACCGCCAAGTCTTTTGCCCTCCATGGT
GCACATGTGATCTTGGCCTGCAGGAACATGGCAAGGGCGAGTGAAGCAGTGTCACGCATTTTAG
AAGAATGGCATAAAGCCAAGGTAGAAGCAATGACCCTGGACCTCGCTCTGCTCCGTAGCGTGCA
GCATTTTGCTGAAGCATTCAAGGCCAAGAATGTGCCTCTTCATGTGCTTGTGTGCAACGCAGCA
ACTTTTGCTCTACCCTGGAGTCTCACCAAAGATGGCCTGGAGACCACCTTTCAAGTGAATCATC
TGGGGCACTTCTACCTTGTCCAGCTCCTCCAGGATGTTTTGTGCCGCTCAGCTCCTGCCCGTGT
CATTGTGGTCTCCTCAGAGTCCCATCGATTTACAGATATTAACGACTCCTTGGGAAAACTGGAC
TTCAGTCGCCTCTCTCCAACAAAAAACGACTATTGGGCGATGCTGGCTTATAACAGGTCCAAGC
TCTGCAACATCCTCTTCTCCAACGAGCTGCACCGTCGCCTCTCCCCACGCGGGGTCACGTCGAA
CGCAGTGCATCCTGGAAATATGATGTACTCCAACATTCATCGCAGCTGGTGGGTGTACACACTG
CTGTTTACCTTGGCGAGGCCTTTCACCAAGTCCATGCAACAGGGAGCTGCCACCACCGTGTACT
GTGCTGCTGTCCCAGAACTGGAGGGTCTGGGAGGGATGTACTTCAACAACTGCTGCCGCTGCAT
GCCCTCACCAGAAGCTCAGAGCGAAGAGACGGCCCGGACCCTGTGGGCGCTCAGCGAGAGGCTG
ATCCAAGAACGGCTTGGCAGCCAGTCCGGCTAAGTGGAGCTCAGAGCGGATGGGCACACACACC
CGCCCTGTGTGTGTCCCCTCACGCAAGTGCCAGGGCTGGGCCCCTTCCAAATGTCCCTCCAACA
CAGATCCGCAAGAGTAAAGGAAATAAGAGCAGTCACAACAGAGTGAAAAATCTTAAGTACCAAT
GGGAAGCAGGGAATTCCTGGGGTAAAGTATCACTTTTCTGGGGCTGGGCTAGGCATAGGTCTCT
TTGCTTTCTGGTGGTGGCCTGTTTGAAAGTAAAAACCTGCTTGGTGTGTAGGTTCCGTATCTCC
CTGGAGAAGCACCAGCAATTCTCTTTCTTTTACTGTTATAGAATAGCCTGAGGTCCCCTCGTCC
CATCCAGCTACCACCACGGCCACCACTGCAGCCGGGGGCTGGCCTTCTCCTACTTAGGGAAGAA
AAAGCAAGTGTTCACTGCTCCTTGCTGCATTGATCCAGGAGATAATTGTTTCATTCATCCTGAC
CAAGACTGAGCCAGCTTAGCAACTGCTGGGGAGACAAATCTCAGAACCTTGTCCCAGCCAGTGA
GGATGACAGTGACACCCAGAGGGAGTAGAATACGCAGAACTACCAGGTGGCAAAGTACTTGTCA
TAGACTCCTTTGCTAATGCTATGCAAAAAATTCTTTAGAGATTATAACAAATTTTTCAAATCAT
TCCTTAGATACCTTGAAAGGCAGGAAGGGAAGCGTATATACTTAAGAATACACAGGATATTTTG
GGGGGCAGAGAATAAAACGTTAGTTAATCCCTTTGTCTGTCAATCACAGTCTCAGTTCTCTTGC
TTTCACATTGTACTTAAACCTCCTGCTGTGCCTCGCATCCTATGCTTAATAAAAGAACATGCTT
GAATATCATTGAGTTTAAACCCGCTGATCAGCCTCGACTGTGCCTTCTAGTTGCCAGCCATCTG
TTGTTTGCCCCTCCCCCGTGCCTTCCTTGACCCTGGAAGGTGCCACTCCCACTGTCCTTTCCTA
ATAAAATGAGGAAATTGCATCGCATTGTCTGAGTAGGTGTCATTCTATTCTGGGGGGTGGGGTG
GGGCAGGACAGCAAGGGGGAGGATTGGGAAGACAATAGCAGGCATGCTGGGGATGCGGTGGGCT
CTATGGCTTCTGAGGCGGAAAGAACCAGATCCTCTCTTAAGGTAGCATCGAGATTTAAATTAGG
GATAACAGGGTAATGGCGCGGGCCGCAGGAACCCCTAGTGATGGAGTTGGCCACTCCCTCTCTG
CGCGCTCGCTCGCTCACTGAGGCCGGGCGACCAAAGGTCGCCCGACGCCCGGGCTTTGCCCGGG
CGGCCTCAGTGAGCGAGCGAGCGCGCAGCTGCCTGCAGGGGCGCCTGATGCGGTATTTTCTCCT
TACGCATCTGTGCGGTATTTCACACCGCATACGTCAAAGCAACCATAGTACGCGCCCTGTAGCG
GCGCATTAAGCGCGGCGGGTGTGGTGGTTACGCGCAGCGTGACCGCTACACTTGCCAGCGCCCT
AGCGCCCGCTCCTTTCGCTTTCTTCCCTTCCTTTCTCGCCACGTTCGCCGGCTTTCCCCGTCAA

GCTCTAAATCGGGGGCTCCCTTTAGGGTTCCGATTTAGTGCTTTACGGCACCTCGACCCCAAAA
AACTTGATTTGGGTGATGGTTCACGTAGTGGGCCATCGCCCTGATAGACGGTTTTTCGCCCTTT
GACGTTGGAGTCCACGTTCTTTAATAGTGGACTCTTGTTCCAAACTGGAACAACACTCAACCCT
ATCTCGGGCTATTCTTTTGATTTATAAGGGATTTTGCCGATTTCGGCCTATTGGTTAAAAAATG
AGCTGATTTAACAAAAATTTAACGCGAATTTTAACAAAATATTAACGTTTACAATTTTATGGTG
CACTCTCAGTACAATCTGCTCTGATGCCGCATAGTTAAGCCAGCCCCGACACCCGCCAACACCC
GCTGACGCGCCCTGACGGGCTTGTCTGCTCCCGGCATCCGCTTACAGACAAGCTGTGACCGTCT
CCGGGAGCTGCATGTGTCAGAGGTTTTCACCGTCATCACCGAAACGCGCGAGACGAAAGGGCCT
CGTGATACGCCTATTTTTATAGGTTAATGTCATGATAATAATGGTTTCTTAGACGTCAGGTGGC
ACTTTTCGGGGAAATGTGCGCGGAACCCCTATTTGTTTATTTTTCTAAATACATTCAAATATGT
ATCCGCTCATGAGACAATAACCCTGATAAATGCTTCAATAATATTGAAAAAGGAAGAGTATGAG
TATTCAACATTTCCGTGTCGCCCTTATTCCCTTTTTTGCGGCATTTTGCCTTCCTGTTTTTGCT
CACCCAGAAACGCTGGTGAAAGTAAAAGATGCTGAAGATCAGTTGGGTGCACGAGTGGGTTACA
TCGAACTGGATCTCAACAGCGGTAAGATCCTTGAGAGTTTTCGCCCCGAAGAACGTTTTCCAAT
GATGAGCACTTTTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACCAGTCACAGAAA
AGCATCTTACGGATGGCATGACAGTAAGAGAATTATGCAGTGCTGCCATAACCATGAGTGATAA
CACTGCGGCCAACTTACTTCTGACAACGATCGGAGGACCGAAGGAGCTAACCGCTTTTTTGCAC
AACATGGGGGATCATGTAACTCGCCTTGATCGTTGGGAACCGGAGCTGAATGAAGCCATACCAA
ACGACGAGCGTGACACCACGATGCCTGTAGCAATGGCAACAACGTTGCGCAAACTATTAACTGG
CGAACTACTTACTCTAGCTTCCCGGCAACAATTAATAGACTGGATGGAGGCGGATAAAGTTGCA
GGACCACTTCTGCGCTCGGCCCTTCCGGCTGGCTGGTTTATTGCTGATAAATCTGGAGCCGGTG
AGCGTGGGTCTCGCGGTATCATTGCAGCACTGGGGCCAGATGGTAAGCCCTCCCGTATCGTAGT
TATCTACACGACGGGGAGTCAGGCAACTATGGATGAACGAAATAGACAGATCGCTGAGATAGGT
GCCTCACTGATTAAGCATTGGTAACTGTCAGACCAAGTTTACTCATATATACTTTAGATTGATT
TAAAACTTCATTTTTAATTTAAAAGGATCTAGGTGAAGATCCTTTTTGATAATCTCATGACCAA
AATCCCTTAACGTGAGTTTTCGTTCCACTGAGCGTCAGACCCCGTAGAAAAGATCAAAGGATCT
TCTTGAGATCCTTTTTTTCTGCGCGTAATCTGCTGCTTGCAAACAAAAAAACCACCGCTACCAG
CGGTGGTTTGTTTGCCGGATCAAGAGCTACCAACTCTTTTTCCGAAGGTAACTGGCTTCAGCAG
AGCGCAGATACCAAATACTGTCCTTCTAGTGTAGCCGTAGTTAGGCCACCACTTCAAGAACTCT
GTAGCACCGCCTACATACCTCGCTCTGCTAATCCTGTTACCAGTGGCTGCTGCCAGTGGCGATA
AGTCGTGTCTTACCGGGTTGGACTCAAGACGATAGTTACCGGATAAGGCGCAGCGGTCGGGCTG
AACGGGGGGTTCGTGCACACAGCCCAGCTTGGAGCGAACGACCTACACCGAACTGAGATACCTA
CAGCGTGAGCTATGAGAAAGCGCCACGCTTCCCGAAGGGAGAAAGGCGGACAGGTATCCGGTAA
GCGGCAGGGTCGGAACAGGAGAGCGCACGAGGGAGCTTCCAGGGGGAAACGCCTGGTATCTTTA
TAGTCCTGTCGGGTTTCGCCACCTCTGACTTGAGCGTCGATTTTTGTGATGCTCGTCAGGGGGG
CGGAGCCTATGGAAAAACGCCAGCAACGCGGCCTTTTTACGGTTCCTGGCCTTTTGCTGGCCTT
TTGCTCACATGT

## Claims

1. A WW domain-containing oxidoreductase (WWOX) wild type gene, or a functional derivative thereof encoding an amino acid sequence having at least 95% sequence identity with the amino acid sequence of SEQ ID NO: 2, for use in a method for the treatment of a WWOX-associated CNS disease, the method comprising: administering to the brain of a patient in need of such treatment, said WWOX wild type gene or functional derivative thereof under control of a regulatory element that results in expression of WWOX in the brain, wherein the WWOX-associated CNS disease is WWOX-related epileptic encephalopathy (WOREE) syndrome or spinocerebellar ataxia, autosomal recessive 12 (SCAR12).

2. The gene for the use of claim 1, wherein the regulatory element is a promoter that directs expression of the WWOX gene in neurons, optionally wherein the promoter is a universal promoter, and optionally wherein the promoter is a CMV promoter, E2F1 promoter, or UlsnRNA promoter.

3. The gene for the use of claim 2, wherein the regulatory element is a neuron-specific promoter, optionally wherein the promoter is selected from synapsin I promoter, CamKII promoter, MeCP2 promoter, NSE promoter, and Hb9 promoter, and/or optionally wherein the promoter does not direct expression or directs expression at a lower level in glial cells, optionally wherein the promoter does not direct expression or directs expression at a lower level in oligodendrocytes and/or astrocytes.

4. The gene for the use of claim 1, wherein the regulatory element is a synapsin I promoter.

5. The gene for the use of claim 1, wherein the regulatory element is a promoter that directs expression of the WWOX gene in oligodendrocytes, optionally wherein the promoter is selected from MBP promoter, PLP1 promoter, and CNP promoter, or wherein the regulatory element is a promoter that directs expression of the WWOX gene in astrocytes, optionally wherein the promoter is GFAP promoter or S100b promoter.

6. The gene for the use of any one of claims 2 to 5, wherein the promoter further comprises one or more enhancer sequences.

7. The gene for the use of any one of claims 1 to 6, wherein the WWOX gene comprises untranslated sequences that enhance mRNA stability, or optionally wherein the WWOX wild type gene is delivered with one or more detectable labels, optionally wherein the detectable label is an encoded fluorescent protein.

8. The gene for the use of any one of claims 1 to 7, wherein the WWOX wild type gene or functional derivative thereof is to be delivered using polymeric nanoparticles, inorganic nanoparticles, liponanoparticles, or exosomes.

9. The gene for the use of any one of claims 1 to 8, wherein the WWOX wild type gene or functional derivative thereof is to be delivered with a Cas enzyme or polynucleotide encoding a Cas enzyme, and gRNA or polynucleotide encoding the gRNA, to direct insertion of the WWOX wild type gene or functional derivative thereof.

10. The gene for the use of any one of claims 1 to 9, wherein the WWOX wild type gene or functional derivative thereof is to be delivered by a viral vector, optionally wherein the viral vector is an adeno-associated virus (AAV) vector, and optionally wherein the AAV vector is AAV9.

11. The gene for the use of any one of claims 1 to 10, wherein the WWOX wild type gene encodes the amino acid sequence of SEQ ID NO: 2, optionally wherein the WWOX wild type gene comprises one or more introns, or optionally wherein the WWOX wild type gene is a cDNA.

12. The gene for the use of any one of claims 1-11, wherein the WWOX wild type gene, or a functional derivative thereof, under control of a regulatory element comprises the nucleotide sequence as set forth in SEQ ID NO: 1 or 3.

13. The gene for the use of any one of claims 1 to 12, wherein the administration is by a route selected from direct injection into the parenchyma, injection into the cerebrospinal fluid via the intracerebroventricular, and by intrathecal (cisternal or lumbar) route.

14. The gene for the use of any one of claims 1 to 13, wherein the individual is a pediatric or neonatal patient, optionally wherein the individual is an adult patient, and optionally wherein the patient exhibits one or more symptoms selected from growth impairment, epileptic episodes, impairment of cognitive function, impairment of social function, impairment of fertility, ataxia, retinopathy, mental retardation, and microcephaly.

15. The gene for the use of any one of claims 1 to 14, wherein there are no more than three administration episodes, optionally wherein there are no more than two administration episodes, and optionally wherein there is one administration episode.

16. An AAV9 vector for use in a method for the treatment of WOREE syndrome or SCAR12, the method comprising: administering to the brain of a patient in need of such treatment, said AAV9 vector, wherein the AAV9 vector comprises a WWOX wild type gene under control of a synapsin- 1 promoter.

17. The AAV9 vector for use of claim 16, wherein the AAV9 vector comprises the nucleotide sequence as set forth in SEQ ID NO: 1 or SEQ ID NO: 3.

18. An expression construct, comprising a WWOX wild type gene, or a functional derivative thereof encoding an amino acid sequence having at least 95% sequence identity with the amino acid sequence of SEQ ID NO: 2, under the expression control of a neuron-specific promoter.

19. The expression construct of claim 18, wherein the promoter is synapsin 1 promoter.

20. The expression construct of any one of claims 18 or 19, wherein the expression construct comprises the nucleotide sequence as set forth in SEQ ID NO: 1, 3, 4, or 5.

21. The expression construct of any one of claims 18-20, wherein the expression construct is a viral vector, optionally wherein the viral vector is adeno-associated virus (AAV), and optionally wherein the AAV is AAV9.

22. A pharmaceutical composition for direct administration into the brain comprising the expression construct of any one of claims 18 to 21 and a pharmaceutically acceptable carrier suitable for direct injection to the brain.

## Patentansprüche

1. WW-Domäne-enthaltendes Oxidoreduktase (WWOX)-Wildtyp-Gen oder ein funktionelles Derivat davon, das für eine Aminosäuresequenz kodiert, die mindestens 95 % Sequenzidentität mit der Aminosäuresequenz von SEQ ID NO: 2 aufweist, zur Verwendung in einem Verfahren zur Behandlung einer WWOX-assoziierten ZNS-Erkrankung, wobei das Verfahren umfasst: Verabreichung des WWOX-Wildtyp-Gens oder eines funktionellen Derivats davon an das Gehirn eines Patienten, der einer solchen Behandlung bedarf, unter der Kontrolle eines regulatorischen Elements, das zur Expression von WWOX im Gehirn führt, wobei die WWOX-assoziierte ZNS-Krankheit das WWOX-bezogene epileptische Enzephalopathie-Syndrom (WOREE) oder die autosomal rezessive spinozerebelläre Ataxie 12 (SCAR12) ist.

2. Gen zur Verwendung nach Anspruch 1, wobei das regulatorische Element ein Promotor ist, der die Expression des WWOX-Gens in Neuronen steuert, wobei der Promotor optional ein universeller Promotor ist, und wobei der Promotor optional ein CMV-Promotor, E2F1-Promotor oder UlsnRNA-Promotor ist.

3. Gen für die Verwendung nach Anspruch 2, wobei das regulatorische Element ein neuronenspezifischer Promotor ist, wobei der Promotor optional aus dem Synapsin I-Promotor, dem CamKll-Promotor, dem MeCP2-Promotor, dem NSE-Promotor und dem Hb9-Promotor ausgewählt ist, und/oder wobei der Promotor optional die Expression nicht oder auf einem niedrigeren Niveau in Gliazellen steuert, wobei der Promotor die Expression optional nicht oder auf einem niedrigeren Niveau in Oligodendrozyten und/oder Astrozyten steuert.

4. Gen zur Verwendung nach Anspruch 1, wobei das regulatorische Element ein Synapsin-I-Promotor ist.

5. Gen zur Verwendung nach Anspruch 1, wobei das regulatorische Element ein Promotor ist, der die Expression des WWOX-Gens in Oligodendrozyten steuert, wobei der Promotor optional aus dem MBP-Promotor, dem PLP1-Promotor und dem CNP-Promotor ausgewählt ist, oder wobei das regulatorische Element ein Promotor ist, der die Expression des WWOX-Gens in Astrozyten steuert, wobei der Promotor optional der GFAP-Promotor oder der S100b-Promotor ist.

6. Gen zur Verwendung nach einem der Ansprüche 2 bis 5, wobei der Promotor ferner eine oder mehrere Enhancer-Sequenzen umfasst.

7. Gen zur Verwendung nach einem der Ansprüche 1 bis 6, wobei das WWOX-Gen untranslatierte Sequenzen umfasst, die die mRNA-Stabilität erhöhen, oder optional, wobei das WWOX-Wildtyp-Gen mit einer oder mehreren nachweisbaren Markierungen übertragen wird, wobei die nachweisbare Markierung optional ein kodiertes fluoreszierendes Protein ist.

8. Gen zur Verwendung nach einem der Ansprüche 1 bis 7, wobei das WWOX-Wildtyp-Gen oder ein funktionelles Derivat davon unter Verwendung von polymeren Nanopartikeln, anorganischen Nanopartikeln, Liponanopartikeln oder Exosomen übertragen werden soll.

9. Gen für die Verwendung nach einem der Ansprüche 1 bis 8, wobei das WWOX-Wildtyp-Gen oder ein funktionelles Derivat davon mit einem Cas-Enzym oder einem Polynukleotid, das für ein Cas-Enzym kodiert, und gRNA oder einem Polynukleotid, das für die gRNA kodiert, übertragen werden soll, um die Insertion des WWOX-Wildtyp-Gens oder eines funktionellen Derivats davon zu steuern.

10. Gen zur Verwendung nach einem der Ansprüche 1 bis 9, wobei das WWOX-Wildtyp-Gen oder ein funktionelles Derivat davon durch einen viralen Vektor übertragen werden soll, wobei der virale Vektor optional ein Adeno-assoziierter Virus (AAV)-Vektor ist und wobei der AAV-Vektor optional AAV9 ist.

11. Gen zur Verwendung nach einem der Ansprüche 1 bis 10, wobei das WWOX-Wildtyp-Gen für die Aminosäuresequenz von SEQ ID NO: 2 kodiert, wobei das WWOX-Wildtyp-Gen optional ein oder mehrere Introns umfasst, oder wobei das WWOX-Wildtyp-Gen optional eine cDNA ist.

12. Gen zur Verwendung nach einem der Ansprüche 1 bis 11, wobei das WWOX-Wildtyp-Gen oder ein funktionelles Derivat davon unter der Kontrolle eines regulatorischen Elements die in SEQ ID NO: 1 oder 3 dargelegte Nukleotidsequenz umfasst.

13. Gen zur Verwendung nach einem der Ansprüche 1 bis 12, wobei die Verabreichung über einen Weg erfolgt, der ausgewählt ist aus direkter Injektion in das Parenchym, Injektion in die Zerebrospinalflüssigkeit über den intrazerebroventrikulären Weg und über den intrathekalen Weg (cisternal oder lumbal).

14. Gen zur Verwendung nach einem der Ansprüche 1 bis 13, wobei das Individuum ein pädiatrischer oder neonataler Patient ist, optional, wobei das Individuum ein erwachsener Patient ist, und optional, wobei der Patient ein oder mehrere Symptome aufweist, ausgewählt aus Wachstumsstörung, epileptischen Episoden, Beeinträchtigung der kognitiven Funktion, Beeinträchtigung der sozialen Funktion, Beeinträchtigung der Fruchtbarkeit, Ataxie, Retinopathie, mentale Retardierung und Mikrozephalie.

15. Gen für die Verwendung nach einem der Ansprüche 1 bis 14, wobei es nicht mehr als drei Verabreichungsepisoden gibt, optional, wobei es nicht mehr als zwei Verabreichungsepisoden gibt, und optional, wobei es eine Verabreichungsepisode gibt.

16. AAV9-Vektor zur Verwendung in einem Verfahren zur Behandlung des WOREE-Syndroms oder SCAR12, wobei das Verfahren umfasst: Verabreichung des AAV9-Vektors an das Gehirn eines Patienten, der eine solche Behandlung benötigt, wobei der AAV9-Vektor ein WWOX-Wildtyp-Gen unter der Kontrolle eines Synapsin-1-Promotors umfasst.

17. AAV9-Vektor zur Verwendung nach Anspruch 16, wobei der AAV9-Vektor die Nukleotidsequenz wie in SEQ ID NO: 1 oder SEQ ID NO: 3 dargelegt umfasst.

18. Expressionskonstrukt, umfassend ein WWOX-Wildtyp-Gen oder ein funktionelles Derivat davon, das für eine Aminosäuresequenz mit mindestens 95% Sequenzidentität mit der Aminosäuresequenz von SEQ ID NO: 2 kodiert, unter der Expressionskontrolle eines neuronenspezifischen Promotors.

19. Expressionskonstrukt nach Anspruch 18, wobei der Promotor der Synapsin-1-Promotor ist.

20. Expressionskonstrukt nach einem der Ansprüche 18 oder 19, wobei das Expressionskonstrukt die Nukleotidsequenz wie in SEQ ID NO: 1, 3, 4 oder 5 dargelegt umfasst.

21. Expressionskonstrukt nach einem der Ansprüche 18 bis 20, wobei das Expressionskonstrukt ein viraler Vektor ist, wobei der virale Vektor optional ein Adenoassoziiertes Virus (AAV) ist, und wobei das AAV optional AAV9 ist.

22. Pharmazeutische Zusammensetzung zur direkten Verabreichung in das Gehirn, umfassend das Expressionskonstrukt nach einem der Ansprüche 18 bis 21 und einen pharmazeutisch akzeptablen Träger, der zur direkten Injektion in das Gehirn geeignet ist.

## Revendications

1. Gène de type sauvage d'oxydoréductase contenant un domaine WW (WWOX), ou dérivé fonctionnel de celui-ci codant pour une séquence d'acides aminés présentant au moins 95 % d'identité de séquence avec la séquence d'acides aminés de SEQ ID NO : 2, pour utilisation dans un procédé de traitement d'une maladie du SNC associée à WWOX, le procédé comprenant : l'administration au cerveau d'un patient ayant besoin d'un tel traitement, dudit gène de type sauvage WWOX ou dudit dérivé fonctionnel de celui-ci sous régulation d'un élément régulateur qui entraîne l'expression de WWOX dans le cerveau, dans lequel la maladie du SNC associée à WWOX est le syndrome d'encéphalopathie épileptique liée à WWOX (WOREE) ou l'ataxie spinocérébelleuse, autosomique récessive 12 (SCAR12).

2. Gène pour utilisation selon la revendication 1, dans lequel l'élément régulateur est un promoteur qui dirige l'expression du gène WWOX dans les neurones, éventuellement dans lequel le promoteur est un promoteur universel, et éventuellement dans lequel le promoteur est un promoteur CMV, un promoteur E2F1 ou un promoteur ARNsn UI.

3. Gène pour utilisation selon la revendication 2, dans lequel l'élément régulateur est un promoteur spécifique aux neurones, éventuellement dans lequel le promoteur est sélectionné parmi le promoteur de synapsine I, le promoteur CamKll, le promoteur MeCP2, le promoteur NSE et le promoteur Hb9, et/ou éventuellement dans lequel le promoteur ne dirige pas l'expression ou ne dirige pas l'expression à un niveau inférieur dans les cellules gliales, éventuellement dans lequel le promoteur ne dirige pas l'expression ou ne dirige pas l'expression à un niveau inférieur dans les oligodendrocytes et/ou les astrocytes.

4. Gène pour utilisation selon la revendication 1, dans lequel l'élément régulateur est un promoteur de synapsine I.

5. Gène pour utilisation selon la revendication 1, dans lequel l'élément régulateur est un promoteur qui dirige l'expression du gène WWOX dans les oligodendrocytes, éventuellement dans lequel le promoteur est sélectionné parmi le promoteur MBP, le promoteur PLP1 et le promoteur CNP, ou dans lequel l'élément régulateur est un promoteur qui dirige l'expression du gène WWOX dans les astrocytes, éventuellement dans lequel le promoteur est le promoteur GFAP ou le promoteur S100b.

6. Gène pour utilisation selon l'une quelconque des revendications 2 à 5, dans lequel le promoteur comprend en outre une ou plusieurs séquences amplificatrices.

7. Gène pour utilisation selon l'une quelconque des revendications 1 à 6, dans lequel le gène WWOX comprend des séquences non traduites qui renforcent la stabilité d'ARNm, ou éventuellement dans lequel le gène de type sauvage WWOX est administré avec une ou plusieurs étiquettes détectables, éventuellement dans lequel l'étiquette détectable est une protéine fluorescente codée.

8. Gène pour utilisation selon l'une quelconque des revendications 1 à 7, dans lequel le gène de type sauvage WWOX ou un dérivé fonctionnel de celui-ci est à administrer à l'aide de nanoparticules polymères, de nanoparticules inorganiques, de liponanoparticules ou d'exosomes.

9. Gène pour utilisation selon l'une quelconque des revendications 1 à 8, dans lequel le gène de type sauvage WWOX ou un dérivé fonctionnel de celui-ci est à administrer avec une enzyme Cas ou un polynucléotide codant pour une enzyme Cas, et un ARNg ou un polynucléotide codant pour l'ARNg, pour l'insertion directe du gène de type sauvage WWOX ou d'un dérivé fonctionnel de celui-ci.

10. Gène pour utilisation selon l'une quelconque des revendications 1 à 9, dans lequel le gène de type sauvage WWOX ou un dérivé fonctionnel de celui-ci est à administrer par un vecteur viral, éventuellement dans lequel le vecteur viral est un vecteur de virus adéno-associé (AAV), et éventuellement dans lequel le vecteur AAV est AAV9.

11. Gène pour utilisation selon l'une quelconque des revendications 1 à 10, dans lequel le gène de type sauvage WWOX code pour la séquence d'acides aminés de SEQ ID NO: 2, éventuellement dans lequel le gène de type sauvage WWOX comprend un ou plusieurs introns, ou éventuellement dans lequel le gène de type sauvage WWOX est un ADNc.

12. Gène pour utilisation selon l'une quelconque des revendications 1-11, dans lequel le gène de type sauvage WWOX, ou un dérivé fonctionnel de celui-ci, sous régulation d'un élément régulateur comprend la séquence de nucléotides telle que présentée dans SEQ ID NO : 1 ou 3.

13. Gène pour utilisation selon l'une quelconque des revendications 1 à 12, dans lequel l'administration s'effectue par une voie sélectionnée parmi l'injection directe dans le parenchyme, l'injection dans le liquide céphalorachidien via l'intracérébroventriculaire, et par voie intrathécale (cisternale ou lombaire).

14. Gène pour utilisation selon l'une quelconque des revendications 1 à 13, dans lequel l'individu est un patient pédiatrique ou néonatal, éventuellement dans lequel l'individu est un patient adulte, et éventuellement dans lequel le patient présente un ou plusieurs symptômes sélectionnés parmi un retard de croissance, des crises épileptiques, une altération de la fonction cognitive, une altération de la fonction sociale, une altération de la fertilité, une ataxie, une rétinopathie, un retard mental et une microcéphalie.

15. Gène pour utilisation selon l'une quelconque des revendications 1 à 14, dans lequel on ne compte pas plus de trois crises liées à l'administration, éventuellement dans lequel on ne compte pas plus de deux crises liées à l'administration, et éventuellement dans lequel on compte une crise liée à l'administration.

16. Vecteur AAV9 pour utilisation dans un procédé de traitement du syndrome de WOREE ou SCAR12, le procédé comprenant : l'administration au cerveau d'un patient ayant besoin d'un tel traitement, dudit vecteur AAV9, dans lequel le vecteur AAV9 comprend un gène de type sauvage WWOX sous régulation d'un promoteur de synapsine-1.

17. Vecteur AAV9 pour utilisation selon la revendication 16, dans lequel le vecteur AAV9 comprend la séquence de nucléotides telle que présentée dans SEQ ID NO : 1 ou SEQ ID NO : 3.

18. Construction d'expression, comprenant un gène de type sauvage WWOX, ou un dérivé fonctionnel de celui-ci codant pour une séquence d'acides aminés présentant au moins 95 % d'identité de séquence avec la séquence d'acides aminés de SEQ ID NO : 2, sous la régulation d'expression d'un promoteur neuronal spécifique.

19. Construction d'expression selon la revendication 18, dans laquelle le promoteur est le promoteur de synapsine 1.

20. Construction d'expression selon l'une quelconque des revendications 18 ou 19, dans laquelle la construction d'expression comprend la séquence de nucléotides telle que présentée dans SEQ ID NO : 1, 3, 4 ou 5.

21. Construction d'expression selon l'une quelconque des revendications 18-20, dans laquelle la construction d'expression est un vecteur viral, éventuellement dans laquelle le vecteur viral est un virus adéno-associé (AAV), et éventuellement dans laquelle l'AAV est AAV9.

22. Composition pharmaceutique pour administration directe dans le cerveau comprenant la construction d'expression selon l'une quelconque des revendications 18 à 21 et un vecteur pharmaceutiquement acceptable adapté à l'injection directe dans le cerveau.
